(19) Europäisches Patentamt
European Patent Office
Office européen des brevets

(11) EP 4 484 433 A1

(12) **EUROPEAN PATENT APPLICATION**
published in accordance with Art. 153(4) EPC

(43) Date of publication:
**01.01.2025 Bulletin 2025/01**

(21) Application number: **23759100.3**

(22) Date of filing: **17.02.2023**

(51) International Patent Classification (IPC):
***C07D 519/00*** *(2006.01)* ***A61K 31/5025*** *(2006.01)*
***A61P 25/00*** *(2006.01)*

(52) Cooperative Patent Classification (CPC):
**A61K 31/5025; A61K 31/519; A61K 31/5377; A61P 9/10; A61P 25/00; A61P 25/02; A61P 25/04; A61P 25/06; A61P 25/28; A61P 29/00; C07D 519/00**

(86) International application number:
**PCT/CN2023/076805**

(87) International publication number:
**WO 2023/160475 (31.08.2023 Gazette 2023/35)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **25.02.2022 CN 202210178772**

(71) Applicant: **Shanghai Simr Biotechnology Co., Ltd.**
**Shanghai 201318 (CN)**

(72) Inventors:
- **WANG, Fei**
  **Shanghai 201318 (CN)**
- **CHEN, Nanyang**
  **Shanghai 201318 (CN)**
- **SUN, Yong**
  **Shanghai 201318 (CN)**

(74) Representative: **Dai, Simin**
**Reyda IP**
**A073**
**157, Quai du Président Roosevelt**
**92130 Issy-les-Moulineaux (FR)**

(54) **IMIDAZOPYRIDAZINE DERIVATIVE, AND PREPARATION METHOD THEREFOR, PHARMACEUTICAL COMPOSITION THEREOF AND USE THEREOF**

(57) Provided are an imidazopyridazine derivative, and a preparation method therefor, a pharmaceutical composition thereof and the use thereof. Specifically provided are a compound as shown in formula (1), and a stereoisomer, tautomer, prodrug, pharmaceutically acceptable salt, amorphous substance, isotopologue, polymorph or solvate thereof, a pharmaceutical composition containing the compound and the use of the compound as a $GABA_A$ receptor modulator.

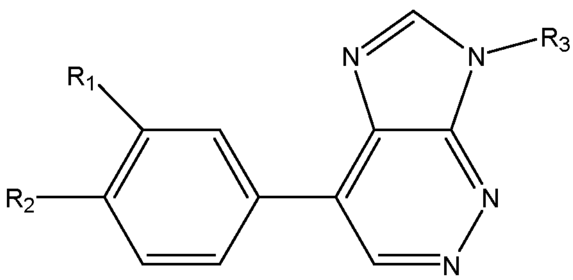

(1)

EP 4 484 433 A1

## Description

CROSS REFERENCE TO RELATED APPLICATIONS

The present application claims priority to Chinese patent application No. 202210178772.9 filed with the Chinese Patent Office on February 25, 2022 and entitled "IMIDAZOPYRIDAZINE DERIVATIVE, AND PREPARATION METHOD THEREFOR, PHARMACEUTICAL COMPOSITION THEREOF AND USE THEREOF". The contents of the above Chinese patent application are incorporated herein by reference in its entirety.

TECHNICAL FIELD

The present disclosure relates to imidazopyridazine derivatives having a regulatory function on the $GABA_A$ receptor, a preparation method therefor, a pharmaceutical composition thereof and the use thereof as a medicament.

BACKGROUND

Gamma-aminobutyric acid (GABA) is an important inhibitory neurotransmitter in the mammalian central nervous system. Substances that modulate GABAergic neurotransmission are widely used in the treatment of various conditions such as epilepsy, anxiety and depression. In nature, there are two classes of GABA receptors, $GABA_A$ receptors ($GABA_A$Rs), which are members of the ligand-gated ion channel superfamily, and $GABA_B$ receptors ($GABA_B$Rs), which are members of the G protein-coupled receptor superfamily. $GABA_A$ receptor subunits found in mammals include $\alpha$1-6, $\beta$1-4, $\gamma$1-3, $\delta$, $\varepsilon$, $\theta$, $\rho$1-2, etc., of which the $\alpha$ subunit, $\beta$ subunit and $\gamma$ subunit are essential for the formation of a complete functional $GABA_A$ receptor, and the $\alpha$ subunit is of great importance for the binding of benzodiazepines to the $GABA_A$ receptor.

Drugs bound at an allosteric binding site can be positive allosteric modulators (or positive allosteric modulators) that increase receptor activity, negative allosteric modulators (or reverse allosteric modulators) that decrease receptor activity, or neutral allosteric modulators that do not alter receptor activity (which refer to compounds that bind to the allosteric binding site but do not modulate receptor activity). Recent evidence suggests that $GABA_A$ receptors containing $\alpha$2 or $\alpha$3 subunits (referred to herein as $\alpha$2/3-$GABA_A$ receptors) may be involved in certain pain states, and that positive allosteric modulators of these receptors may be potent analgesics (Mirza, N. R. and Munro, G., Drug News and Perspectives, 2010, 23(6), 351-360).

International Patent Applications PCT/GB01/04948 (Announcement No. WO2002/038568) and PCT/GB02/03114 (Announcement No. WO2003/008418) describe 7-phenylimidazo[1,2-b][1,2,4]triazine derivatives, which have an affinity for $\alpha$2, $\alpha$3 and/or $\alpha$5 subunits. International Patent Application PCT/US99/14935 (Announcement No. WO2000/001697) discloses inter alia 4-phenyl-7H-imidazo[4,5-c]pyridazine derivatives, which are corticotropin releasing factor antagonists. International Patent Applications PCT/IB2013/060631 (Announcement No. WO2014/091368) and PCT/IB2015/054200 (Announcement No. WO2015/189744) and the article by Robert M. Owen (Robert M. Owen, J. Med. Chem.2019, 62, 5773-5796) describe 4-(biphenyl-3-yl)-7H-imidazo[4,5-c]pyridazine derivatives, which interact with the $\alpha$2/3-$GABA_A$ receptor and are used in the treatment of a variety of diseases including pain. The compounds are also used in the treatment of pruritus (International Patent Application PCT/US2019/033598, Announcement No. WO2019/26820A1) and epilepsy (Duveau, V, CNS Neurosci. Ther. 2019. 25(2): p. 255-260.).

The prevailing view is that the modulatory activity at the $GABA_A$ receptor containing $\alpha$1 subunits is the main source of side effects (such as sedation, addiction, lethargy and amnesia) of current $GABA_A$ modulators (such as benzodiazepines) (Uwe Rudolph and Frederic Knoflach, Nature Reviews: Drug Discovery, 2011,10(9),685-697). The search for new compounds that interact with the $GABA_A$ receptor and have fewer $\alpha$1-$GABA_A$ receptor-associated side effects will have great therapeutic potential.

SUMMARY

According to one aspect of the present disclosure, a compound of formula (1), a stereoisomer thereof, a tautomer thereof, a prodrug thereof, a pharmaceutically acceptable salt thereof, an amorphous material thereof, a polymorph thereof, or a solvate thereof is provided:

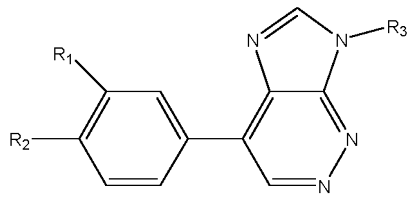

Formula (I)

wherein

$R_1$ is a fused group formed by a substituted or unsubstituted heterocyclic ring and a heterocyclic ring;
$R_2$ is selected from H, halogen, OH, C1-C6 alkoxy or CN;
$R_3$ is selected from H, substituted or unsubstituted linear or branched C1-C6 alkyl or substituted or unsubstituted substituted C3-C6 cycloalkyl.

Unless otherwise indicated, the following definitions are provided to illustrate and define the meaning and scope of the various terms used herein in describing the present disclosure.
Whether used alone or in combination, the following definitions of the general terms apply.
Nomenclature rules used in the present disclosure are based on IUPAC rules.
Unless otherwise specified, the term "substituted" means that a specified group or a part thereof may have 1, 2, 3, 4, 5 or 6 substituents thereon. When a group may have a plurality of substituents thereon and a plurality of possible substituents are given, the substituents are selected independently and need not be identical.
The term "unsubstituted" means that a specified group has no substituent thereon.
The term "optionally substituted" means that a specified group is unsubstituted or substituted with one or more substituents independently selected from the possible substituents.
When specifying the number of substituents, the term "one or more" refers to one substitution to the maximum possible number of substitutions, i.e., substitution of one substituent for one hydrogen to substitution of substituents for all hydrogens. Unless otherwise indicated, 1, 2, 3, 4 or 5 substituents are preferred.
In particular, in the definitions of the $R_1$ and $R_3$ groups, "substituted" means that one or more hydrogens on the groups are substituted with a group selected from C1-C4 alkyl, C1-C4 alkoxy, C1-C4 halogen-substituted alkyl or halogen.
The term "halogen" refers to fluorine, chlorine, bromine and iodine, preferably fluorine.
The compound of the present disclosure may comprise an asymmetric or chiral center, and thus presents in different stereoisomeric forms. All stereoisomeric forms of the compound of the present disclosure, including but not limited to diastereomers, enantiomers and atropisomers, and mixtures thereof, e.g., racemic mixtures, will form part of the present disclosure. In the present disclosure, all stereoisomers are contemplated when the stereochemistry of any particular chiral atom is not determined. Furthermore, the present disclosure relates to all geometric and positional isomers. The compound of the present disclosure may present in different tautomeric forms, and all those forms fall within the scope of the present disclosure. All stereoisomers of the compound of the present disclosure are expected to include a mixture form or a pure or substantially pure form. The resolution may be achieved by physical methods such as fractional crystallization, separation or crystallization of diastereomeric derivatives, or separation by chiral column chromatography.
The term "prodrug" is a functional derivative of the compound of formula (1), and the derivative is readily converted *in vivo* to the compound of formula (1). Suitable said derivative may be selected and prepared by conventional techniques well known to those skilled in the art, see, for example, Design of Prodrugs, ed.H. Bundgaard, Elsevier, 1985.
The term "pharmaceutically acceptable salt" used herein refers to pharmaceutically acceptable organic or inorganic salts of the compound of the present disclosure. Exemplary salts include, but are not limited to, sulfate, citrate, acetate, oxalate, chloride, bromide, iodide, nitrate, bisulfate, isonicotinic acid salt, lactate, salicylate, acid citrate, succinate, maleate, fumarate, gluconate, formate, methanesulfonate and pamoate. The "pharmaceutically acceptable salt" may relate to the inclusion of another molecule, e.g., a maleate or other counterions. The counterion stabilizes the charge in the parent compound. The "pharmaceutically acceptable salt" may have more than one charged atom, where multiple charged atoms may have multiple counterions.
If the compound of the present disclosure is a base, the desired "pharmaceutically acceptable salt" may be prepared by a suitable method, for example, treating the free base with the following inorganic acids: hydrochloric acid, hydrobromic acid, sulfuric acid, nitric acid, phosphoric acid; or with the following organic acids: acetic acid, maleic acid, succinic acid, mandelic acid, fumaric acid, malonic acid, pyruvic acid, salicylic acid, glucopyranosiduronic acid, e.g., glucuronic acid or galacturonic acid, $\alpha$-hydroxy acid, e.g., citric acid or tartaric acid, amino acid, e.g., glutamic acid, aromatic acid, e.g., benzoic acid or cinnamic acid, and sulfonic acid, e.g., methanesulfonic acid or *p*-toluenesulfonic acid.
If the compound of the present disclosure is an acid, the desired "pharmaceutically acceptable salt" may be

prepared by a suitable method, for example, treating the free acid with the following inorganic or organic bases: amine, alkali metal hydroxide or alkaline earth metal hydroxide, etc. Illustrative examples of suitable salts include, but are not limited to, organic salts derived from amino acids, salts of primary, secondary and tertiary amines, salts of cyclic amines, e.g., piperidine, morpholine and piperazine, and inorganic salts derived from sodium, calcium, potassium, magnesium, manganese, iron, copper, zinc, aluminum and lithium.

The compound of the present disclosure may exist in a continuous solid state ranging from completely amorphous to completely crystalline. The term "amorphous" refers to a state in which a material lacks long-range order at the molecular level, and the material may exhibit the physical properties of a solid or a liquid, depending on the temperature. Typically, without giving a distinct X-ray diffraction pattern while showing the properties of a solid, the material is more formally described as a liquid. On heating, a change occurs from the properties of a solid to that of a liquid, characterized by a change of state, usually secondary ("glass transition"). The term "crystal" refers to a solid phase in which a material has a regularly ordered internal structure on the molecular level and gives a unique X-ray diffraction pattern with defined peaks. The material will also exhibit the properties of a liquid when heated sufficiently, but the change from solid to liquid is characterized by a phase change, typically first order ("melting point").

The term "polymorph" used herein refers to different solid crystalline phases of certain compounds of the present disclosure in the solid state due to the presence of two or more different molecular arrangements. Certain compounds of the present disclosure may exist in more than one crystalline form, and the present disclosure is intended to include the various crystalline forms and mixtures thereof.

The term "solvate" used herein refers to a conjugate or complex of one or more solvent molecules with the compound of the present disclosure. Examples of solvents that form solvates include, but are not limited to, water, isopropanol, ethanol, methanol, dimethyl sulfoxide, ethyl acetate, acetic acid and ethanolamine. The compound of the present disclosure may exist in unsolvated forms as well as solvated forms with pharmaceutically acceptable solvents, e.g., water and ethanol, and thus the present disclosure is intended to include both solvated and unsolvated forms.

The compound of the present disclosure may comprise an unnatural proportion of atomic isotope at one or more of the atoms that constitute the compound. The term "isotopic variant" refers to having the same number of atoms, but having an atomic mass or mass number different from the atomic mass or mass number found predominantly in nature. For example, the compound may be labeled with a radioisotope, e.g., deuterium (2H), tritium (3H), iodine-125 (125I) or C-14 (14C). All isotopic variations of the compound of the present disclosure, whether radioactive or not, are encompassed within the scope of the present disclosure. Isotopic variants may enhance certain therapeutic benefits, for example, deuterium enrichment may increase *in vivo* half-life or reduce dosage requirements, or may provide standard product compounds which may be used as the characterization of biological samples. Isotopically enriched compounds of formula (1) can be prepared by conventional techniques well known to those skilled in the art or by methods similar to those described in the schemes and examples herein using appropriate isotopically enriched reagents and/or intermediates without undue experimentation.

**[$R_2$]**

In the compound of the present disclosure, $R_2$ is selected from H, halogen, OH, C1-C6 alkoxy or CN; preferably, the $R_2$ is preferably H or halogen, more preferably H or F.

**[$R_3$]**

In the compound of the present disclosure, $R_3$ is selected from H, substituted or unsubstituted linear or branched C1-C6 alkyl or substituted or unsubstituted C3-C6 cycloalkyl. In $R_3$, the C1-C6 alkyl may be linear or branched, and exemplary C1-C6 alkyl includes methyl, ethyl, *n*-propyl (1-propyl), isopropyl (2-propyl, 1-methylethyl), *n*-butyl (1-butyl), *sec*-butyl (2-butyl, 1-methylpropyl), isobutyl (2-methylpropyl) or Fe/F-butyl (1,1-dimethylethyl). One to more hydrogen atoms on the above alkyl may be substituted with a substituent, e.g., methyl, ethyl, *n*-propyl, methoxy, ethoxy, propoxy, fluoromethyl, fluoroethyl, fluorine, chlorine and bromine.

The term "cycloalkyl" refers to a monovalent saturated cyclic hydrocarbonyl, and in $R_3$, exemplary C3-C5 cycloalkyl includes cyclopropyl, cyclobutyl or cyclopentyl.

In $R_3$, exemplary C3-C6 cycloalkyl includes 1-methylcyclopropyl, 2-methylcyclopropyl, 1-methylcyclobutyl, 2-methylcyclobutyl, 3-methylcyclobutyl, 1-methylcyclopentyl, 2-methylcyclopentyl or 3-methylcyclopentyl. One to more hydrogen atoms on the above cycloalkyl may be substituted with a substituent, e.g., methyl, ethyl, *n*-propyl, methoxy, ethoxy, propoxy, fluoromethyl, fluoroethyl, fluorine, chlorine and bromine.

The term "alkoxy" refers to an -O-alkyl group, and in $R_3$, exemplary C1-C6 alkoxy includes methoxy, ethoxy, *n*-propoxy, isopropoxy, *n*-butoxy, *sec*-butoxy, etc. One to more hydrogen atoms on the above alkoxy may be substituted with a substituent, e.g., methyl, ethyl, *n*-propyl, methoxy, ethoxy, propoxy, fluoromethyl, fluoroethyl, fluorine, chlorine and bromine.

In an optional embodiment of the present disclosure, $R_3$ is selected from linear or branched C1-C6 alkyl, C1-C6 alkoxy, and is more preferably C2-C4 alkyl, more preferably ethyl or isopropyl.

**[$R_1$]**

In the schemes of the present disclosure, $R_1$ is a fused group of a heterocyclic ring and a heterocyclic ring, and both heterocyclic rings independently refer to a saturated or partially unsaturated monocyclic or polycyclic group having a heteroatom, wherein optionally, the heteroatom is N, O or S. Optionally, the two heterocyclic rings are independently a 3- to 7-membered saturated or partially unsaturated monocyclic or polycyclic group comprising 1-3 ring heteroatoms selected from N, O or S. Optionally, S and O may be present as -SO or $SO_2$.
Optionally, the two heterocyclic rings are independently a 5- to 7-membered saturated or unsaturated monocyclic group comprising 1, 2 or 3 ring heteroatoms selected from N, O or S. Optionally, the two heterocyclic rings are independently a 5- to 6-membered saturated or unsaturated monocyclic group comprising 1, 2 or 3 ring heteroatoms selected from N or O.
Optionally, $R_1$ has the structure of formula (2), (3), (4), (5), (6) or (7):

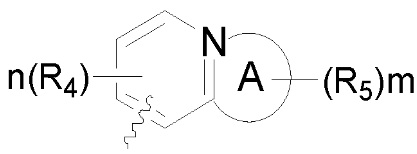

(2)

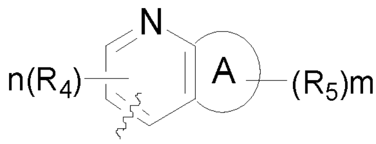

(3)

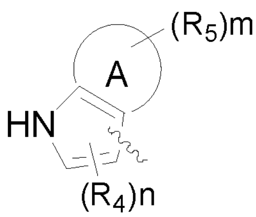

(4)

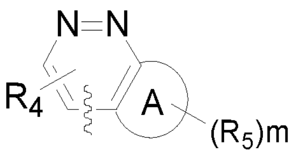

(5)

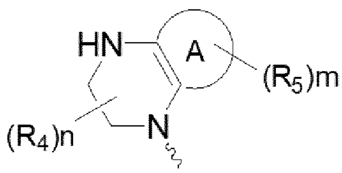

(6)

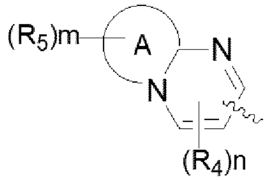

;

(7)

In particular,

$R_4$ is selected from hydrogen, C1-C6 alkyl, C3-C6 cycloalkyl, C1-C6 alkoxy, C1-C6 alkylthio or C1-C6 alkylsulfonyl, wherein the above groups (including the alkyl, cycloalkyl, alkoxy, alkylthio or alkylsulfonyl concerned) are optionally unsubstituted or each independently substituted with 1-4 groups each selected from at least one of halogen, hydroxyl, $C_1$-$C_3$ alkyl, halo $C_1$-$C_3$ alkyl, $C_1$-$C_3$ alkoxy or halo $C_1$-$C_3$ alkoxy;
$R_5$ is selected from hydrogen, halogen, hydroxyl, oxo, C1-C6 alkylacyl, C1-C6 alkylamido, C1-C6 alkoxyimino, C1-C6 alkyl, C2-C6 alkenyl, C2-C6 alkynyl, C3-C6 cycloalkyl, C1-C6 alkoxy, C6-C10 aryl, 5- to 10-membered heteroaryl comprising 1-3 heteroatoms and C1-C6 non-aromatic heterocyclic ring comprising 1-3 heteroatoms, wherein the above groups (including the alkylacyl, alkylamido, alkoxyimino, alkyl, alkenyl, alkynyl, cycloalkyl, alkoxy, aryl, heteroaryl and non-aromatic heterocycloalkyl concerned) are optionally unsubstituted or each independently substituted with 1-4 groups each selected from at least one of halogen, hydroxyl, C1-C3 alkyl, halo C1-C3 alkyl, C1-C3 alkoxy or 3- to 7-membered heterocycloalkyl of C1-C6 comprising 1-3 heteroatoms of N or O; ring A refers to a 5- to 7-membered saturated or partially unsaturated monocyclic group comprising N; m is 1, 2 or 3, and n is 1 or 2; preferably, m is 1 and n is 1; 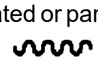 represents a linking site.

$R_1$ is attached to the phenyl group of the compound of formula (1) via 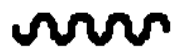.

$R_4$ or $R_5$ is a substituent on the heterocyclic ring, and may be attached to a heteroatom or to a carbon atom.

In the $R_5$ group, the heteroatom is selected from N, O or S.

In some optional embodiments, $R_4$ is selected from hydrogen, methyl, ethyl, *n*-propyl, isopropyl, *n*-butyl, isobutyl, *tert*-butyl, vinyl, ethynyl, cyclopropyl, cyclobutyl, methoxy, ethoxy, *n*-propoxy, $-CH_2-O-CH_3$, methylsulfonyl or ethylsulfonyl, wherein the above groups are optionally unsubstituted or each independently substituted with C1-C3 alkoxy or halogen. Preferably, $R_4$ is selected from H, C1-C3 alkyl, C1-C3 alkoxy-substituted C1-C3 alkyl or C1-C3 alkoxy, most preferably methoxymethyl or methoxy.

In some optional embodiments, $R_5$ is selected from hydrogen, F, Cl, Br, hydroxyl, formyl, acetyl, formamido, acetamido, methoxyimino, ethoxyimino, methyl, ethyl, isopropyl, cyclopropyl, cyclobutyl, cyclopentyl, cyclohexyl, methoxy, ethoxy, phenyl, benzyl, tetrahydrofuryl, hexahydropyranyl, hexahydropyridyl, oxetane, azetidine, wherein the above groups are optionally unsubstituted or each independently substituted with 1-4 groups each selected from at least one of F, Cl, Br, hydroxyl, methoxy, methyl, tetrahydropyrrole, morpholinyl or $-CH_2-CF_3$.

In optional embodiments, $R_5$ is selected from H, methyl, ethyl, isopropyl,

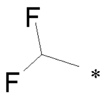,

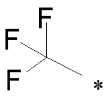, 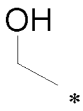, 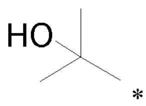, 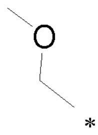,

cyclopropyl, fluorocyclopropyl,

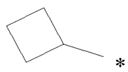,

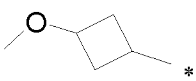, 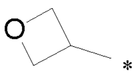, 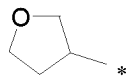, 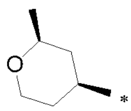, 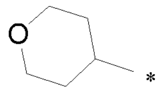, 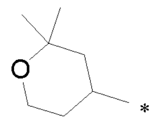, 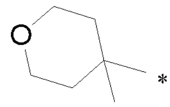,

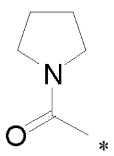, 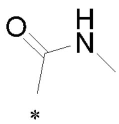, 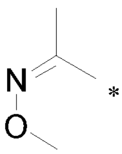, 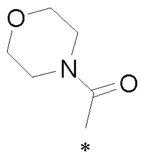, 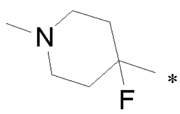, 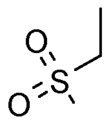, 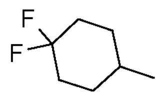 or

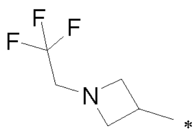.

Optionally, ring A refers to a 5- to 6-membered saturated or unsaturated monocyclic group comprising 1, 2 or 3 ring heteroatoms of N.

In some optional embodiments, when $R_1$ has the structure of formula (2) or (7), ring A has the following structure:

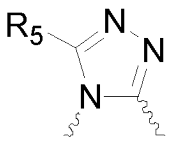, 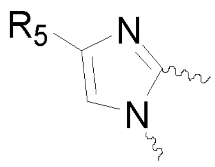, 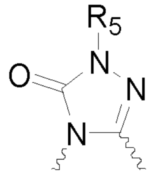, 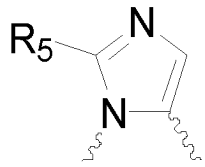, 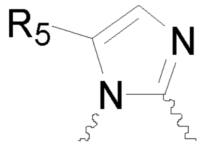 or

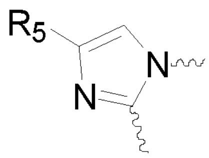;

∿∿∿ represents a linking site; $R_5$ is as defined in any one of the foregoing of the present disclosure.

In some optional embodiments, when $R_1$ has the structure of formula (3), (4), (5) or (6), ring A has the following structure:

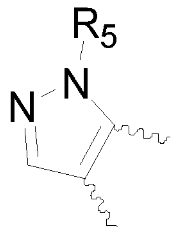, 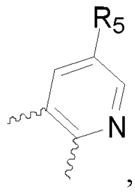, 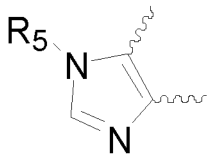 or 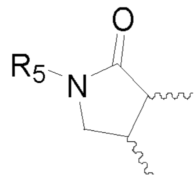;

∿∿∿ represents a linking site; $R_5$ is as defined in any one of the foregoing of the present disclosure.

In the schemes of the present disclosure, optionally, $R_1$ is a substituted or unsubstituted pyridine fused heterocyclic ring; more preferably, $R_1$ is substituted or unsubstituted pyridotriazole or substituted or unsubstituted pyridoimidazole.

As a more preferable scheme, $R_1$ is selected from any one of the following compounds:

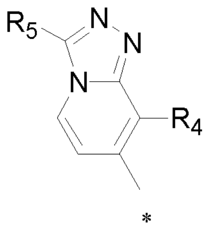, 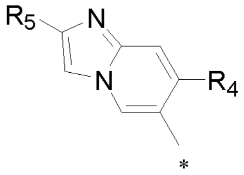, 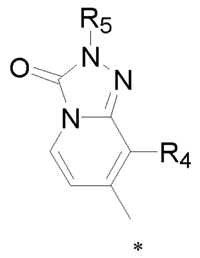, 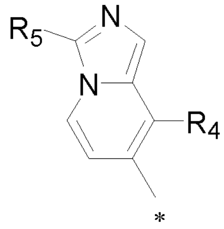, 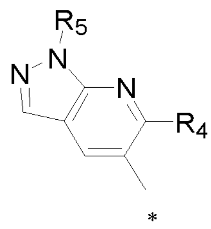,

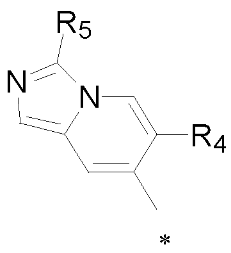, 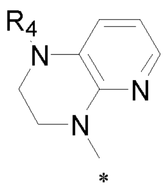, 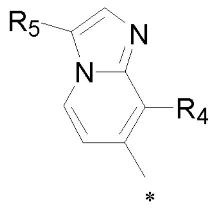, 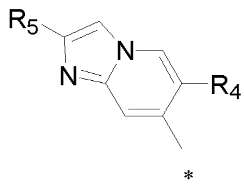, 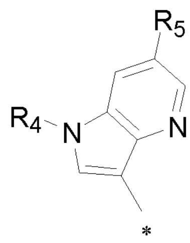,

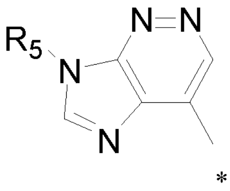 , 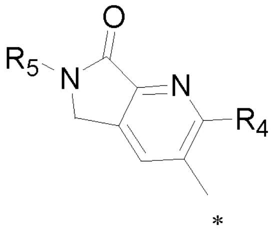 , 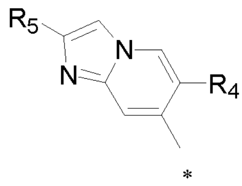 , 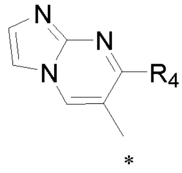 ,

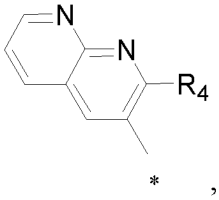 , 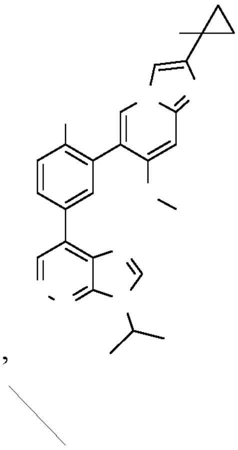 or 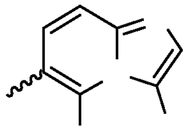 .

Where * represents a linking site.

$R_4$ and $R_5$ are as defined in any one of the foregoing of the present disclosure.

As an optional structure, $R_1$ is selected from any one of the following structures:

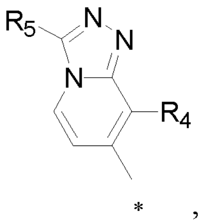 , 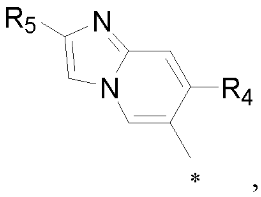 , 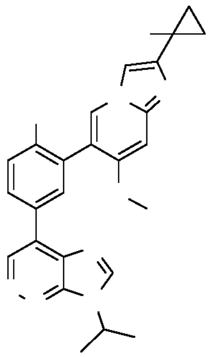 or 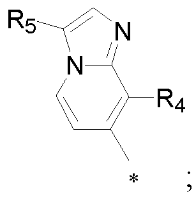 ;

and

$R_4$ is selected from methoxy-C1-C3 alkyl or C1-C3 alkoxy, and is more preferably methoxymethyl or methoxy;

$R_5$ is selected from H, methyl, ethyl, isopropyl,

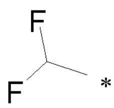 ,

cyclopropyl, fluorocyclopropyl,

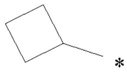, 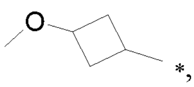, 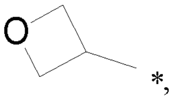, 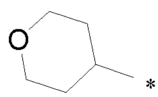 and 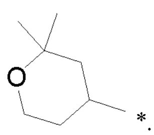.

More preferably, the compound of the present disclosure has the structures shown in Table 1 below.

Table 1 Preferable compound structures

| No. | Structural formula | Chemical name | No. | Structural formula | Chemical name |
|---|---|---|---|---|---|
| 1 | 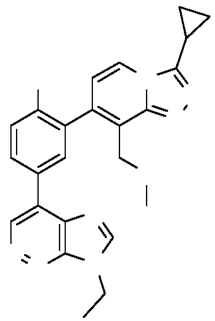 | 4-(3-(3-Cyclopropyl-8-(methoxymethyl)-[1,2,4] triazolo[4,3 -a]pyridin-7-yl)-4-fluorophenyl)-7-ethyl-7H-imidazo[4,5-c]pyridazine | 30 | 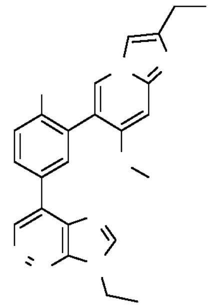 | 7-Ethyl-4-(3-(2-ethyl-7-methoxyimidazo[1,2 -a]pyridin-6-yl)-4-fluorophenyl)-7H-imidazo [4,5-c]pyridazine |
| 2 | 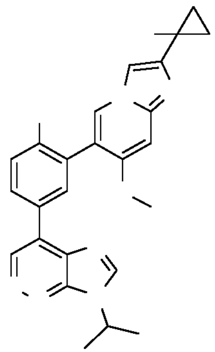 | 7-Isopropyl-4-(4-fluoro-3-(2-(1-fluorocyclopropyl) -7-methoxyimidazo[1 ,2-a]pyridin-6-yl)phenyl)-7H-imidazo[4,5-c]pyridazine | 31 | 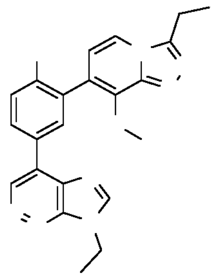 | 7-Ethyl-4-(4-fluoro-3-(8-methoxy-3-ethyl-[1,2,4]triazolo[4,3-a]pyridin-7-yl)phenyl)-7H-imidazo[4,5-c]pyridazine |
| 3 | 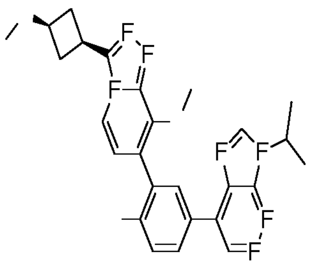 | Rel-4-(4-fluoro-3-(8-methoxy-3-((1s,3s)-3-methoxycyclobuty l)-[1,2,4]triazolo[4,3 -a]pyridin-7-yl)phenyl)-7-isopropyl-7H-imidazo[4,5-c] pyridazine | 32 | 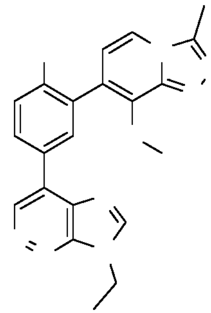 | 7-Ethyl-4-(4-fluoro-3-(8-methoxy-3-methyl-[1,2,4]triazolo[4,3-a]pyridin-7-yl)phenyl)-7H-imidazo[4,5-c]pyridazine |

(continued)

| No. | Structural formula | Chemical name | No. | Structural formula | Chemical name |
|---|---|---|---|---|---|
| 4 | 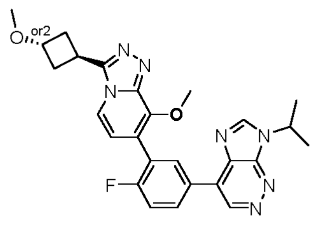 | Rel-4-(4-fluoro-3-(8-methoxy-3-((1r,3r)-3-methoxycyclobuty l)-[1,2,4]triazolo[4,3 -a]pyridin-7-yl)phenyl)-7-isopropyl-7H-imidazo[4,5-c]pyridazine | 33 | 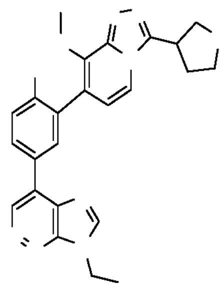 | 7-Ethyl-4-(4-fluoro-3-(8-methoxy-3-(tetrahydrofuran-3-yl)-[1,2,4]triazolo[4,3-a]pyridin-7-yl)phenyl)-7H-imidazo[4,5-c]pyridazine |
| 5 | 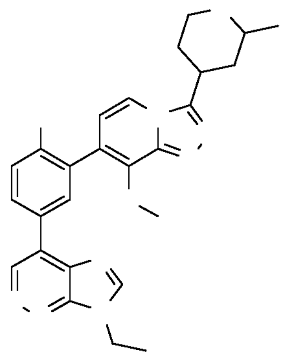 | 7-Ethyl-4-(4-fluoro-3-(8-methoxy-3-(2-methyltetrahydro-2H-pyran-4-yl)-[1,2,4]triazolo[4,3 -a] pyridin-7-yl)phenyl)-7H-imidazo[4,5-c]pyridazine | 34 | 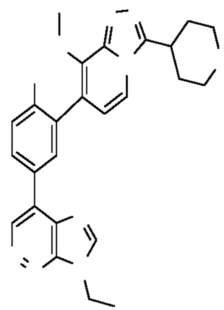 | 7-Ethyl-4-(4-fluoro-3-(8-methoxy-3-(tetrahydro-2H-pyran-4-yl)-[1,2,4]triazolo[4,3-a]pyridin-7-yl)phenyl)-7H-imidazo[4,5-c]pyridazine |
| 6 | 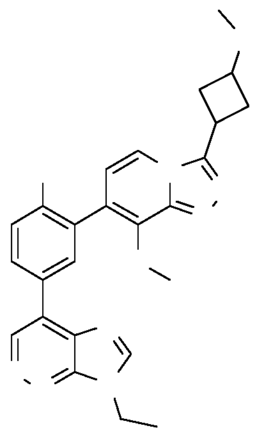 | 7-Ethyl-4-(4-fluoro-3-(8-methoxy-3-(3-methoxycyclobuty l)-[1,2,4]triazolo[4,3 -a]pyridin-7-yl) phenyl)-7H-imidazo[4,5-c]pyridazine | 35 | 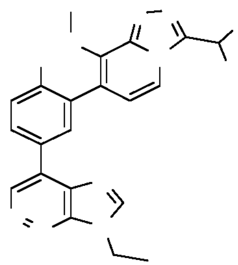 | 4-(3-(3-(Difluoromethyl)-8-methoxy-[1,2,4] triazolo[4,3-a]pyridin-7-yl)-4-fluorophenyl)-7-ethyl-7H-imidazo[4,5-c]pyridazine |

(continued)

| No. | Structural formula | Chemical name | No. | Structural formula | Chemical name |
|---|---|---|---|---|---|
| 7 | 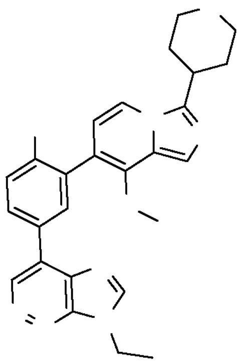 | 7-Ethyl-4-(4-fluoro-3-(8-methoxy-3-(tetrahydro-2H-pyran-4-yl)imidazo[1,5-a]pyridin-7-yl)phenyl)-7H-imidazo[4,5-c]pyridazine | 36 | 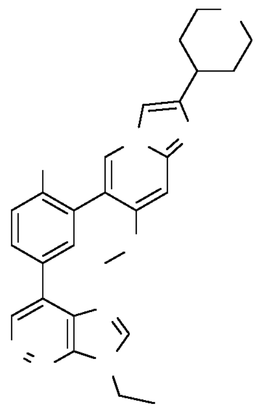 | 7-Ethyl-4-(4-fluoro-3-(7-methoxy-2-(tetrahydro-2H-pyran-4-yl)imidazo[1,2-a]pyridin-6-yl)phenyl)-7H-imidazo[4,5-c]pyridazine |
| 8 | 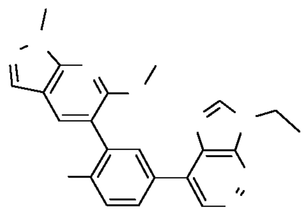 | 7-Ethyl-4-(4-fluoro-3-(6-methoxy-1-methyl-1H-pyrazolo[3,4-b]pyridin-5-yl)phenyl)-7H-imidazo[4,5-c]pyridazine | 37 | 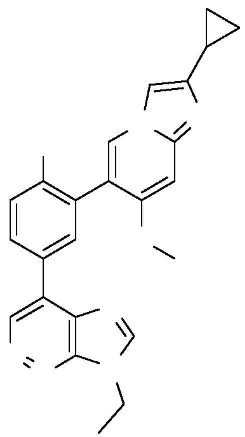 | 4-(3-(2-Cyclopropyl-7-methoxyimidazo[1,2-a]pyridin-6-yl)-4-fluorophenyl)-7-ethyl-7H-imidazo[4,5-c]pyridazine |

(continued)

| No. | Structural formula | Chemical name | No. | Structural formula | Chemical name |
|---|---|---|---|---|---|
| 9 | 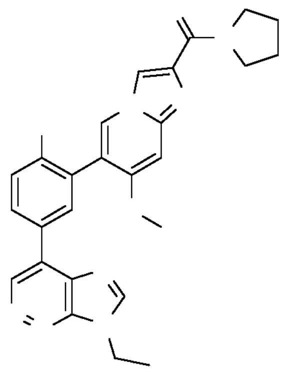 | (6-(5-(7-Ethyl-7H-imidazo[4,5-c]pyridazin-4-yl)-2-fluorophenyl)-7-methoxyimidazo[1 ,2-a] pyridin-2-yl)(pyrrolidin-1-yl)methanone | 38 | 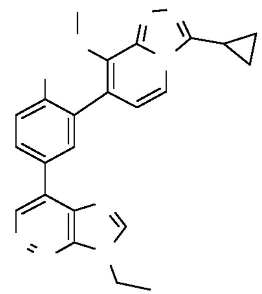 | 4-(3-(3-Cyclopropyl-8-methoxy-[1,2,4]triazolo[4,3-a]pyridin-7-yl)-4-fluorophenyl)-7-ethyl-7H-imidazo[4,5-c]pyridazine |
| 10 | 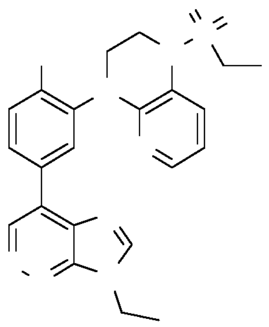 | 4-(5-(7-Ethyl-7H-imidazo[4,5-c]pyridazin-4-yl)-2-fluorophenyl)-1-(ethylsulfonyl)-1,2,3,4-tetrahydropyrido[2 ,3-b]pyrazine | 39 | 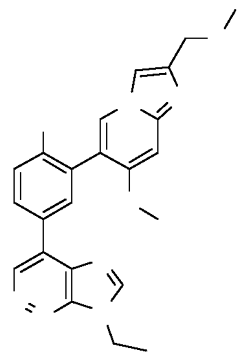 | 7-Ethyl-4-(4-fluoro-3-(7-methoxy-2-(methoxymethyl)imi dazo[1,2-a]pyridin-6-yl)phenyl)-7H-imidazo[4,5-c]pyridazine |
| 11 | 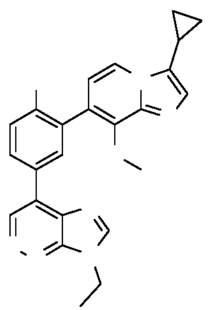 | 4-(3-(3-Cyclopropyl-8-methoxyimidazo[1 ,2-a] pyridin-7-yl)-4-fluorophenyl)-7-ethyl-7H-imidazo [4,5-c]pyridazine | 40 | 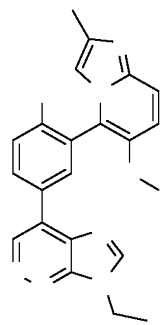 | 7-Ethyl-4-(4-fluoro-3-(6-methoxy-2-methylimidazo[1,2-a]pyridin-5-yl)phenyl)-7H-imidazo[4,5-c]pyridazine |

(continued)

| No. | Structural formula | Chemical name | No. | Structural formula | Chemical name |
|---|---|---|---|---|---|
| 12 | 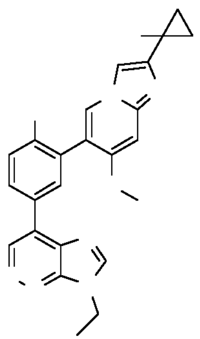 | 7-Ethyl-4-(4-fluoro-3-(2-(1-fluorocyclopropyl) -7-methoxyimidazo[1 ,2-a]pyridin-6-yl)phenyl)-7H-imidazo[4, 5-c]pyridazine | 41 | 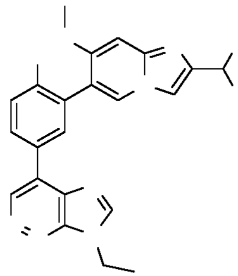 | 4-(3-(2-(Difluoromethyl)-7-methoxyimidazo [1,2 -a]pyridin-6-yl)-4-fluorophenyl)-7-ethyl-7H-imidazo[4,5-c]pyridazine |
| 13 | 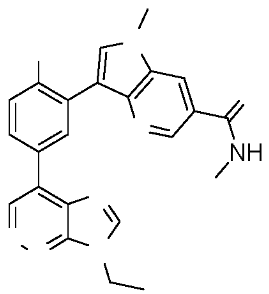 | 3-(5-(7-Ethyl-7H-imidazo[4,5-c]pyridazin-4-yl)-2-fluorophenyl)-N,1-dimethyl-1H-pyrrolo [3,2-b]pyridine-6-carboxamide | 42 | 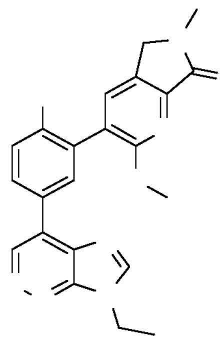 | 3-(5-(7-Ethyl-7H-imidazo[4,5-c]pyridazin-4-yl)-2-fluorophenyl)-2-methoxy-6-methyl-5,6-dihydro-7H-pyrrolo[3,4-b]pyridin-7-one |
| 14 | 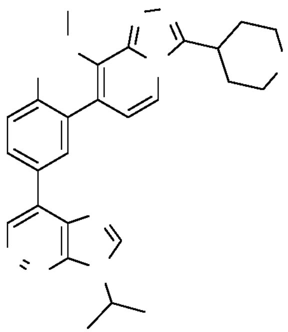 | 4-(4-Fluoro-3-(8-methoxy-3-(tetrahydro-2H-pyran-4-yl)-[1,2,4]tri azolo[4,3 -a]pyridin-7-yl)phenyl)-7-isopropyl-7H-imidazo[4,5-c]pyridazine | 43 | 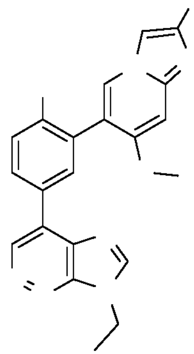 | 7-Ethyl-4-(4-fluoro-3-(7-methoxy-2-(trifluoromethyl)imi dazo[1,2-a]pyridin-6-yl)phenyl)-7H-imidazo[4,5-c]pyridazine |

(continued)

| No. | Structural formula | Chemical name | No. | Structural formula | Chemical name |
|---|---|---|---|---|---|
| 15 | 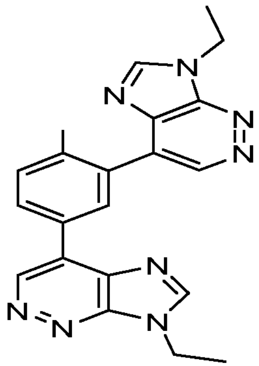 | 4,4'-(4-Fluoro-1,3-phenylene)bis(7-ethyl-7H-imidazo[4,5-c]pyridazine) | 44 | 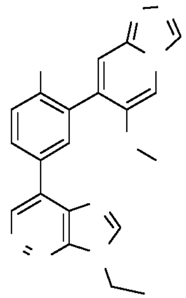 | 7-Ethyl-4-(4-fluoro-3-(6-methoxy-[1,2,4]triazolo[4,3-a]pyridin-7-yl)phenyl)-7H-imidazo [4,5-c]pyridazine |
| 16 | 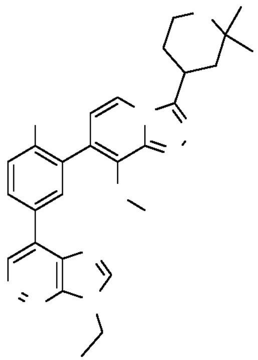 | 4-(3-(3-(2,2-Dimethyltetrahydr o-2H-pyran-4-yl)-8-methoxy-[1,2,4]triazolo[4,3 -a]pyridin-7-yl)-4-fluorophenyl)-7-ethyl-7H-imidazo[4,5-c] pyridazine | 45 | 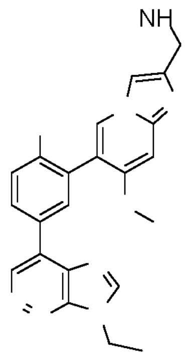 | (6-(5-(7-Ethyl-7H-imidazo[4,5-c]pyridazin-4-yl)-2-fluorophenyl)-7-methoxyimidazo[1,2 -a]pyridin-2-yl)methanol |

(continued)

| No. | Structural formula | Chemical name | No. | Structural formula | Chemical name |
|---|---|---|---|---|---|
| 17 | 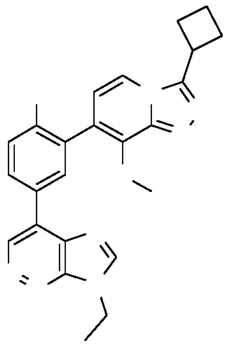 | 4-(3-(3-Cyclobutyl-8-methoxy-[1,2,4]triazolo[4,3 -a]pyridin-7-yl)-4-fluorophenyl)-7-ethyl-7H-imidazo[4,5-c]pyridazine | 46 | 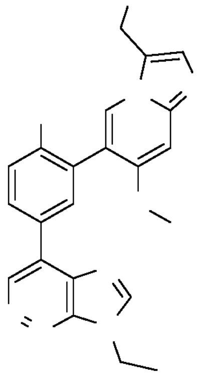 | (6-(5-(7-Ethyl-7H-imidazo[4,5-c]pyridazin-4-yl)-2-fluorophenyl)-7-methoxyimidazo[1,2 -a]pyridin-3-yl)methanol |
| 18 | 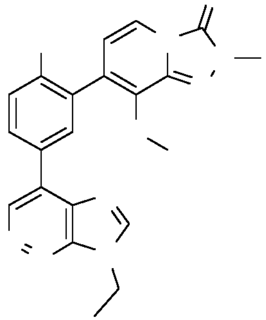 | 7-(5-(7-Ethyl-7H-imidazo[4,5-c]pyridazin-4-yl)-2-fluorophenyl)-8-methoxy-2-methyl-[1,2,4] triazolo[4,3 -a]pyridin-3(2H)-one | 47 | 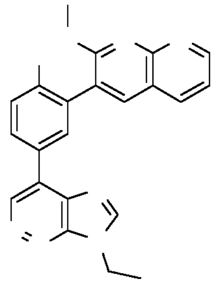 | 3-(5-(7-Ethyl-7H-imidazo[4,5-c]pyridazin-4-yl)-2-fluorophenyl)-2-methoxy-1,8-naphthyridine |
| 19 | 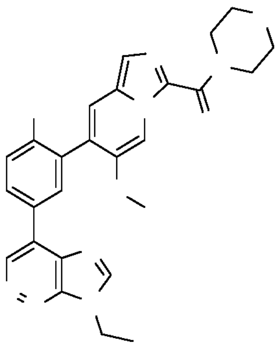 | (7-(5-(7-Ethyl-7H-imidazo[4,5-c]pyridazin-4-yl)-2-fluorophenyl)-6-methoxyimidazo[1 ,5-a] pyridin-3-yl)(morpholino)m ethanone | 48 | 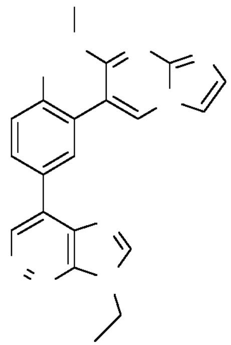 | 7-Ethyl-4-(4-fluoro-3-(7-methoxyimidazo[1,2 -a]pyrimidin-6-yl)phenyl)-7H-imidazo[4,5-c] pyridazine |

(continued)

| No. | Structural formula | Chemical name | No. | Structural formula | Chemical name |
|---|---|---|---|---|---|
| 20 | 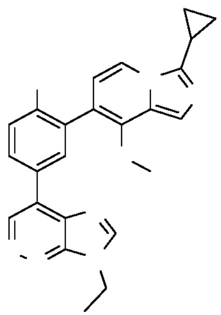 | 4-(3-(3-Cyclopropyl-8-methoxyimidazo[1 ,5-a] pyridin-7-yl)-4-fluorophenyl)-7-ethyl-7H-imidazo [4,5- | 49 | 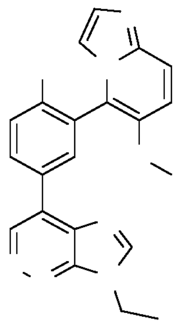 | 7-Ethyl-4-(4-fluoro-3-(6-methoxyimidazo[1,2 -a]pyridin-5-yl)phenyl)-7H-imidazo[4,5-c] pyridazine |
| | | c]pyridazine | | | |
| 21 | 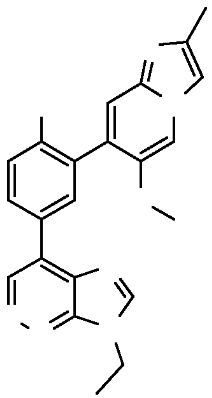 | 7-Ethyl-4-(4-fluoro-3-(6-methoxy-2-methylimidazo[1,2 -a]pyridin-7-yl)phenyl)-7H-imidazo [4,5-c]pyridazine | 50 | 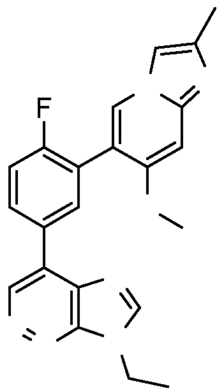 | 7-Ethyl-4-(4-fluoro-3-(7-methoxy-2-methylimidazo[1,2-a]pyridin-6-yl)phenyl)-7H-imidazo[4,5-c]pyridazine |

(continued)

| No. | Structural formula | Chemical name | No. | Structural formula | Chemical name |
|---|---|---|---|---|---|
| 22 | 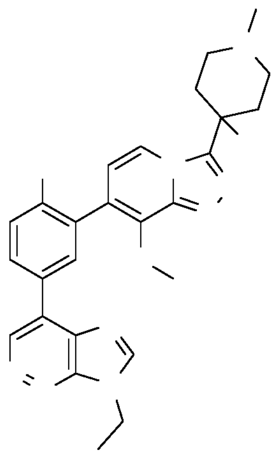 | 7-Ethyl-4-(4-fluoro-3-(3-(4-fluoro-1-methylpiperidin-4-yl)-8-methoxy-[1,2,4]triazolo[4,3 -a]pyridin-7-yl)phenyl)-7H-imidazo[4,5-c]pyridazine | 51 | 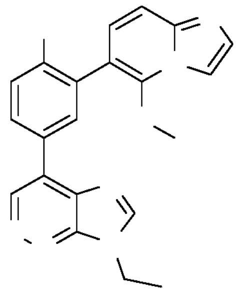 | 7-Ethyl-4-(4-fluoro-3-(5-methoxyimidazo[1,2 -a]pyridin-6-yl)phenyl)-7H-imidazo[4,5-c] pyridazine |
| 23 | 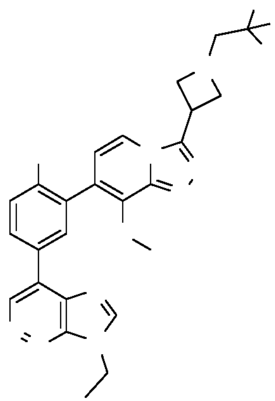 | 7-Ethyl-4-(4-fluoro-3-(8-methoxy-3-(1-(2,2,2-trifluoroethyl)azeti din-3-yl)-[1,2,4]tri azolo[4,3 -a] pyridin-7-yl)phenyl)-7H-imidazo[4,5-c]pyridazine | 52 | 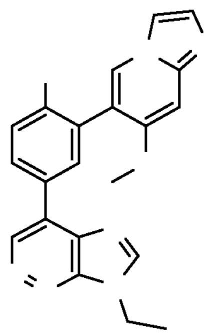 | 7-Ethyl-4-(4-fluoro-3-(7-methoxyimidazo[1,2 -a]pyridin-6-yl)phenyl)-7H-imidazo[4, 5-c] pyridazine |

(continued)

| No. | Structural formula | Chemical name | No. | Structural formula | Chemical name |
|---|---|---|---|---|---|
| 24 | 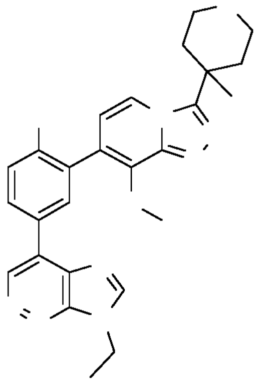 | 7-Ethyl-4-(4-fluoro-3-(8-methoxy-3-(4-methyltetrahydro-2H-pyran-4-yl)-[1,2,4]triazolo[4,3 -a] pyridin-7-yl)phenyl)-7H-imidazo[4,5-c]pyridazine | 53 | 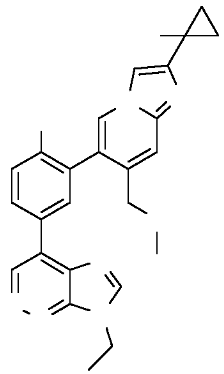 | 7-Ethyl-4-(4-fluoro-3-(2-(1-fluorocyclopropyl)-7-(methoxymethyl)imi dazo[1,2-a]pyridin-6-yl)phenyl)-7H-imidazo[4,5-c]pyridazine |
| 25 | 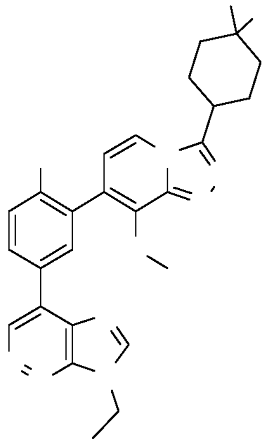 | 4-(3-(3-(4,4-Difluorocyclohexy l)-8-methoxy-[1,2,4]triazolo[4,3 -a]pyridin-7-yl)-4-fluorophenyl)-7-ethyl-7H-imidazo[4,5-c]pyridazine | 54 | 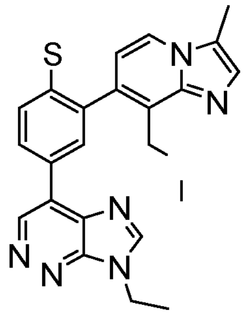 | 7-Ethyl-4-(4-fluoro-3-(8-(methoxymethyl)-3-methylimidazo[1,2-a]pyridin-7-yl)phenyl)-7H-imidazo[4,5-c]pyridazine |

(continued)

| No. | Structural formula | Chemical name | No. | Structural formula | Chemical name |
|---|---|---|---|---|---|
| 26 | 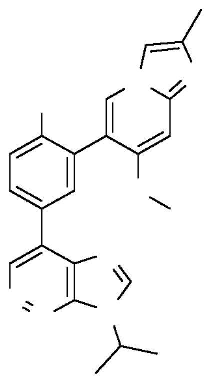 | 4-(4-Fluoro-3-(7-methoxy-2-methylimidazo[1,2 -a]pyridin-6-yl)phenyl)-7-isopropyl-7H-imidazo [4,5-c]pyridazine | 55 | 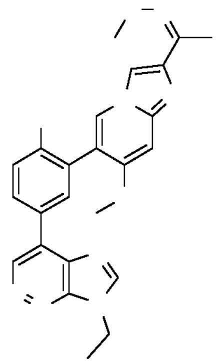 | (Z)-1-(6-(5-(7-Ethyl-7H-imidazo[4,5-c]pyridazin-4-yl)-2-fluorophenyl)-7-methoxyimidazo[1,2 -a]pyridin-2-yl)ethan-1-one O-methyl oxime |
| 27 | 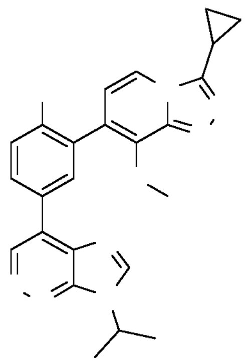 | 4-(3-(3-Cyclopropyl-8-methoxy-[1,2,4]triazolo [4,3 -a]pyridin-7-yl)-4-fluorophenyl)-7-isopropyl-7H-imidazo[4,5-c]pyridazine | 56 | 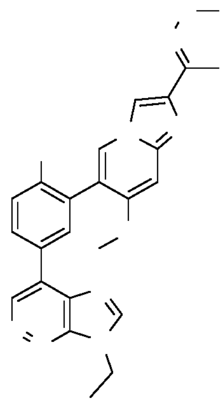 | (E)-1-(6-(5-(7-Ethyl-7H-imidazo[4,5-c]pyridazin-4-yl)-2-fluorophenyl)-7-methoxyimidazo[1,2 -a]pyridin-2-yl)ethan-1-one O-methyl oxime |

(continued)

| No. | Structural formula | Chemical name | No. | Structural formula | Chemical name |
|---|---|---|---|---|---|
| 28 | 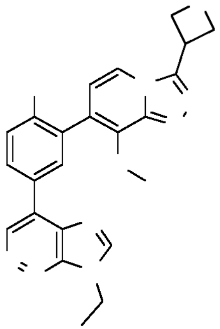 | 7-Ethyl-4-(4-fluoro-3-(8-methoxy-3-(oxetan-3 -yl)-[1,2,4]triazolo[4,3 -a]pyridin-7-yl)phenyl)-7H-imidazo[4, 5-c]pyridazine | 57 | 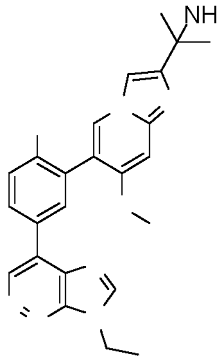 | 2-(6-(5-(7-Ethyl-7H-imidazo[4,5-c]pyridazin-4-yl)-2-fluorophenyl)-7-methoxyimidazo [1,2 -a]pyridin-2-yl)propan-2-ol |
| 29 | 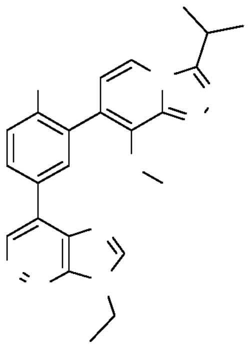 | 7-Ethyl-4-(4-fluoro-3-(8-methoxy-3-isopropyl-[1,2,4]tri azolo[4,3 -a]pyridin-7-yl)phenyl)-7H-imidazo[4,5-c]pyridazine | 58 | 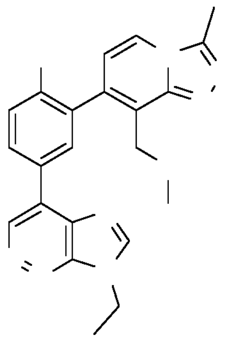 | 7-Ethyl-4-(4-fluoro-3-(8-(methoxymethyl)-3-methyl-[1,2,4]tri azolo[4,3-a]pyridin-7-yl) phenyl)-7H-imidazo[4,5-c]pyridazine |

The present disclosure further provides a pharmaceutical mixture, wherein the pharmaceutical mixture comprises two or more compounds selected from the group consisting of: the compound or the stereoisomer thereof, the tautomer thereof, the prodrug thereof, the pharmaceutically acceptable salt thereof, the amorphous material thereof, the isotopic variant thereof, the polymorph thereof or the solvate thereof according to any one aspect of the present disclosure.

The present disclosure further provides a pharmaceutical composition, wherein the pharmaceutical composition comprises at least one of the compounds or the stereoisomers thereof, the tautomers thereof, the prodrugs thereof, the pharmaceutically acceptable salts thereof, the amorphous materials thereof, the isotopic variants thereof, the polymorphs thereof or the solvates thereof according to any one aspect of the present disclosure, and optionally supplemented with pharmaceutically acceptable carriers and/or adjuvants.

Provided is use of the compound or the stereoisomer thereof, the tautomer thereof, the prodrug thereof, the pharmaceutically acceptable salt thereof, the amorphous material thereof, the polymorph thereof, the solvate thereof, the pharmaceutical mixture or the pharmaceutical composition according to any one aspect of the present disclosure in the manufacture of a medicament for treating or preventing a $GABA_A$ receptor-associated disease.

Further, the $GABA_A$ receptor-associated disease is selected from at least one of the following: pain, Alzheimer's disease, multi-infarct dementia and stroke.

Further, the pain is neuropathic pain, inflammatory pain and cancerous pain.

Further, the pain is selected from: headache, facial pain, neck pain, shoulder pain, back pain, chest pain, abdominal pain, dorsalgia, low back pain, lower limb pain, musculoskeletal pain, vascular pain, gout, arthritis pain, visceral pain, pain due to infectious diseases, bony pain, pain associated with sickle cell anemia, autoimmune diseases, multiple sclerosis or inflammation, chronic pain caused by injury or surgery, nociceptive pain, painful diabetes, trigeminal neuralgia, pain of lumbar or cervical radiculopathy, glossopharyngeal neuralgia, autonomic neuroreflex pain, and pain associated with reflex sympathetic dystrophy, nerve root avulsion, cancer, chemical injury, toxin, nutritional deficiencies, viral or bacterial infection or degenerative osteoarthropathy.

The present disclosure further provides a method for treating or preventing the $GABA_A$ receptor-associated disease, wherein the method comprises administering to a patient an effective amount of the compound or the stereoisomer thereof, the tautomer thereof, the prodrug thereof, the pharmaceutically acceptable salt thereof, the amorphous material thereof, the polymorph thereof, the solvate thereof, the pharmaceutical mixture or the pharmaceutical composition according to any one aspect of the present disclosure.

The present disclosure further provides a method for treating or preventing pain, Alzheimer's disease, multi-infarct dementia or stroke, wherein the method comprises administering to a patient an effective amount of the compound or the stereoisomer thereof, the tautomer thereof, the prodrug thereof, the pharmaceutically acceptable salt thereof, the amorphous material thereof, the polymorph thereof, the solvate thereof, the pharmaceutical mixture or the pharmaceutical composition according to any one aspect of the present disclosure.

The present disclosure further relates to a preparation method for the compound of formula (1) as described above, wherein the method comprises:

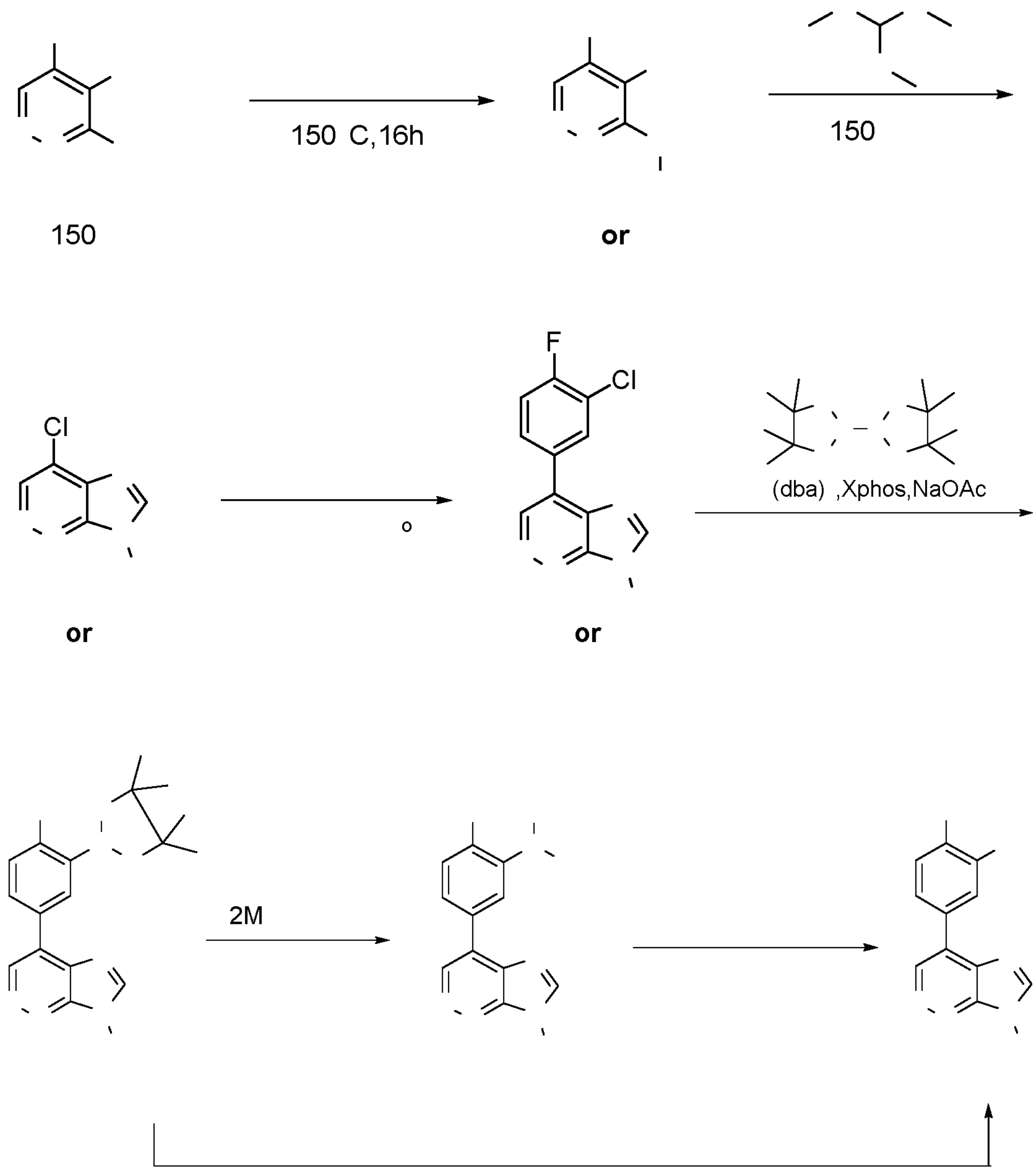


Note: $R_3$ is ethyl or isopropyl for which the experiment process is the same.

**Z-1a: 5-Chloro-N3-ethylpyridazine-3,4-diamine**

3,5-Dichloropyridazin-4-amine (80.5 g, 0.5 mol) and an ethylamine ethanol solution (30%, 600 mL) were placed in an autoclave, and the mixture was allowed to react at 150 °C for 16 h. The reaction mixture was cooled to room temperature, and a solid was participated. The mixture was filtered, and the filter cake was washed with dichloromethane (300 mL). The mother liquor was concentrated (without evaporation), and a solid was participated. The mixture was filtered again, and this operation was repeated 3 times. All the filter cakes were collected to obtain a crude product (containing ethylamine hydrochloride). The crude product was stirred and slurried with water, and the mixture was filtered. The filter cake was concentrated to dryness by rotary evaporation to obtain 65 g of the target product (yield: 76.5%) as a pale yellow solid. LC-MS: m/z $[M+H]^+$ = 173.

**Z-2a: 4-Chloro-7-ethyl-7H-imidazo[4,5-c]pyridazine**

5-Chloro-N3-ethylpyridazine-3,4-diamine (30 g, 0.17 mol) was added to trimethyl orthoformate (600 ml). The mixture was allowed to react at 120 °C for 4 h. The reaction mixture was directly concentrated to dryness by rotary evaporation to obtain a crude product. The crude product was dissolved by adding dichloromethane and methanol, and the

mixture was stirred and then subjected to column chromatography (dichloromethane, dichloromethane/methanol = 50/1, V/V) to obtain 20 g of the target product (yield: 63%) as a yellow solid. LC-MS: m/z $[M+H]^+$ = 183.

**Z-3a: 4-(3-Chloro-4-fluorophenyl)-7-ethyl-7H-imidazo[4,5-c]pyridazine**

4-Chloro-7-ethyl-7H-imidazo[4,5-c]pyridazine (20 g,108 mmol), (3-chloro-4-fluorophenyl)boronic acid (19.08 g, 108 mmol), sodium carbonate (24 g, 216 mmol) and [1,1'-bis(diphenylphosphino)ferrocene]palladium dichloride dichloromethane complex (9.2 g, 10.8 mmol) were added to 1,4-dioxane/water (160 ml/40 ml), and the mixture was allowed to react at 90 °C for 2 h. The reaction mixture was purified directly by column chromatography (petroleum ether/ethyl acetate = 10/1, 5/1, or 1/1, ethyl acetate, V/V) to obtain the title compound (26 g, yield: 85.7%) as a yellow solid. LC-MS: m/z $[M+H]^+$ = 277.

**Z-4a-1: 7-Ethyl-4-(4-fluoro-3-(4,4,5,5-tetramethyl-1,3,2-dioxaborolan-2-yl)phenyl)-7H-imidazo [4,5-c] pyridazine**

4-(3-Chloro-4-fluorophenyl)-7-ethyl-7H-imidazo[4,5-c]pyridazine (15 g, 54 mmol), bis(pinacolato)diboron (27.6 g, 108 mmol), sodium acetate (13 g, 163 mmol), 2-dicyclohexylphosphino-2,4,6-triisopropylbiphenyl (2.7 g, 5.4 mmol) and tris(dibenzylideneacetone)dipalladium (5 g, 5.4 mmol) were added to anhydrous 1,4-dioxane (150 ml), and the mixture was stirred at 110 °C overnight. The reaction mixture was purified directly by column chromatography (dichloromethane/anhydrous methanol = 50/1) to obtain the title compound (12 g, yield: 60%) as a pale yellow solid. $^1H$ NMR (400 MHz, CHLOROFORM-*d*) ppm 1.40 (s, 12 H) 1.69 (t, *J* = 7.09 Hz, 3 H) 4.58 (q, *J* = 7.34 Hz, 2 H) 7.26 (d, *J* = 6.36 Hz, 1 H) 8.29 (s, 1 H) 8.45 (d, *J* = 1.96 Hz, 2 H) 9.39 (s, 1 H). LC-MS: m/z $[M+H]^+$ = 287, 369.

**Z-4a-2: (5-(7-Ethyl-7H-imidazo[4,5-c]pyridazin-4-yl)-2-fluorophenyl)boronic acid**

7-Ethyl-4-(4-fluoro-3-(4,4,5,5-tetramethyl-1,3,2-dioxaborolan-2-yl)phenyl)-7H-imidazo[4,5-c]pyridazine (12 g, 32.5 mmol) was dissolved in a 200 mL aqueous hydrochloric acid solution (2 M), and the resulting solution was stirred at room temperature for 2 h, and lyophilized to obtain the title compound (9.3 g, crude product) as a white solid.

The compound of formula (1) and the pharmaceutically acceptable salt thereof of the present disclosure may be prepared by the above method.

If the preparation method is not described in the examples, the compound of formula (1) and the intermediate product thereof may be prepared according to a similar method or according to the aforementioned method. Starting materials known in the art may be obtained commercially, or may be prepared according to known methods in the art or methods similar to the known methods.

It should be understood that the compound of formula (1) of the present disclosure may be derivatized at functional groups to obtain derivatives capable of being reconverted *in vivo* to the parent compound.

The present disclosure therefore further relates to a pharmaceutically acceptable composition, wherein the pharmaceutically acceptable composition comprises the compound or the pharmaceutically acceptable salt thereof or the prodrug thereof and the pharmaceutically acceptable carrier and/or the adjuvant as defined above.

Similarly, the present disclosure further comprises use of the compound or the composition as described above in the manufacture of a medicament for treating or preventing an $\alpha2/3\text{-GABA}_A$ receptor-associated disease, in particular for treating or preventing the following diseases: pain, epilepsy, anxiety, pruritus and depression.

Preferably, pain is treated or prevented.

Particularly preferably, neuropathic pain, inflammatory pain and cancerous pain are treated or prevented.

As used herein, the term "cancerous pain" refers to pain that occurs during the development of a malignant tumor, and it is currently believed that there are three mechanisms by which cancerous pain occurs, namely: pain directly from cancer development, pain following cancer treatment and painful disease associated with cancer patients.

As used herein, the term "neuropathic pain" refers to pain that is provoked or caused by a primary lesion and dysfunction of the nervous system.

As used herein, the term "inflammatory pain" refers to pain caused by regional acute inflammation or by chronic inflammation that irritates nerves.

As used herein, the term "treating" also includes prophylactic administration, to alleviate or eliminate the condition once the condition is established.

As used herein, the term "patient" is defined as any warm-blooded animal, such as, but not limited to, mice, guinea pigs, dogs, horses or humans, and the patient is preferably a human.

As used herein, the term "acute pain" is defined as pain caused by harmful stimuli resulting from injury and/or disease of the skin, body structures or viscera, or caused by abnormal functioning of muscles or viscera, which do not produce actual tissue damage.

As used herein, the term "chronic pain" is defined as pain that persists beyond the usual course of an acute

disease or a reasonable time for injury to heal, or that is associated with a chronic pathological process inducing continuous pain, or that recurs at regular intervals for months or years. Pain is considered chronic if it persists after a cure should have been achieved or beyond the usual course of treatment. The length of time for pain to elapse depends on the nature of the pain and the course of treatment associated with the pain, and the pain is chronic if it persists beyond the usual course of treatment. The chronic pain includes, but is not limited to, headache, facial pain, neck pain, shoulder pain, chest pain, abdominal pain, dorsalgia, low back pain, lower limb pain, musculoskeletal pain, pain associated with somatoform disorders, visceral pain, painful diabetic neuropathy, vascular pain, gout, arthritic pain, cancerous pain, autonomic neuroreflex pain, pain due to infectious diseases (e.g., AIDS and shingles), pain due to autoimmune diseases (rheumatism), pain due to acute and chronic inflammation, post-operative pain and post-burn pain.

The medicaments disclosed in the present disclosure are effective in treating chronic pain as defined above, and the medicaments disclosed in the present disclosure may be used to treat hyperalgesia associated with other conditions including hyperalgesia, allodynia, enhanced algesia and enhanced pain memory, for which the application will improve the treatment of pain.

As used herein, the term "headache" may be divided into primary headaches, including tension headaches, migraine headaches and cluster headaches, and secondary headaches caused by other diseases. Various headaches may be caused when pain-sensitive tissues of the head and face are diseased or stimulated. These pain-sensitive tissues include those distributed in the scalp, face, oral cavity and throat, etc. Since these tissues are mainly muscles or blood vessels in the head and contain abundant nerve fibers, and are more sensitive to pain, so headaches may be caused when they are injured.

As used herein, the term "facial pain" includes, but is not limited to, trigeminal neuralgia, atypical facial pain, facial paralysis and facial spasm.

As used herein, the term "trigeminal neuralgia" refers to a unique chronic painful disease, also known as trismus dolorificus, which is characterized by transient, paroxysmal and recurrent electrical shock-like severe pain in the trigeminal nerve distribution region, or with ipsilateral facial spasm. The trigeminal neuralgia is divided into primary trigeminal neuralgia and secondary trigeminal neuralgia, wherein the primary trigeminal neuralgia is characterized in that no nervous system sign is found clinically and no organic lesion is found in the examination; the secondary trigeminal neuralgia is characterized by the clinical presence of nervous system signs and organic lesions, e.g., tumors and inflammations.

As used herein, the term "atypical facial pain" refers to pain caused by a variety of etiologies. The atypical facial pain is characterized by persistent burning-like pain, no intermittency, no relation to specific movements or triggering stimuli, and is mostly bilateral and often extends beyond the distribution of the trigeminal nerve and even to the skin of the neck. The pain may be caused by irritation or injury of the trigeminal nerve due to nasosinusitis, malignant tumor, infection of the jaw and skull base, etc.

As used herein, the term "neck pain, back pain and shoulder pain" refers to pain due to acute or chronic muscle strain, degeneration and trauma of bone joints, etc. Common diseases causing neck, shoulder and upper limb pain include neck and shoulder myofascitis, ligamentum nuchae inflammation, cervical spondylosis, scapulohumeral periarthritis, thoracic outlet syndrome, external humeral epicondylitis, etc., or the pain caused by autoimmune diseases is common in diseases such as polyarthritis destruens, ankylosing spondylitis and rheumatoid arthritis. Other diseases which may cause the neck pain, back pain and shoulder pain also include neck and shoulder tumors, neuritis, arteriovenous diseases and various infections, referred pain caused by pathological changes of thoracic organs and abdominal organs, etc.

As used herein, the term "chest pain, abdominal pain and dorsalgia" refers to pain due to diseases of the thoracic and abdominal viscera and tissues of the thoracic and abdominal walls, including but not limited to, intercostal neuralgia, intercostal chondritis, angina pectoris, abdominal pain (acute abdominal visceral pain) and myofascial syndrome of the low back.

As used herein, the term "low back and lower limb pain" refers to lower back, lumbosacral, sacroiliac, hip, buttocks and lower limb pain. The low back and lower limb pain is not an independent disease but a common feature of multiple diseases, with various clinical manifestations and very complex etiologies, of which degeneration and injury are the most common, including but not limited to, pain related to lumbar disc herniation, acute lumbar sprain, sciatica, osteoporosis, third lumbar transverse process syndrome, piriformis syndrome, knee osteoarthritis, coccygodynia, heel pain, etc.

As used herein, the term "musculoskeletal pain" includes, but is not limited to, myofascial pain, trauma induced pain and chronic regional pain syndrome.

As used herein, the term "painful diabetes" refers to pain caused by nerve injury complicated by diabetes, in which the nerve injury in diabetes is at least partially caused by reduced blood flow and hyperglycemia. Some diabetics do not develop neuropathy, while other patients develop the disease early. Diabetic neuropathy pain may be classified into mononeuropathies and generalized polyneuropathy involving one or more focal sites, of which the polyneuropathy may be diffuse and symmetrical, usually involving mainly sensory modalities (Merrit's Textbook of Neurology, 9th edition, edited by LPRowland LP). Manifestations of diabetic neuropathy may include vegetative nerve functional disturbance, leading to dysregulation of heart, smooth muscle, glands, etc., resulting in hypotension, diarrhea, constipation and impotence.

Diabetic neuropathy tends to develop in stages. It occurs in the nerve ending region during the early stage, then in the feet during the vegetative nerve functional disturbance or sensory neuropathy stage, and then in the face and around the eyes during the cerebral neuropathy stage, with intermittent pain and tingling sensations. In the subsequent stages, the pain becomes stronger and more frequent. Finally, when the pain is lost in a certain region, painless neuropathy occurs, which in the absence of the pain as an indication of injury, greatly increases the risk of severe tissue injury.

As used herein, the term "visceral pain" includes, but is not limited to, pain due to irritable bowel syndrome (IBS), chronic fatigue syndrome (CFS) (with or without), inflammatory bowel disease (IBD) and interstitial cystitis.

As used herein, the term "vascular pain" refers to pain resulting from one or more of the following factors. The first factor is the improper perfusion of tissues, which causes transient or continuous ischemia. For example, ischemia occurs in limb muscles during exercise; the second factor is delayed changes, for example, ulcers or gangrene in the skin or abdominal viscera; the third factor is sudden or accelerated changes in the caliber of large vessels, for example, changes due to aneurysms; the fourth factor is the rupture of the aorta, which results in blood extravasation and stimulation of nociceptive fibers in the peritoneum or parietal pleura; the fifth factor is strong spasm caused by severe stimulation of the arterial endothelium by intra-arterial injection; the sixth factor is damage from venous return, which results in massive edema that rapidly dilates the fascial septa (Bonica et al, The Management of Pain, vol. I (2nd edition), Philadelphia; Lea&Feboger, 1990). The examples include, but are not limited to, arteriosclerosis obliterans, thromboangiitis obliterans, acute arterial closure, embolism, congenital arteriovenous aneurysm, vasospastic diseases, Rayaud's disease, acrocyanosis, acute venous closure, thrombophlebitis, varicose veins and lymphedema.

As used herein, the term "autonomic neuroreflex pain" refers to pain due to the "reflex sympathetic dystrophy syndrome". The reflex sympathetic dystrophy syndrome is characterized by severe spontaneous pain after acute and chronic injuries of the body with hyperaphia and hyperalgesia, which may be accompanied by edema and blood circulation disorders. Then, symptoms such as skin and musculoskeletal nutrition disorders and atrophy may appear.

As used herein, the term "post-operative pain" refers to a complex physiological reaction of the body to the disease itself and to the tissue injury resulting from surgery, which is manifested as a psychologically and behaviorally unpleasant experience.

As used herein, the term "arthritic pain" includes, but is not limited to, pain due to diseases such as osteoarthritis, polyarthritis destruens, spondylitis rhizomelica, psoriatic arthropathy, gout, pseudogout, infectious arthritis, tendonitis, bursitis, bone injury and soft tissue inflammation of joints.

As used herein, the term "postherpetic neuralgia" refers to long-standing severe pain beneath the skin in the original rash region after the healing of the rash of shingles.

As used herein, the term "nociceptive pain" refers to pain caused by a process of tissue damage which is transmitted by nociceptors upon receiving stimulation, or pain caused by prolonged excitation of nociceptors. The pain caused by prolonged excitation of nociceptors may be due to persistent noxious stimulation of nociceptors or sensitization of nociceptors or both, or the pain may be caused by these factors and prolonged by the persistence and various reflex mechanisms of these factors, as well as other factors.

The present disclosure provides use of a pharmaceutical compound comprising a therapeutically effective amount of an $\alpha$2/3-$GABA_A$ positive allosteric modulator. Although the $\alpha$2/3-$GABA_A$ positive allosteric modulator for use in the treatment of the present disclosure may be administered in the form of a starting compound, it is preferred to mix the active ingredient, optionally in the form of a physiologically acceptable salt, together with one or more additives, excipients, carriers, buffers, diluents and/or other conventional pharmaceutical auxiliary materials into a pharmaceutical composition.

In optional embodiments, the present disclosure provides a pharmaceutical composition comprising the $\alpha$2/3-$GABA_A$ positive allosteric modulator, wherein the $\alpha$2/3-$GABA_A$ positive allosteric modulator is mixed with one or more pharmaceutically acceptable carriers, and optionally with other therapeutic and/or prophylactic components known or used in the art. The carrier must be "acceptable", i.e., compatible with the other ingredients of the preparation and not deleterious to the recipient thereof.

The compound for use in the present disclosure may thus be formulated together with conventional additives or diluents into pharmaceutical compositions and unit dosage forms thereof. The forms include, for example, solids (especially in the form of tablets, filled capsules, powders and pills), and liquids (especially aqueous or non-aqueous solutions, suspensions, emulsions and elixirs), and capsules filled in the above forms, all forms for oral administration, suppositories for rectal administration and sterile injectable solutions for parenteral administration. The pharmaceutical compositions and the unit dosage forms thereof may include, for example, conventional ingredients in conventional proportions, with or without additional active compounds or ingredients, and the unit dosage forms may contain any suitable effective amount of the active ingredient commensurate with the desired range of daily applied doses.

The compound for use in the present disclosure may be administered in a variety of oral and parenteral dosage forms. It will be apparent to those skilled in the art that the dosage forms described below may contain the compound or the pharmaceutically acceptable salt thereof of the present disclosure as the active ingredient.

To formulate the compound for use in the present disclosure into pharmaceutical compositions, the pharmaceutically acceptable carrier may be either a solid or a liquid. Preparations in a solid form include powders, tablets, pills,

capsules, cachets, suppositories and dispersible granules. A solid carrier may be one or more substances that also function as diluents, flavoring agents, solubilizers, lubricants, suspending agents, binders, preservatives, tablet disintegrating agents or encapsulating materials.

In the powders, the carrier is a finely divided solid which is mixed with the finely divided active ingredient.

In the tablets, the active ingredient is mixed with a carrier having the necessary binding properties in a suitable proportion and compressed into desired shapes and sizes.

The powders and tablets preferably contain 5% or 10% to about 70% active compound. The suitable carriers are magnesium carbonate, magnesium stearate, talcum powder, sugar, lactose, pectin, dextrin, starch, gelatin, tragacanth, methylcellulose, sodium carboxymethylcellulose, wax with low melting point, cocoa butter, etc. The term "preparation" includes an active compound formulated with an encapsulating material as a carrier, in which the encapsulating material provides a capsule, and the active ingredient, with or without the carrier, is surrounded by the carrier, such that the active ingredient and the carrier are combined. Similarly, the preparation includes cachets and lozenges. The tablets, powders, capsules, pills, cachets and lozenges may be used as solid forms suitable for oral administration.

For preparing the suppositories, a wax with a low melting point, e.g., a puree of fatty glyceride or cocoa butter, is first melted, and the active ingredient is then dispersed homogeneously therein by stirring. The molten homogeneous mixture is then poured into appropriately sized molds, and allowed to cool and thereby to solidify.

Compositions suitable for vaginal administration may be present as pessaries, tampons, creams, gels, pastes, foams or sprays. In addition to the active ingredient, the compositions also contain suitable carriers known in the art.

Liquid preparations include solutions, suspensions and emulsions, e.g., aqueous solutions or water-propylene glycol solutions. For example, liquid preparations for parenteral injection may be formulated as water-polyethylene glycol solutions.

Therefore, the compound for use in the present disclosure may be formulated for parenteral administration (e.g., by injection, such as bolus injection or continuous infusion), and may be present in unit dosage forms in ampoules, pre-filled syringes, small-volume infusion bags or multi-dose containers with an added preservative. The composition may be present in the form of a suspension, solution or emulsion of an oily or aqueous carrier, and may contain formulation ingredients, e.g., suspending agents, stabilizers and/or dispersing agents. In addition, the active ingredient may be in the form of a powder, and may be obtained by aseptic isolation of a sterile solid or by lyophilization from a solution for reconstitution with a suitable carrier, e.g., sterile and pyrogen-free water, immediately before use.

Aqueous solutions suitable for oral administration may be prepared by dissolving the active ingredient in water and adding the required colorants, flavoring agents, stabilizers and thickening agents.

Aqueous suspensions suitable for oral administration may be prepared by dispersing the finely divided active component in water containing viscous materials, e.g., natural or synthetic gums, resins, methylcellulose, sodium carboxymethylcellulose or other well-known suspending agents.

The preparations also include solid preparations designed to be converted, immediately before use, to liquid preparations for oral administration. The liquid preparations include solutions, suspensions and emulsions. The preparations may contain, in addition to the active ingredient, colorants, flavoring agents, stabilizers, buffers, artificial and natural sweetening agents, dispersing agents, thickening agents, solubilizers, etc.

For topical application to the epidermis, the compound of the present disclosure may be formulated as ointments, creams or lotions or transdermal patches. For example, the ointments and creams may be formulated using an aqueous or oily base with the addition of suitable thickening agents and/or gels. The lotions may be formulated using an aqueous or oily base and generally also contain one or more emulsifying agents, stabilizers, dispersing agents, suspending agents, thickening agents or colorants.

Compositions suitable for topical administration in the mouth include lozenges containing the active ingredient in a flavored base, typically sucrose and acacia gum or tragacanth; pastilles containing the active ingredient in an inert base, e.g., gelatin and glycerol or sucrose and acacia gum; and mouthwashes containing the active ingredient in a suitable liquid carrier.

The solution or suspension may be applied directly to the nasal cavity by conventional means, e.g., using a dropper, pipette or nebulizer. The composition may be in single or multiple-dose form.

Respiratory tract administration may also be effected by means of an aerosol in which the active ingredient is packaged in a pressurized pack with a suitable propellant, including chlorofluorocarbon (CFC), e.g., dichlorodifluoromethane, trichloromonofluoromethane or dichlorotetrafluoroethane, carbon dioxide or other suitable gas. The aerosol may also contain surfactants, e.g., lecithin, as appropriate. The dosage of the drug may be controlled by a metering valve.

In addition, the active ingredient may be in the form of a dry powder, e.g., a powder mixture of a compound with a suitable powder base such as lactose, starch and starch derivatives such as hydroxy propyl methylcellulose and polyvinylpyrrolidone (PVP). The powder carrier may conveniently form a gel in the nasal cavity. The powder composition may be present in unit dosage forms, e.g., in capsules or cartridges (such as gelatin coat or cartridge), or in blister packs where a powder may be administered by means of an inhaler.

In compositions for use in respiratory tract administration (including compositions for intranasal use), the

compounds typically have a small particle size, e.g., a particle size of 5 micrometers or a smaller order of magnitude. The particle size may be obtained by methods known in the art, e.g., by micronization.

The particle size may be applied to compositions suitable for delayed release of the active ingredient, as needed.

The pharmaceutical preparation may optionally be in unit dosage forms. In the forms, the preparation is subdivided into unit doses containing an appropriate amount of the active ingredient. The unit dosage form may be a packaged preparation, of which the sealed package contains a large amount of the preparation separated, e.g., packaged tablets, capsules and powders in vials or ampoules. Further, the unit dosage forms may be capsules, tablets, cachets or lozenges per se, or may be an appropriate amount of the above capsules and tablets, etc., in any packaged forms.

The preferable composition is the tablet or capsule for oral administration and the liquid for intravenous administration and continuous infusion.

More detailed information on preparations and administration techniques may be found in the latest version of Remington's Pharmaceutical Sciences (Maack Publishing Co., Easton, PA).

The amount of active ingredient in the preparation in unit dosage forms may vary depending on the particular application and the potency of the active ingredient, and may be adjusted to 0.01 mg to about 0.1 g. For example, in pharmaceutical applications, the drug may be administered three times per day in a capsule of 0.01 to about 100 mg, and the composition may also contain other compatible therapeutic agents as necessary.

Therapeutic method

In therapeutic use, the compound for use in the present disclosure is administered at a starting dose of 0.001 mg/kg to 10 mg/kg body weight per day. However, the doses may vary depending on the patient's needs, the severity of the condition being treated and the compound being used, and generally, treatment is initiated at a smaller dose than the optimum dose of the compound, after which the dose is increased by small amounts to achieve the optimum effect. For convenience, the total daily dose may be further subdivided for administration at several times over the course of the day.

The pharmaceutical composition of the present disclosure may further be used simultaneously with other drugs for the treatment of pain, epilepsy, anxiety and depression, including but not limited to morphine, gabapentin, etc. Accordingly, the present disclosure provides a drug for treating pain, epilepsy, anxiety and depression, wherein the drug is not only effective but also free from significant side effects. Another object of the present disclosure is to provide a drug having high safety for a specific patient group, e.g., the elderly, patients with liver or kidney function deterioration or cardiovascular diseases.

The present disclosure further provides a method for treating or preventing a disease, wherein the method comprises administering to a patient an effective amount of the compound or the composition as described above.

The present disclosure further provides a method for treating or preventing a $GABA_A$ receptor-associated disease, wherein the method comprises administering to a patient an effective amount of the compound or the composition as described above.

The present disclosure further provides use of the compound or the composition as described above in the manufacture of a medicament for treating or preventing the following diseases: pain, Alzheimer's disease, multi-infarct dementia and stroke.

The pain is neuropathic pain, inflammatory pain and cancerous pain. Optionally, the pain is selected from: headache, facial pain, neck pain, shoulder pain, back pain, chest pain, abdominal pain, dorsalgia, low back pain, lower limb pain, musculoskeletal pain, vascular pain, gout, arthritis pain, visceral pain, pain due to infectious diseases, bony pain, pain associated with sickle cell anemia, autoimmune diseases, multiple sclerosis or inflammation, chronic pain caused by injury or surgery, nociceptive pain, painful diabetes, trigeminal neuralgia, pain of lumbar or cervical radiculopathy, glossopharyngeal neuralgia, autonomic neuroreflex pain, and pain associated with reflex sympathetic dystrophy, nerve root avulsion, cancer, chemical injury, toxin, nutritional deficiencies, viral or bacterial infection or degenerative osteoarthropathy.

The present disclosure further provides a method for treating or preventing pain, Alzheimer's disease, multi-infarct dementia or stroke, wherein the method comprises administering to a patient an effective amount of the compound or the composition as described above.

Beneficial effects:

The compound and the pharmaceutically acceptable salt thereof or the prodrug thereof of the present disclosure have important pharmacological properties, and are $\alpha$2/3-$GABA_A$ receptor positive allosteric modulators. The compound of the present disclosure has excellent affinity activity and positive regulation activity on $\alpha$2/3-$GABA_A$ receptors, and has good genetic toxicity safety and druggability. Therefore, the compound and the pharmaceutically acceptable salt or the prodrug thereof of the present disclosure may be used alone or in combination with other drugs for treating or preventing $\alpha$2/3-GABAA-associated diseases.

DETAILED DESCRIPTION OF THE PREFERRED EMBODIMENT

The technical solutions of the present disclosure are clearly and completely described below, and it is obvious that the described examples are some, but not all, examples of the present disclosure. Based on the examples of the present disclosure, all other examples obtained by those of ordinary skill in the art without creative efforts shall fall within the protection scope of the present disclosure. The ratio of the solvent used in the purification step (for example, preparative thin-layer chromatography and column chromatography) in the following examples is a volume ratio.

**Example 1**

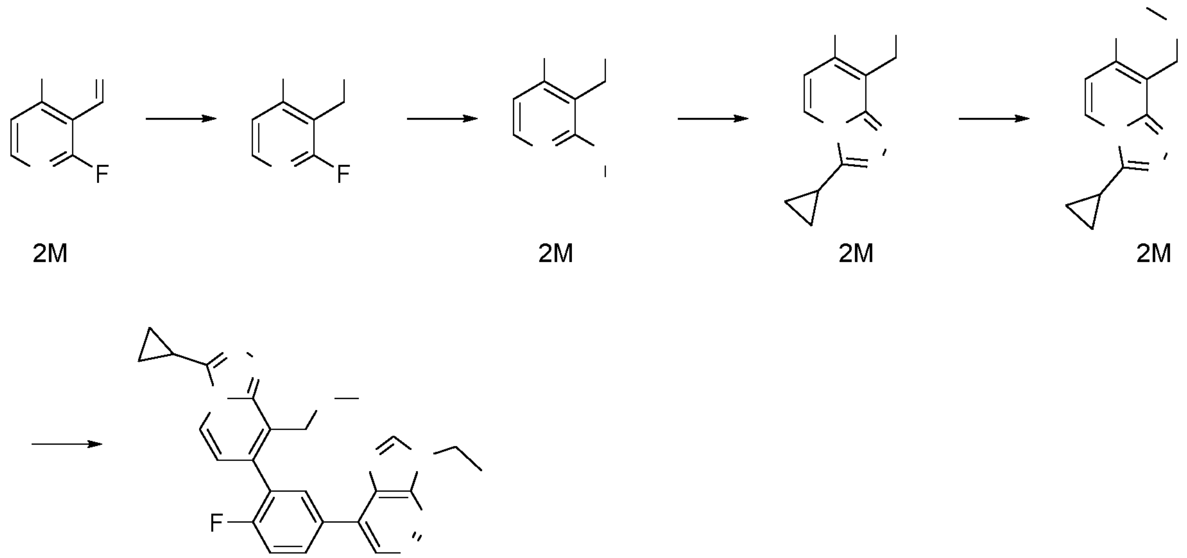


1

**1-1: (4-Bromo-2-fluoropyridin-3-yl)methanol**

4-Bromo-2-fluoronicotinaldehyde (565 mg, 2.77 mmol) was added to tetrahydrofuran (3 ml), and sodium borohydride (63 mg, 1.67 mmol) was added to the reaction mixture. The mixture was stirred at room temperature for 20 min. The reaction mixture was poured into methanol (10 ml) to quench the reaction, and then the mixture was poured into water (30 ml), and extracted with dichloromethane (30 ml × 3 times). The organic phases were combined, dried over anhydrous sodium sulfate, and concentrated to obtain a crude product (508 mg, 89%) as a colorless oily liquid. LC-MS: m/z $[M+H]^+$ = 206, 208.

**1-2: (4-Bromo-2-hydrazineylpyridin-3-yl)methanol**

(4-Bromo-2-fluoropyridin-3-yl)methanol (505 mg, 2.45 mmol) and hydrazine hydrate (246 mg, 4.9 mmol) were added to ethanol (4 ml), and the mixture was allowed to react at 95 °C for 2 h. The reaction mixture was directly subjected to column chromatography (dichloromethane/methanol = 10/1) to obtain the title compound (388 mg, 51%) as a white solid. LC-MS: m/z $[M+H]^+$ = 218, 220.

**1-3: (7-Bromo-3-cyclopropyl-[1,2,4]triazolo[4,3-a]pyridin-8-yl)methanol**

(4-Bromo-2-hydrazineylpyridin-3-yl)methanol (150 mg, 0.48 mmol) and cyclopropylcarboxaldehyde (50 mg, 0.72 mmol) were added to ethanol (2 ml), and the mixture was allowed to react at 90 °C for 1 h. Potassium carbonate (66 mg, 0.66 mmol) and iodine (12 mg, 0.05 mmol) were added to the reaction mixture, and the mixture was stirred at room temperature for 4 h. The reaction mixture was subjected to preparative thin-layer chromatography (dichloromethane/-methanol = 25/1) to obtain the title compound (65 mg, 51%) as a brown solid. LC-MS: m/z $[M+H]^+$ = 268, 270.

**1-4: 7-Bromo-3-cyclopropyl-8-(methoxymethyl)-[1,2,4]triazolo[4,3-a]pyridine**

(7-Bromo-3-cyclopropyl-[1,2,4]triazolo[4,3-a]pyridin-8-yl)methanol (60 mg, 0.22 mmol) was added to tetrahydrofuran (2 ml), and sodium hydride (8 mg, 0.26 mmol) was added to the reaction mixture. The mixture was stirred at room temperature for 10 min. Iodomethane (47 mg, 0.33 mmol) was added to the reaction mixture, and the mixture was stirred at room temperature for 30 min. The reaction mixture was subjected to preparative thin-layer chromatography (dichloromethane/methanol = 30/1) to obtain the title compound (55 mg, 89%) as a brown solid. LC-MS: m/z $[M+H]^+$ = 282, 284.

**Compound 1: 4-(3-(3-Cyclopropyl-8-(methoxymethyl)-[1,2,4]triazolo[4,3-a]pyridin-7-yl)-4-fluorophenyl)-7-ethyl-7H-imidazo[4,5-c]pyridazine**

7-Bromo-3-cyclopropyl-8-(methoxymethyl)-[1,2,4]triazolo[4,3-a]pyridine (30 mg, 0.11 mmol), (5-(7-ethyl-7H-imidazo[4,5-c]pyridazin-4-yl)-2-fluorophenyl)boronic acid (31 mg, 0.11 mmol), sodium carbonate (23 mg, 0.22 mmol) and bis(triphenylphosphino)palladium dichloride (8 mg, 0.01 mmol) were added to tetrahydrofuran (2 ml) and water (0.1 ml), and the mixture was allowed to react at 80 °C for 16 h under an argon atmosphere. The reaction mixture was subjected to preparative thin-layer chromatography (dichloromethane/methanol = 15/1) to obtain the title compound (8 mg, 16%) as a brown solid.

$^1$H NMR (400MHz, CHLOROFORM-d) 9.40 (s, 1 H), 8.42 (d, J = 6.8 Hz, 2 H), 8.28 (s, 1 H), 8.16 - 8.10 (m, 1 H), 7.46 - 7.38 (m, 1 H), 6.93 (br. s., 1 H), 4.83 (br. s., 2 H), 4.59 (d, J = 7.3 Hz, 2 H), 3.41 (s, 3 H), 2.13 - 2.00 (m, 1 H), 1.71 - 1.68 (m, 3 H), 0.88 (br. s., 4 H). LC-MS: m/z $[M+H]^+$ = 444.

**Example 2**

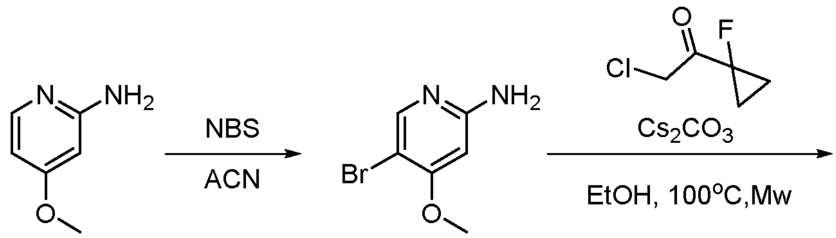


**2-0** **2-1**

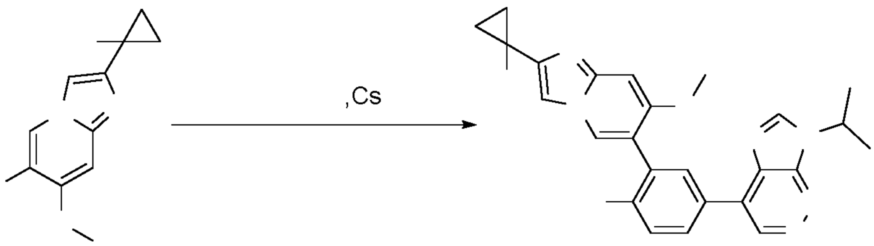


**2-1: 5-Bromo-4-methoxypyridin-2-amine**

2-Amino-4-methoxypyridine (5 g, 40 mmol) was dissolved in anhydrous acetonitrile (80.0 mL), and N-bromosuccinimide (NBS, 7 g, 40 mmol) was added in batches. The mixture was stirred at 0 °C for 2 h, and then added with a saturated aqueous sodium bicarbonate solution (100 mL) to quench the reaction. The mixture was separated, and the organic layer was collected. The aqueous phase was then extracted with dichloromethane (100 mL × 3), and concentrated to dryness by rotary evaporation to obtain a crude product. The crude product was purified by normal-phase silica gel column chromatography (petroleum ether:ethyl acetate = 10:1-2:1) to obtain the title product (4 g, 58%) as a white solid. LC-MS: m/z $[M+1]^+$ = 203.

**2-2: 6-Bromo-2-(1-fluorocyclopropyl)-7-methoxyimidazo[1,2-a]pyridine**

5-Bromo-4-methoxypyridin-2-amine (500 mg, 2.5 mmol), 2-chloro-1-(1-fluorocyclopropyl)ethan-1-one (670 mg, 5 mmol) and cesium carbonate (1.6 g, 5 mmol) were dissolved in an ethanol solution (10.0 mL), and the resulting solution was heated to 100 °C in a microwave reactor, and allowed to react for 1 h. The reaction mixture was purified by preparative HPLC to obtain the title product (100 mg, 27%) as a white solid. LC-MS: m/z $[M+1]^+$ = 285.

**Compound 2: 7-Isopropyl-4-(4-fluoro-3-(2-(1-fluorocyclopropyl)-7-methoxyimidazo[1,2-a]pyridin-6-yl)phenyl)-7H-imidazo[4,5-c]pyridazine**

6-Bromo-2-(1-fluorocyclopropyl)-7-methoxyimidazo[1,2-a]pyridine (100.0 mg, 0.36 mmol), (5-(7-isopropyl-7H-imidazo[4,5-c]pyridazin-4-yl)-2-fluorophenyl)boronic acid (105 mg, 0.36 mmol), cesium carbonate (210 mg, 0.54 mmol) and [1,1-bis(di-*tert*-butylphosphino)ferrocene]palladium dichloride (II) (16.5 mg, 0.036 mmol) were suspended in a 1,4-dioxane/water solution (5.5 mL, v/v = 10/1), and the mixture was heated to 100 °C and allowed to react for 2 h. The reaction mixture was diluted with ethyl acetate (50 mL), and then washed with a saturated aqueous sodium chloride solution (50 mL × 3). The organic phase was dried, concentrated and then purified by preparative HPLC to obtain the title product (30 mg, 17%) as a white solid.

$^1$H NMR (400 MHz, CDCl3) 9.64 (s, 1H), 9.06 (s, 1H), 8.98 (s, 1H), 8.70-8.62 (m, 1H), 8.58 (dd, J = 7.0, 2.2 Hz, 1H), 8.25 (s, 1H), 7.65 (t, J = 9.2 Hz, 1H), 7.30 (s, 1H), 5.22-5.02 (m, 1H), 4.02 (s, 3H), 1.67 - 1.63 (m, 8H), 1.42 (q, J = 8.5 Hz, 2H). LC-MS: m/z $[M+1]^+$ = 461.

**Example 3 and Example 4**

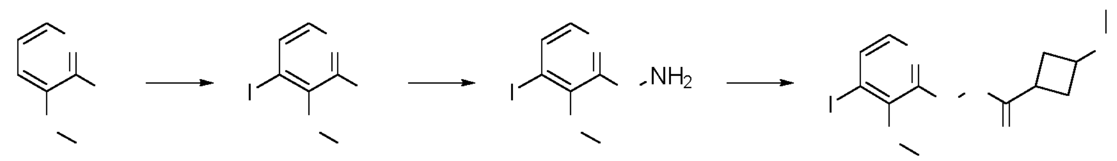

3-1

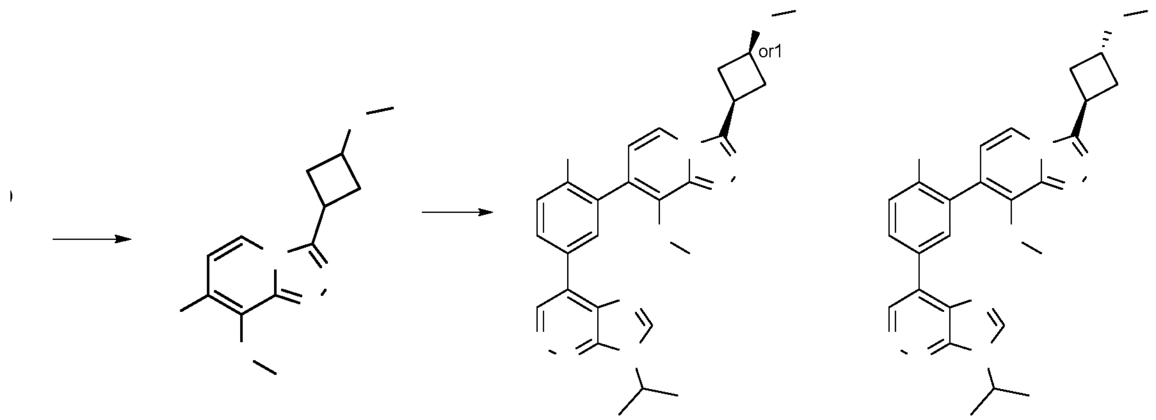

**3-1: 2-Fluoro-4-iodo-3-methoxypyridine**

2-Fluoro-3-methoxypyridine (5 g, 39.3 mmol) was dissolved in anhydrous tetrahydrofuran (150 mL), and the reaction mixture was cooled to -60 °C under a nitrogen atmosphere. N-butyllithium (2.5 M) (23.6 mL, 59 mmol) was added slowly and dropwise with the temperature controlled at -60 to -65 °C for 10 min, and the mixture was stirred at - 60 °C for 1 h. A solution (100 mL) of iodine (11 g, 43 mmol) in anhydrous tetrahydrofuran was added slowly with the temperature maintained at about -60 °C for 2 h, and then the mixture was allowed to react at -60 °C for 2 h. A saturated aqueous ammonium chloride solution (200 mL) was added to quench the reaction. The mixture was extracted with ethyl acetate

(100 mL × 3), and then washed with saturated brine (100 mL), dried over anhydrous sodium sulfate and concentrated. Then, the residue was purified by normal phase chromatography (petroleum ether:ethyl acetate = 3:1) to obtain the title product (6.8 g, white solid) with a yield of 68%. $^1$H NMR (400 MHz, CDCl3) 7.56 (m, 2H), 3.99 (s, 3H).

**3-2: 2-Hydrazineyl-4-iodo-3-methoxypyridine**

2-Fluoro-4-iodo-3-methoxypyridine (6.8 g, 26.88 mmol) was dissolved in ethanol (68.0 mL), and hydrazine hydrate (2.7 g, 53.8 mmol) was added. The mixture was allowed to react at 90 °C for 16 h. After the mixture was concentrated, water (100 mL) was added, and the mixture was stirred for 30 min and filtered. The filter cake was collected and dried under vacuum to obtain the title product (5.5 g, white solid) with a yield of 78%. LCMS: m/z $[M+1]^+$ = 266.

**3-3: N'-(4-Iodo-3-methoxypyridin-2-yl)-3-methoxycyclobutane-1-carbohydrazide**

2-Hydrazineyl-4-iodo-3-methoxypyridine (300 mg, 1.13 mmol), 3-methoxycyclobutane-1-carboxylic acid (147 mg, 1.13 mmol) and O-(7-azabenzotriazol-1-yl)-N,N,N',N'-tetramethyluronium hexafluorophosphate (472.6 mg, 1.24 mmol) were dissolved in anhydrous dichloromethane (12 mL), and diisopropylethylamine (366 mg, 2.83 mmol) was added slowly and dropwise under a nitrogen atmosphere. The mixture was stirred at 30 °C for 2 h. The reaction mixture was poured into ice water, and extracted with dichloromethane (100 ml × 3). After the mixture was concentrated, the residue was purified by reverse phase chromatography to obtain the title product (270 mg, yellow oil) with a yield of 71%. LCMS: m/z $[M+1]^+$ = 378.

**3-4: 7-Iodo-8-methoxy-3-(3-methoxycyclobutyl)-[1,2,4]triazolo[4,3-a]pyridine**

N'-(4-Iodo-3-methoxypyridin-2-yl)-3-methoxycyclobutane-1-carbohydrazide (270 mg, 0.72 mmol) was dissolved in anhydrous tetrahydrofuran (10 mL), and a Burgess reagent (methyl N-(triethylammoniumsulphonyl)carbamate, 512 mg, 2.15 mmol) was added. The reaction mixture was allowed to react at 60 °C for 3 h under a nitrogen atmosphere. The reaction mixture was poured into water (50 mL). The mixture was extracted with ethyl acetate (50 mL × 3) and then concentrated to obtain a crude product. The crude product was purified by prep-HPLC to obtain the title product (170 mg, white solid) with a yield of 66%. LCMS: m/z $[M+1]^+$ = 360.

**Compound 3: Rel-4-(4-fluoro-3-(8-methoxy-3-((1s,3s)-3-methoxycyclobutyl)-[1,2,4]triazolo[4,3-a]pyridin-7-yl) phenyl)-7-isopropyl-7H-imidazo[4,5-c]pyridazine**

**Compound 4: Rel-4-(4-fluoro-3-(8-methoxy-3-((1r,3r)-3-methoxycyclobutyl)-[1,2,4]triazolo[4,3-a]pyridin-7-yl) phenyl)-7-isopropyl-7H-imidazo[4,5-c]pyridazine**

Following the same experimental procedure as in Example 2, with starting materials of 7-iodo-8-methoxy-3-(3-methoxycyclobutyl)-[1,2,4]triazolo[4,3-a]pyridine (100.0 mg, 0.278 mmol) and (2-fluoro-5-(7-isopropyl-7H-imidazo[4,5-c]pyridazin-4-yl)phenyl)boronic acid (100.3 mg, 0.334 mmol), a mixture of the two title compounds was obtained as a white solid. Chiral resolution was then performed to obtain compounds of Example 3 and Example 4. Resolution method: instrument: MG II preparative SFC (SFC-1); chromatographic column: ChiralPakAD, 250 × 30mm I.D., 10 μm; mobile phase: A is $CO_2$, B is isopropanol; B is 55 vt%; flow rate: 80 mL/min; back pressure: 100 bar; column temperature: 38 °C; wavelength: 220 nm.

Example 3: The first peak, 14 mg, was a white solid with a yield of 10%.

$^1$H NMR (400 MHz, DMSO-d6) 9.58 (s, 1H), 8.95 (s, 1H), 8.64-8.44 (m, 2H), 8.13 (d, 1H), 7.67-7.46 (m, 1H), 7.03 (d, 1H), 5.24-4.92 (m, 1H), 4.33 (d, J = 3.2 Hz, 3H), 4.22-3.88 (m, 2H), 3.21 (s, 3H), 2.72 (m, 2H), 2.59-2.49 (m, 2H), 1.68 (d, J = 6.8 Hz, 6H). LCMS: m/z$[M+H]^+$ = 488.

Example 4: The second peak, 38 mg, was a white solid with a yield of 28%.

$^1$H NMR (400 MHz, DMSO-d6) 9.58 (s, 1H), 8.95 (s, 1H), 8.65-8.43 (m, 2H), 8.19 (d, 1H), 7.70-7.44 (m, 1H), 7.04 (d, 1H), 5.22-5.00 (m, 1H), 4.33 (s, 3H), 4.02 (t, J = 6.9 Hz, 1H), 3.60 (d, J = 7.9 Hz, 1H), 3.21 (s, 3H), 2.97-2.75 (m, 2H), 2.32 (dd, J = 10.5, 8.8 Hz, 2H), 1.68 (d, J = 6.8 Hz, 6H). LCMS: m/z $[M+H]^+$ = 488.

**Example 5**

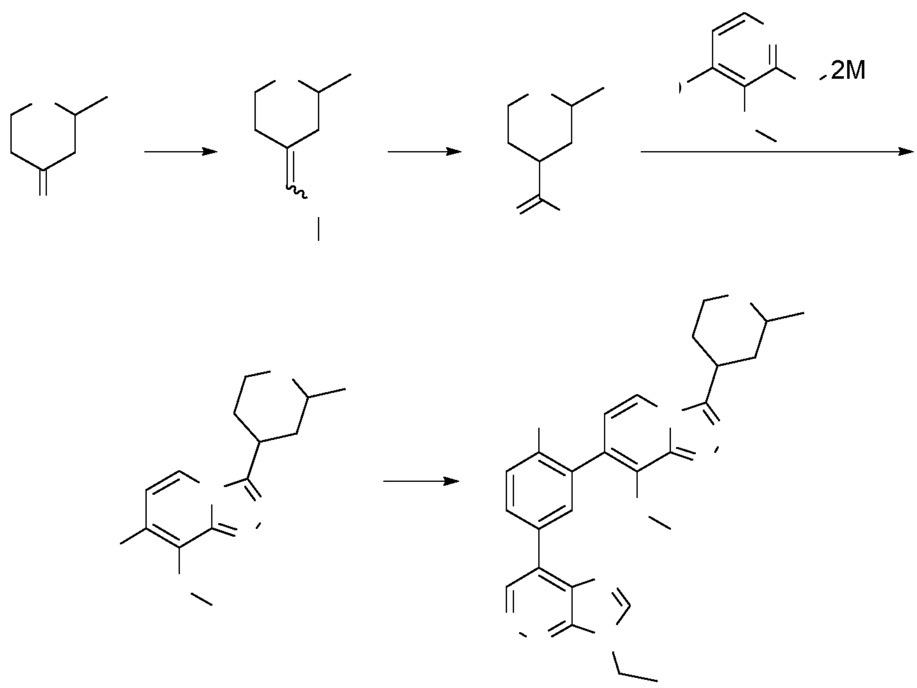


**5-1: 4-(Methoxymethylene)-2-methyltetrahydro-2H-pyran**

(Methoxymethyl)triphenylphosphonium chloride (22 g, 64.4 mmol) was suspended in anhydrous tetrahydrofuran (180 mL), and the reaction mixture was cooled to -10 to -20 °C under a nitrogen atmosphere. Lithium bis(trimethylsilyl) amide (1 M, 64.3 mL, 64.4 mmol) was added slowly and dropwise with the temperature controlled at -10 to -20 °C for 30 min, and the mixture was stirred at -10 to -20 °C for 2 h. Then, a solution of 2-methyltetrahydro-4H-pyran-4-one (4.9 g, 42.9 mmol) in anhydrous tetrahydrofuran (50 mL) was added dropwise for 10 min, and then the mixture was warmed to room temperature and allowed to react for 16 h. Saturated ammonium chloride (200 mL) was added to quench the reaction. The mixture was extracted with ethyl acetate (200 mL × 3), and then washed with saturated brine (200 mL), dried over anhydrous sodium sulfate and concentrated. Then, the residue was purified by normal phase chromatography (petroleum ether:ethyl acetate = 10:1) to obtain the title product (4.9 g, yellow oil) with a yield of 80%. $^1$H NMR (400 MHz, CDCl3) 5.95-5.76 (m, 1H), 4.07-3.95 (m, 1H), 3.54 (m, 3H), 3.43-3.26 (m, 2H), 2.62 (m, 1H), 2.01-1.78 (m, 2H), 1.70-1.55 (m, 1H), 1.20 (m, 3H).

**5-2: 2-Methyltetrahydro-2H-pyran-4-carbaldehyde**

4-(Methoxymethylene)-2-methyltetrahydro-2H-pyran (4.9 g, 34.4 mmol) was dissolved in formic acid (59 mL), and water (30 mL) was added. The mixture was heated to 90 °C and allowed to react for 6 h. After the mixture was cooled, water (300 mL) was added. The mixture was stirred for 30 min, and adjusted to pH=8-9 with 6 N NaOH, extracted with dichloromethane (150 mL × 3), then washed with saturated brine (100 mL), dried over anhydrous sodium sulfate and concentrated. Then, the residue was purified by normal phase chromatography (petroleum ether:ethyl acetate = 2:1) to obtain the title product (0.7 g, yellow oil) with a yield of 16%. $^1$H NMR (400 MHz, CDCl3) 9.67 (s, 1H), 4.09 (m, 1H), 3.60-3.36 (m, 2H), 2.51 (m, 1H), 1.84 (m, 2H), 1.56 (m, 1H), 1.24 (d, J = 6.2 Hz, 3H), 1.22-1.14 (m, 1H).

**5-3: 7-Iodo-8-methoxy-3-(2-methyltetrahydro-2H-pyran-4-yl)-[1,2,4]triazolo[4,3-a]pyridine**

2-Methyltetrahydro-2H-pyran-4-carbaldehyde (400 mg, 1.51 mmol) and 2-hydrazineyl-4-iodo-3-methoxypyridine (193 mg, 1.51 mmol) were dissolved in ethanol (8 mL), and the reaction mixture was heated to 90 °C under a nitrogen atmosphere and allowed to react for 2 h. The reaction mixture was concentrated, and dissolved in dichloromethane (16 mL). Iodine (460 mg, 1.81 mmol) and potassium carbonate (625 mg, 4.53 mmol) were added, and the mixture was stirred at room temperature for 16 h. Water (100 mL) was added, and the mixture was extracted with dichloromethane (50 mL × 3), then washed with saturated sodium thiosulfate (50 mL), washed with saturated brine (50 mL), dried over anhydrous sodium sulfate and concentrated. Then, the residue was purified by reverse phase chromatography (0.5% HCOOH-CH3CN) to obtain the title product (241 mg, white solid) with a yield of 43%. LCMS: m/z [M+H]$^+$ = 374.

**Compound 5: 7-Ethyl-4-(4-fluoro-3-(8-methoxy-3-(2-methyltetrahydro-2H-pyran-4-yl)-[1,2,4]triazolo[4,3-a]pyridin-7-yl)phenyl)-7H-imidazo[4,5-c]pyridazine**

Following the same experimental procedure as in Example 2, with starting materials of 7-iodo-8-methoxy-3-(2-methyltetrahydro-2H-pyran-4-yl)-[1,2,4]triazolo[4,3-a]pyridine (100 mg, 0.27 mmol) and (2-fluoro-5-(7-ethyl-7H-imidazo[4,5-c]pyridazin-4-yl)phenyl)boronic acid (77 mg, 0.27 mmol), the title compound (35 mg, white solid) was obtained with a yield of 27.0%.

$^1$H NMR (400 MHz, DMSO-d6) 9.58 (s, 1H), 8.88 (s, 1H), 8.68-8.49 (m, 2H), 8.44 (d, 1H), 7.66-7.53 (m, 1H), 7.04 (d, 1H), 4.52 (q, 2H), 4.31 (s, 3H), 4.02 (m, 1H), 3.61 (m, 3H), 2.01 (m, 2H), 1.84 (m, 1H), 1.65-1.46 (m, 4H), 1.18 (d, 3H). LCMS: m/z [M+H]$^+$ = 488.

**Example 6**

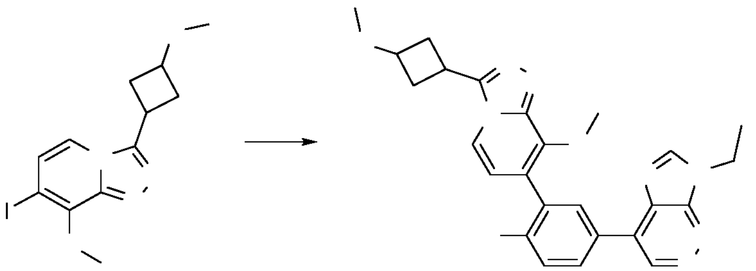

**Compound 6: 7-Ethyl-4-(4-fluoro-3-(8-methoxy-3-(3-methoxycyclobutyl)-[1,2,4]triazolo[4,3-a]pyridin-7-yl)phenyl)-7H-imidazo[4,5-c]pyridazine**

Following the same experimental procedure as in Example 2, with starting materials of 7-iodo-8-methoxy-3-(3-methoxycyclobutyl)-[1,2,4]triazolo[4,3-a]pyridine (85 mg, 0.24 mmol) and (5-(7-ethyl-7H-imidazo[4,5-c]pyridazin-4-yl)-2-fluorophenyl)boronic acid (66 mg, 0.24 mmol), the title compound (41.0 mg, white solid) was obtained with a yield of 36.2%.

$^1$H NMR (400 MHz, DMSO-d6) 9.58 (s, 1H), 8.88 (s, 1H), 8.63-8.46 (m, 2H), 8.18 (d, J = 7.0 Hz, 1H), 7.59 (t, J = 9.2 Hz, 1H), 7.03 (d, J = 7.0 Hz, 1H), 4.52 (q, J = 7.2 Hz, 2H), 4.32 (s, 3H), 4.02 (m, 1H), 3.61 (m, 1H), 3.21 (s, 3H), 2.87 (m, 2H), 2.31 (m, 2H), 1.56 (t, J = 7.3 Hz, 3H). LCMS: m/z [M+1]$^+$ = 474.

**Example 7**

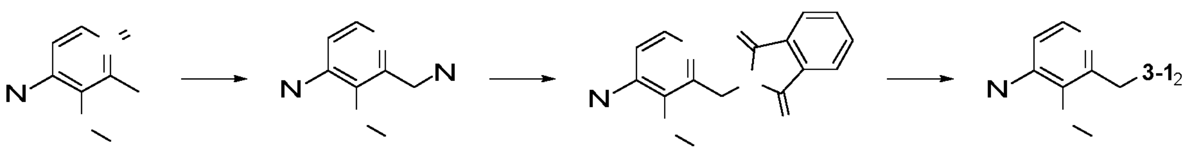

3-1

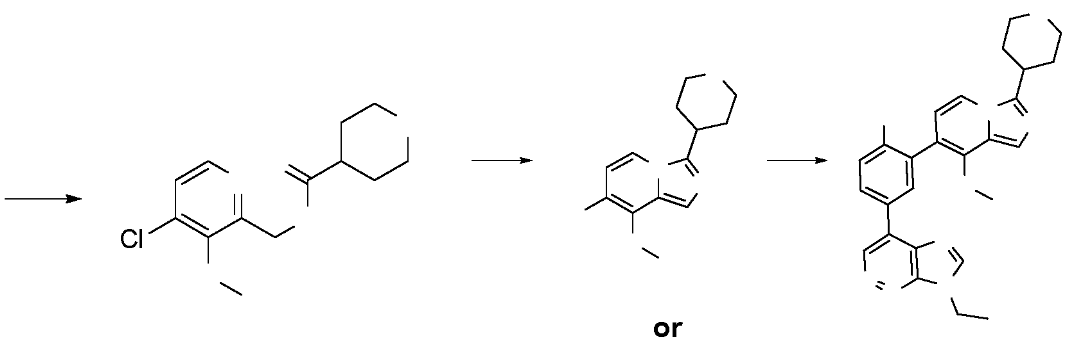

or

**7-1: 4-Chloro-2-(chloromethyl)-3-methoxypyridine**

4-Chloro-3-methoxy-2-methylpyridine 1-oxide (4.9 g, 28.31 mmol) was dissolved in anhydrous dichloromethane (250 mL), and phosphorus oxychloride (6.5 g, 42.47 mmol) dissolved in dichloromethane (40 mL), and triethylamine (5.7 g, 56.62 mmol) dissolved in dichloromethane (40 mL) were simultaneously and slowly added dropwise at the same speed under a nitrogen atmosphere. After the simultaneous addition was completed, the mixture was stirred at 30 °C for 3 h. The reaction mixture was poured into a saturated sodium bicarbonate solution (300 mL), extracted with dichloromethane (200 mL × 3), then washed with saturated brine, dried over anhydrous sodium sulfate and concentrated to obtain the title product (4.9 g, yellow solid) with a yield of 89%. LCMS: m/z $[M+1]^+$ = 192.

**7-2: 2-((4-Chloro-3-methoxypyridin-2-yl)methyl)isoindoline-1,3-dione**

4-Chloro-2-(chloromethyl)-3-methoxypyridine (5.4 g, 28.31 mmol) and phthalimide potassium salt (5.2 g, 28.31 mmol) were dissolved in N,N-dimethylformamide (100.0 mL), and the reaction mixture was allowed to react at 60 °C for 16 h under a nitrogen atmosphere. The reaction mixture was poured into water (100 mL). The mixture was extracted with ethyl acetate (100 mL × 3), then washed with 5% lithium chloride and saturated brine separately, dried over anhydrous sodium sulfate and concentrated to obtain a crude product. The crude product was purified by normal phase chromatography (CH2Cl2-ethyl acetate) to obtain the title product (3.7 g, white solid) with a yield of 48%. $^1$H NMR (400 MHz, CDCl3) 8.09 (d, J = 5.2 Hz, 1H), 7.91- 7.85 (m, 2H), 7.78-7.71 (m, 2H), 7.21 (d, J = 5.2 Hz, 1H), 5.08 (s, 2H), 4.03 (s, 3H). LCMS: m/z $[M+1]^+$ = 303.

**7-3: (4-Chloro-3-methoxypyridin-2-yl)methanamine**

2-((4-Chloro-3-methoxypyridin-2-yl)methyl)isoindoline-1,3-dione (3.8 g, 12.53 mmol) was dissolved in ethanol (38 mL), and hydrazine hydrate (7.6 mL) was added. The reaction mixture was allowed to react at 80 °C for 16 h. The reaction mixture was subjected to rotary evaporation to remove the ethanol, and water (50 mL) was added. The mixture was adjusted to pH=1 with concentrated hydrochloric acid, and a large amount of solid was precipitated. The mixture was filtered, and the filtrate was collected, adjusted to pH = 9-10 with NaOH, then extracted with ethyl acetate (100 mL × 3), washed with saturated brine, dried over anhydrous sodium sulfate and concentrated to obtain the title product (1.35 g, yellow oil) with a yield of 62%. LCMS: m/z $[M+1]^+$ = 173.

**7-4: N-((4-Chloro-3-methoxypyridin-2-yl)methyl)tetrahydro-2H-pyran-4-carboxamide**

(4-Chloro-3-methoxypyridin-2-yl)methanamine (300 mg, 1.74 mmol) was dissolved in dichloromethane (6 mL), and diisopropylethylamine (562 mg, 4.35 mmol) was added. Then, tetrahydro-2H-pyran-4-carbonyl chloride (258.5 mg, 1.74 mmol) was added slowly and dropwise to the reaction mixture, and the reaction mixture was allowed to react at 30 °C for 3 h. The reaction mixture was poured into water (50 mL), and extracted with dichloromethane (50 mL × 3), then washed with saturated brine, dried over anhydrous sodium sulfate and concentrated to obtain the title product (497 mg, yellow oil).

**7-5: 7-Chloro-8-methoxy-3-(tetrahydro-2H-pyran-4-yl)imidazo[1,5-a]pyridine**

N-((4-Chloro-3-methoxypyridin-2-yl)methyl)tetrahydro-2H-pyran-4-carboxamide (200 mg, 0.7 mmol) was dissolved in anhydrous tetrahydrofuran (4 mL), and a Burgess reagent (502 mg, 2.1 mmol) was added. The reaction mixture was allowed to react at 60 °C for 3 h under N2 atmosphere. The reaction mixture was poured into water (50 mL). The mixture was extracted with ethyl acetate (50 mL × 3) and then concentrated to obtain a crude product. The crude product was purified by reverse phase chromatography (0.5% HCOOH-CH3CN) to obtain the title product (102 mg, white solid) with a yield of 54%. LCMS: m/z $[M+1]^+$ = 267.

**7: 7-Ethyl-4-(4-fluoro-3-(8-methoxy-3-(tetrahydro-2H-pyran-4-yl)imidazo[1,5-a]pyridin-7-yl)phenyl)-7H-imidazo [4,5-c]pyridazine**

Following the same experimental procedure as in Example 2, with starting materials of 7-chloro-8-methoxy-3-(tetrahydro-2H-pyran-4-yl)imidazo[1,5-a]pyridine (102.1 mg, 0.38 mmol) and (5-(7-ethyl-7H-imidazo[4,5-c]pyridazin-4-yl)-2-fluorophenyl)boronic acid (109.5 mg, 0.38 mmol), the title compound (22 mg, white solid) was obtained with a yield of 12%.

$^1$H NMR (400 MHz, DMSO-d6) 9.57 (s, 1H), 8.87 (s, 1H), 8.56 (d, 1H), 8.52-8.38 (m, 1H), 8.19 (d, 1H), 7.61 (s, 1H), 7.55 (t, 1H), 6.71 (d, 1H), 4.52 (q, J = 7.2 Hz, 2H), 4.04-3.88 (m, 5H), 3.53 (m, 3H), 1.96-1.75 (m, 4H), 1.56 (t, J = 7.2 Hz, 3H). LCMS: m/z $[M+1]^+$ = 473.

**Example 8**

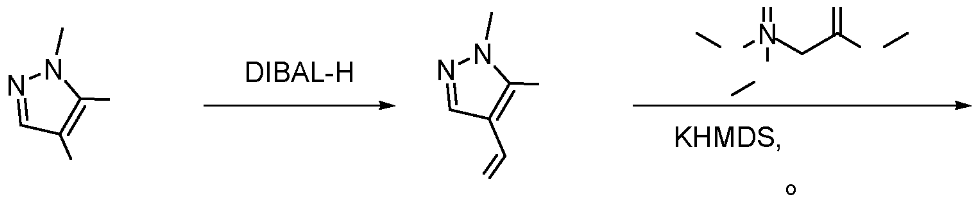


**8-1**

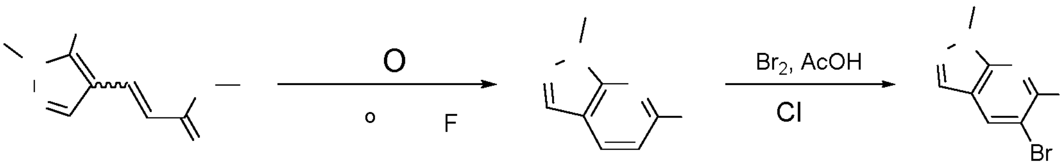


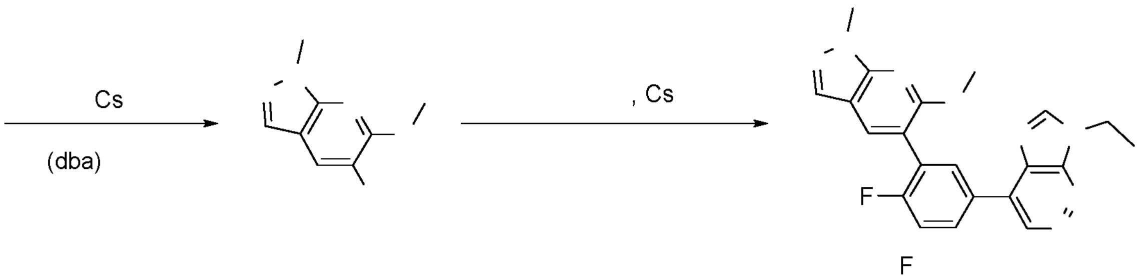


**8-1: 5-Amino-1-methyl-1H-pyrazole-4-carbaldehyde**

5-Amino-1-methyl-1H-pyrazole-4-carbonitrile (5 g, 128 mmol) was added to tetrahydrofuran (20 mL), and the mixture was cooled to -20 °C under an argon atmosphere. Diisobutylaluminium hydride (40 mL, 40 mmol) was added dropwise, and after the addition was completed, the mixture was stirred at room temperature for 16 h. The reaction mixture was poured into ice water (50 mL) and filtered, and the filtrate was extracted with ethyl acetate (50 mL × 3) and concentrated. The residue was purified by column chromatography (dichloromethane: methanol = 15:1) to obtain a compound (420 mg, yield: 20%) as a white solid. LC-MS: m/z $[M+H]^+$ = 126.0.

**8-2: Methyl 3-(5-amino-1-methyl-1H-pyrazol-4-yl)acrylate**

18-Crown-6 (2.9 g, 11.0 mmol) was dissolved in tetrahydrofuran (20 mL), and the resulting solution was cooled to -40 °C. Then, methyl 2-(dimethoxyphosphoryl)acetate (420 mg, 2.2 mmol) and lithium bis(trimethylsilyl)amide (2.2 mL, 2.2 mmol) were added, and 5-amino-1-methyl-1H-pyrazole-4-carbaldehyde (275 mg, 2.2 mmol) dissolved in tetrahydrofuran (10 mL) was added dropwise to the system. Then, the reaction mixture was stirred at room temperature for 16 h, and poured into water (50 mL), extracted with ethyl acetate (50 mL × 3), and concentrated. The residue was purified by column chromatography (petroleum ether:ethyl acetate = 1:1) to obtain a compound (200 mg, yield: 50%) as a white solid. LC-MS: m/z $[M+H]^+$ = 182.

**8-3: 1-Methyl-1H-pyrazolo[3,4-b]pyridin-6-ol**

Methyl 3-(5-amino-1-methyl-1H-pyrazol-4-yl)acrylate (200 mg, 1.1 mmol) was dissolved in dilute hydrochloric acid (3 mol/L, 20 mL), and then the resulting solution was stirred at 100 °C for 4 h. The mixture was directly concentrated to dryness by rotary evaporation, and then dissolved with distilled water (5 mL) under heating. The liquid was left standing on

the upper layer of a freezer and cooled, and a large amount of white flocculent precipitate was precipitated. The mixture was filtered to obtain a white solid (150 mg, yield: 91%). LC-MS: m/z $[M+H]^+$ = 150.

**8-4: 5-Bromo-1-methyl-1H-pyrazolo[3,4-b]pyridin-6-ol**

1-Methyl-1H-pyrazolo[3,4-b]pyridin-6-ol (150 mg, 1.0 mmol) was added to acetic acid (15 mL), and liquid bromine (480 mg, 3.0 mmol) was added dropwise. Then, the mixture was stirred at room temperature for 2 h, and the reaction mixture was directly concentrated to dryness by rotary evaporation. Deionized water (20 mL) was added, and a large amount of white solid was precipitated. The mixture was filtered to obtain a white solid (100 mg, yield: 44%). LC-MS: m/z $[M+H]^+$ = 228.

**8-5: 5-Bromo-6-methoxy-1-methyl-1H-pyrazolo[3,4-b]pyridine**

5-Bromo-1-methyl-1H-pyrazolo[3,4-b]pyridin-6-ol (114 mg, 0.5 mmol) was added to N,N-dimethylformamide (20 mL), and cesium carbonate (326 mg, 1.0 mmol) and iodomethane (142 mg, 1.0 mmol) were added. The mixture was allowed to react at 60 °C for 2 h, and the reaction mixture was poured into ice water (50 mL), extracted with ethyl acetate (50 mL × 3), and concentrated. The residue was purified by column chromatography (petroleum ether:ethyl acetate = 8:1) to obtain a compound (120 mg, yield: 99%) as a white solid. LC-MS: m/z $[M+H]^+$ = 242.

**8: 7-Ethyl-4-(4-fluoro-3-(6-methoxy-1-methyl-1H-pyrazolo[3,4-b]pyridin-5-yl)phenyl)-7H-imidazo[4,5-c]pyridazine**

Following the same experimental procedure as in Example 2, with starting materials of 5-bromo-6-methoxy-1-methyl-1H-pyrazolo[3,4-b]pyridine (100 mg, 0.40 mmol) and 7-ethyl-4-(4-fluoro-3-(4,4,5,5-tetramethyl-1,3,2-dioxaborolan-2-yl)phenyl-7H-imidazo[4,5-c]pyridazine (125 mg, 0.44 mmol), a white solid (30 mg, yield: 20%) was obtained.

$^1H$ NMR (400 MHz, CDCl3) 9.42 (s, 1H), 8.36 (s, 1H), 8.30 (s, 2H), 7.96 (s, 1H), 7.92 (s, 1H), 7.37 (t, 1H), 4.59 (d, J = 6.8 Hz, 2H), 4.11 (s, 3H), 4.06 (s, 3H), 1.70 (t, J = 6.4 Hz, 3H). LC-MS: m/z $[M+H]^+$ = 404.

**Example 9**

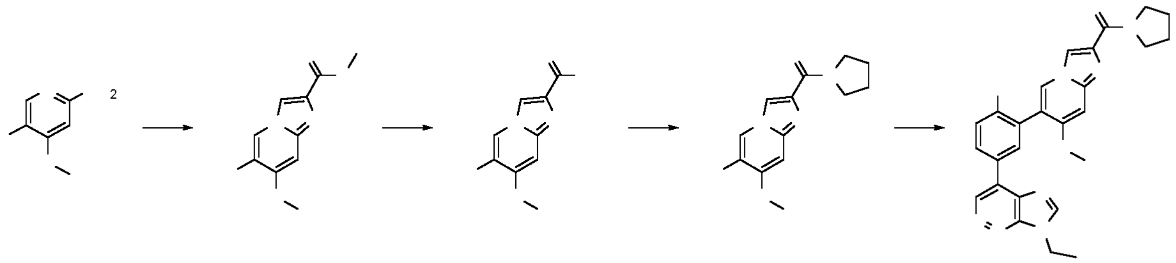


**9-1: Methyl 6-bromo-7-methoxyimidazo[1,2-a]pyridine-2-carboxylate**

5-Bromo-4-methoxypyridin-2-amine (1 g, 3.5 mmol) and methyl 3-bromo-2-oxopropanoate (762 mg, 4.2 mmol) were added to anhydrous ethanol (10 mL), and the mixture was stirred in a sealed container at 90 °C overnight. The reaction mixture was separated and purified directly by a preparative plate (petroleum ether/ethyl acetate = 3/1) to obtain the title compound (600 mg, 60%).

**9-2: 6-Bromo-7-methoxyimidazo[1,2-a]pyridine-2-carboxylic acid**

Methyl 6-bromo-7-methoxyimidazo[1,2-a]pyridine-2-carboxylate (400 mg, 1.4 mmol) and a (2 M) aqueous sodium hydroxide solution (2 mL) were added to tetrahydrofuran (4 mL), and the mixture was stirred at room temperature overnight. The reaction mixture was concentrated. The aqueous phase was extracted three times with ethyl acetate, and the organic phase was concentrated. The residue was separated and purified by a preparative plate (dichloromethane/methanol = 20/1) to obtain the title compound (300 mg, 79%).

**9-3: (6-Bromo-7-methoxyimidazo[1,2-a]pyridin-2-yl)(pyrrolidin-1-yl)methanone**

6-Bromo-7-methoxyimidazo[1,2-a]pyridine-2-carboxylic acid (300 mg, 1.1 mmol), 1-hydroxybenzotriazole (234 mg, 1.1 mmol), 1-(3-dimethylaminopropyl)-3-ethylcarbodiimide hydrochloride (95 mg, 0.5 mmol), N,N-diisopropylethylamine (425 mg, 3.3 mmol), and pyrrolidine (213 mg, 3.3 mmol) were added to N,N-dimethylformamide (6 mL), and the mixture was stirred at 35 °C overnight. The reaction mixture was separated and purified directly by a preparative plate (dichloromethane/methanol = 20/1) to obtain the title compound (60 mg, 17%).

**9: (6-(5-(7-Ethyl-7H-imidazo[4,5-c]pyridazin-4-yl)-2-fluorophenyl)-7-methoxyimidazo[1,2-a]pyridin-2-yl)(pyrrolidin-1-yl)methanone**

Following the same experimental procedure as in Example 2, with starting materials of **(6-bromo-7-methoxyimidazo[1,2-a]pyridin-2-yl)(pyrrolidin-1-yl)methanone** (60 mg, 0.19 mmol) and 7-ethyl-4-(4-fluoro-3-(4,4,5,5-tetramethyl-1,3,2-dioxaborolan-2-yl)phenyl)-7H-imidazo[4,5-c]pyridazine (72 mg, 0.19 mmol), the title compound was obtained (50 mg, 54%).

$^1$H NMR (400MHz, CHLOROFORM-d) 9.38 (s, 1 H), 8.30 (br. s., 1 H), 8.15 - 8.04 (m, 1 H), 7.79 - 7.67 (m, 1 H), 7.53 (br. s., 1 H), 7.38 (br. s., 1 H), 4.59 (d, J = 6.8 Hz, 1 H), 4.13 (br. s., 1 H), 3.90 (br. s., 3 H), 3.75 (br. s., 1 H), 2.03 - 1.93 (m, 4 H), 1.70 (d, J = 7.3 Hz, 1 H). LC-MS: m/z[M+H]$^+$= 486.

**Example 10**

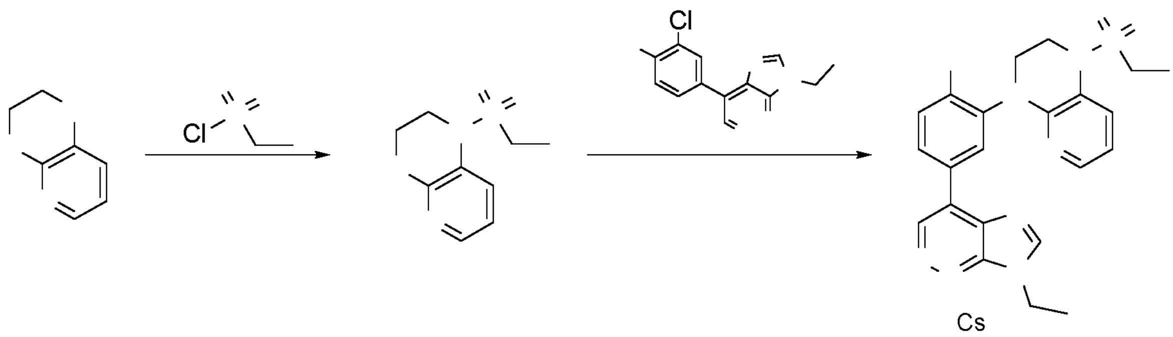

**10-1: 1-(Ethylsulfonyl)-1,2,3,4-tetrahydropyrido[2,3-b]pyrazine**

1,2,3,4-Tetrahydropyrido[2,3-b]pyrazine (500 mg, 3.7 mmol) was dissolved in tetrahydrofuran (10 mL). Triethylamine (1.12 g, 11.1 mmol) was added, and then ethanesulfonyl chloride (480 mg, 3.7 mmol) was added. The mixture was stirred at room temperature for 12 h, diluted with ethyl acetate (200 mL), washed with water (150 mL × 2), dried, and concentrated. The residue was purified by column chromatography (petroleum ether:ethyl acetate = 1:1) to obtain a yellow solid (300 mg, yield: 35.7%). LC-MS: m/z [M+H]$^+$ = 228.0.

**10: 4-(5-(7-Ethyl-7H-imidazo[4,5-c]pyridazin-4-yl)-2-fluorophenyl)-1-(ethylsulfonyl)-1,2,3,4-tetrahydropyrido [2,3-b]pyrazine**

1-(Ethylsulfonyl)-1,2,3,4-tetrahydropyrido[2,3-b]pyrazine (200 mg, 0.88 mmol), 4-(3-chloro-4-fluorophenyl)-7-ethyl-7H-imidazo[4,5-c]pyridazine (364 mg, 1.32 mmol), potassium phosphate (374 mg, 1.76 mmol), tris(dibenzylideneacetone)dipalladium (80 mg, 0.09 mmol), and toluene (20 mL) were successively added into a reaction flask, and the mixture was heated to 90 °C and allowed to react for 16 h. The reaction mixture was concentrated directly. The residue was purified by prep-HPLC (0.5% NH3 .H2O-acetonitrile) to obtain a white solid (30 mg, yield: 15%).

$^1$H NMR (400 MHz, CDCl3) 9.39 (s, 1H), 8.32 (m, , 2H), 8.28-8.19 (m, 1H), 7.91 (d, J = 4.8 Hz, 1H), 7.85 (d, J = 8.0 Hz, 1H), 7.43-7.33 (m, 1H), 6.74 (m, 1H), 4.58 (q, J = 7.3 Hz, 2H), 4.14-4.00 (m, 2H), 4.00-3.90 (m, 2H), 3.18 (q, J = 7.4 Hz, 2H), 1.70 (t, J = 7.3 Hz, 3H), 1.44 (t, J = 7.4 Hz, 3H). LC-MS: m/z [M+H]$^+$ = 468.

**Example 11**

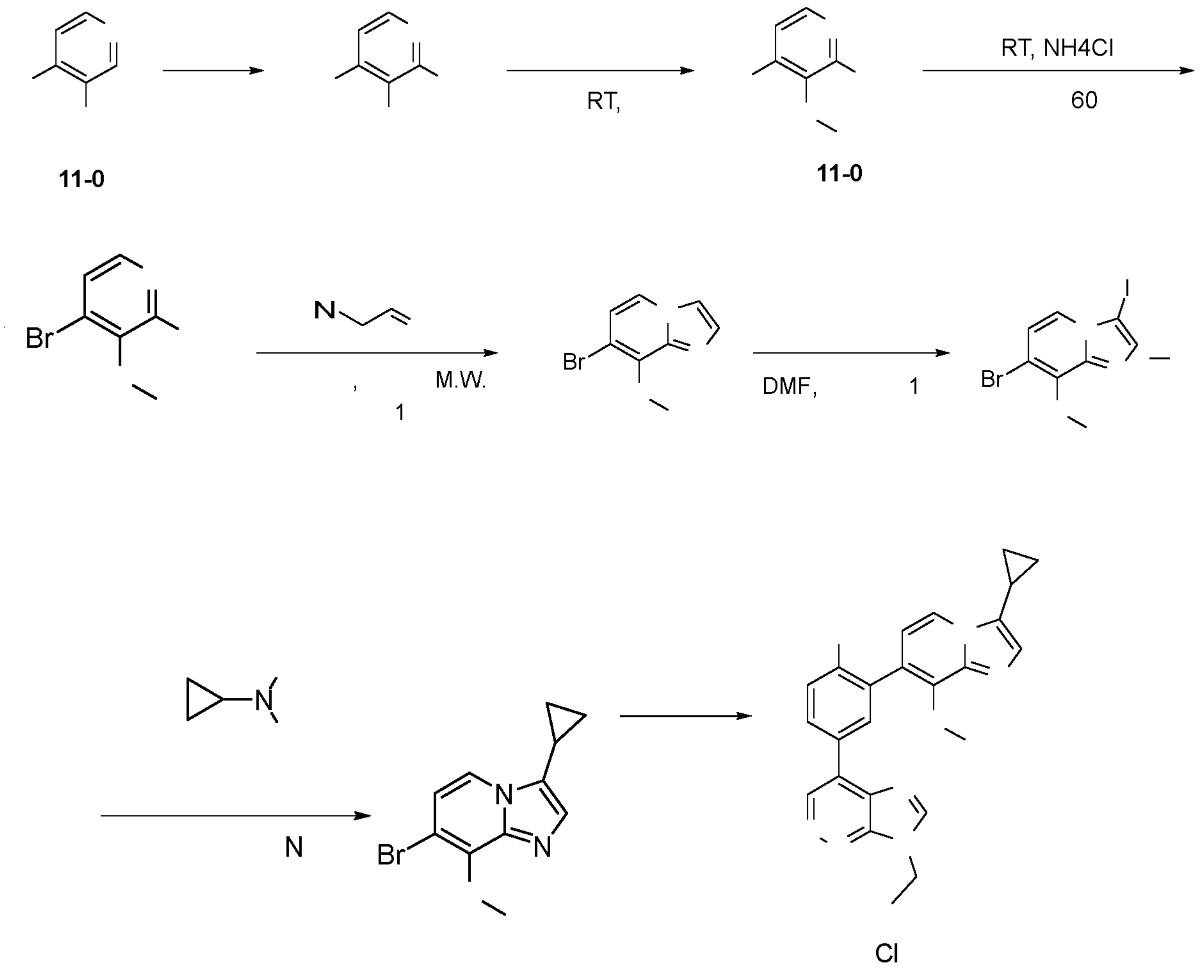


**11-1: 4-Bromo-2-nitropyridin-3-ol**

4-Bromopyridin-3-ol (3 g, 17.3 mmol) was added to 8 mL of concentrated sulfuric acid under an inert atmosphere of nitrogen, and the mixture was stirred for 15 min. Fuming nitric acid (1.64 g, 26 mmol) was added dropwise at 0 °C, and the mixture was stirred overnight. The reaction mixture in 10 g of ice was extracted with ethyl acetate (10 mL × 3), and concentrated to dryness by rotary evaporation to obtain a crude product. The crude product was purified by normal-phase silica gel column chromatography (petroleum ether:ethyl acetate = 10:1-1:1) to obtain the title product (2.19 g, yield: 56%) as a yellow solid. LC-MS: m/z $[M+H]^+$ = 219.

**11-2: 4-Bromo-3-methoxy-2-nitropyridine**

4-Bromo-2-nitropyridin-3-ol (2.19 g, 10 mmol) and potassium carbonate (1.39 g, 20 mmol) were dissolved in N,N-dimethylformamide (17.0 mL), and iodomethane (1.43 g, 20 mmol) was added dropwise. The mixture was stirred at room temperature for 17 h. The reaction was quenched with water (20 mL), and then the mixture was extracted with ethyl acetate (20 mL × 2) and concentrated to dryness by rotary evaporation to obtain a crude product. The crude product was purified by normal-phase silica gel column chromatography (petroleum ether:ethyl acetate = 10:1-2:1) to obtain the title product (0.65 g, yield: 28%) as a white solid. LC-MS: m/z $[M+H]^+$ = 233.

**11-3: 4-Bromo-3-methoxypyridin-2-amine**

4-Bromo-3-methoxy-2-nitropyridine (550 mg, 2.37 mmol) and ammonium chloride (628 mg, 11.85 mmol) were dissolved in a mixture of ethanol:water = 1:1 (16.6 mL). The resulting solution was heated to 60 °C, and iron powder (664 mg, 11.85 mmol) was added in batches. The reaction mixture was further stirred at 60 °C for 2 h and filtered. The filter cake was rinsed with methanol (10 mL × 3), and the rinse eluates were combined and concentrated to dryness by rotary evaporation. The residue was diluted with 5 mL of water. The aqueous phase was extracted with ethyl acetate (10 mL × 3) and concentrated to dryness by rotary evaporation to obtain the title product (514 mg, 100%) as a brown solid. LC-MS: m/z

$[M+H]^+$ = 203.

**11-4: 7-Bromo-8-methoxyimidazo[1,2-a]pyridine**

4-Bromo-3-methoxypyridin-2-amine (200 mg, 0.99 mmol), chloroacetaldehyde (295 mg, 40 Wt%, 1.5 mmol), and sodium bicarbonate (159 mg, 1.5 mmol) were dissolved in an ethanol solution (10.0 mL). The resulting solution was heated to 90 °C in a microwave reactor and allowed to react for 1 h. The reaction mixture was concentrated, and the residue was diluted with 2 mL of water. The aqueous phase was extracted with dichloromethane (2 mL × 3), and the organic phases were combined, dried, and concentrated to dryness by rotary evaporation to obtain the title product (290 mg, 100%) as a brown solid. LC-MS: m/z $[M+H]^+$ = 227.

**11-5: 7-Bromo-3-iodo-8-methoxyimidazo[1,2-a]pyridine**

7-Bromo-8-methoxyimidazo[1,2-a]pyridine (400 mg, 1.28 mmol) was dissolved in N,N-dimethylformamide (7.5 mL), and N-iodosuccinimide (375 mg, 1.67 mmol) was added. The reaction mixture was stirred at 100 °C for 1 h. The mixture was then cooled to room temperature and diluted with 10 mL of water. The aqueous phase was then extracted with methyl *tert*-butyl ether (10 mL × 3) and concentrated to dryness by rotary evaporation to obtain the title product (220 mg, 100%) as a brown solid. LC-MS: m/z $[M+H]^+$ = 353.

**11-6: 7-Bromo-3-cyclopropyl-8-methoxyimidazo[1,2-a]pyridine**

7-Bromo-3-iodo-8-methoxyimidazo[1,2-a]pyridine (400 mg, 1.13 mmol), cyclopropylboronic acid (97 mg, 1.13 mmol), tetrakis(triphenylphosphine)palladium (131 mg, 0.113 mmol), and potassium phosphate (481 mg, 2.27 mmol) were dissolved in a mixture of dioxane:$H_2O$ = 10:1 (11 mL). The resulting solution was purged three times with argon and then stirred at 95 °C for 17 h. The reaction mixture was cooled to room temperature, and purified by normal-phase silica gel column chromatography (petroleum ether:ethyl acetate = 10:1-1:1) to obtain the title product (100 mg, 33%) as a yellow solid. LC-MS: m/z $[M+H]^+$ = 267.

**11: 4-(3-(3-Cyclopropyl-8-methoxyimidazo[1,2-a]pyridin-7-yl)-4-fluorophenyl)-7-ethyl-7H-imidazo[4,5-c]pyridazine**

Following the same experimental procedure as in Example 2, with starting materials of 7-bromo-3-cyclopropyl-8-methoxyimidazo[1,2-a]pyridine (100 mg, 0.37 mmol) and (5-(7-ethyl-7H-imidazo[4,5-c]pyridazin-4-yl)-2-fluorophenyl) boronic acid (107 mg, 0.37 mmol), the title compound (60 mg, 37%) was obtained as an off-white solid.

$^1$H NMR (400 MHz, CDCl3) 9.37 (s, 1H), 8.38-8.30 (m, 1H), 8.29-8.20 (m, 2H), 7.85 (d, 1H), 7.36 (dd, 2H), 6.78 (d, 1H), 4.58 (q, 2H), 4.24 (s, 3H), 2.12 (s, 1H), 1.69 (t, 3H), 0.97 (m, 4H). LC-MS: m/z $[M+H]^+$ = 429.

**Example 12**

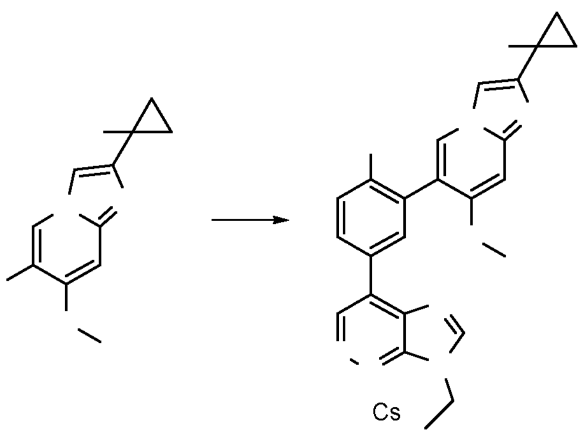

**12: 7-Ethyl-4-(4-fluoro-3-(2-(1-fluorocyclopropyl)-7-methoxyimidazo[1,2-a]pyridin-6-yl)phenyl)-7H-imidazo[4,5-c]pyridazine**

Following the same experimental procedure as in Example 2, with starting materials of 6-bromo-2-(1-fluorocyclopropyl)-7-methoxyimidazo[1,2-a]pyridine (100.0 mg, 0.36 mmol) and (5-(7-ethyl-7H-imidazo[4,5-c]pyridazin-4-yl)-2-fluorophenyl)boronic acid (100 mg, 0.36 mmol), the title product was obtained (30 mg, yield: 17%) as a white solid.
$^1$H NMR (400 MHz, DMSO-d6) 9.38 (s, 1H), 8.29 (m, 3H), 8.05 (s, 1H), 7.55 (s, 1H), 7.37 (t, 1H), 6.99 (s, 1H), 4.59 (q, 2H), 3.85 (s, 3H), 1.70 (t, 3H), 1.56-1.33 (m, 4H). LC-MS: m/z [M+H]$^+$ = 447.

**Example 13**

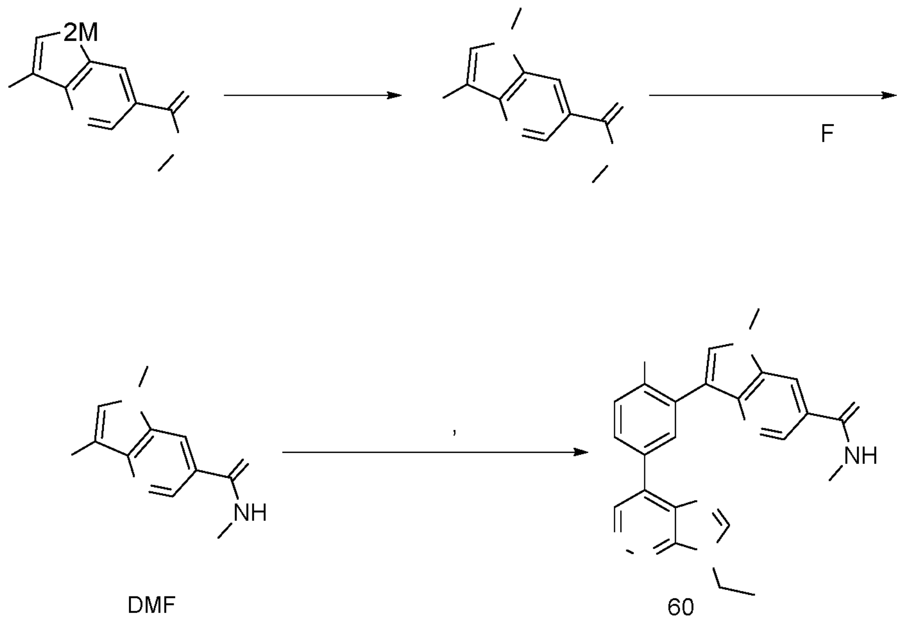


**13-1: Methyl 3-bromo-1-methyl-1H-pyrrolo[3,2-b]pyridine-6-carboxylate**

Methyl 3-bromo-1H-pyrrolo[3,2-b]pyridine-6-carboxylate (102 mg, 0.4 mmol) was dissolved in N,N-dimethylformamide (5 mL). Sodium cyanide (32 mg, 0.8 mmol) was added at 0 °C. The mixture was stirred for 30 min, and then iodomethane (65 mg, 0.6 mmol) was added. The mixture was stirred at room temperature for 16 h. The reaction mixture was poured into ice water (30 mL) and extracted with ethyl acetate (50 mL × 3). The organic phases were combined, dried, and concentrated. The residue was purified by column chromatography (petroleum ether:ethyl acetate = 3:1) to obtain a white solid (100 mg, yield: 92.9%). LC-MS: m/z [M+H]$^+$ = 269.

**13-2: 3-Bromo-N,1-dimethyl-1H-pyrrolo[3,2-b]pyridine-6-carboxamide**

Methyl 3-bromo-1-methyl-1H-pyrrolo[3,2-b]pyridine-6-carboxylate (110 mg, 0.41 mmol) was dissolved in a solution of methylamine in ethanol (5 mL). The resulting solution was stirred at 80 °C for 16 h and then concentrated directly to obtain a white solid (80 mg, yield: 73.1%). LC-MS: m/z [M+H]$^+$ = 268.

13: **3-(5-(7-Ethyl-7H-imidazo[4,5-c]pyridazin-4-yl)-2-fluorophenyl)-N,1-dimethyl-1H-pyrrolo[3,2-b]pyridine-6-carboxamide**

Following the same experimental procedure as in Example 2, with starting materials of 3-bromo-N,1-dimethyl-1H-pyrrolo[3,2-b]pyridine-6-carboxamide (80 mg, 0.30 mmol) and (5-(7-ethyl-7H-imidazo[4,5-c]pyridazin-4-yl)-2-fluorophenyl)boronic acid (101 mg, 0.36 mmol), the title compound (45.0 mg, white solid) was obtained with a yield of 35%.
$^1$H NMR (400 MHz, CDCl3) 9.59-9.42 (m, 1H), 9.41-9.25 (m, 1H), 8.99-8.88 (m, 1H), 8.30-8.18 (m, 2H), 8.13-8.03 (m, 1H), 8.00-7.92 (m, 1H), 7.35-7.28 (m, 1H), 4.64-4.45 (m, 2H), 4.02-3.89 (m, 3H), 3.15-2.92 (m, 3H), 1.69-1.60 (m, 3H).

LC-MS: m/z $[M+H]^+$ = 430.

**Example 14**

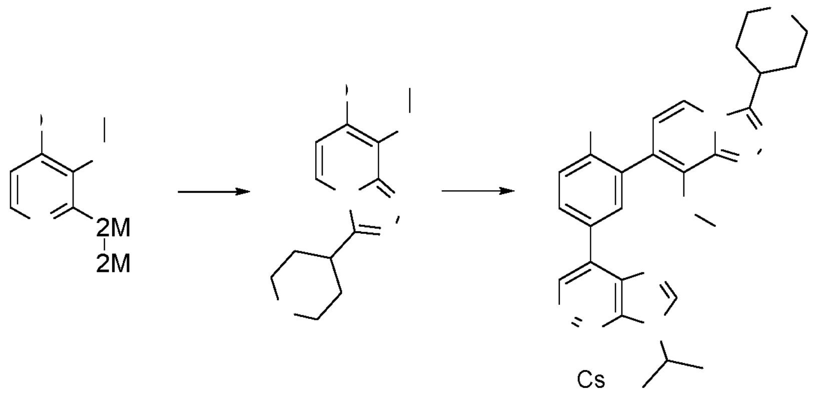

**14-1: 7-Iodo-8-methoxy-3-(tetrahydro-2H-pyran-4-yl)-[1,2,4]triazolo[4,3-a]pyridine**

Following the same experimental procedure as the synthesis method for 5-3 in Example 5, with starting materials of 2-hydrazineyl-4-iodo-3-methoxypyridine (100 mg, 0.38 mmol) and tetrahydro-2H-pyran-4-carbaldehyde (50 mg, 0.38 mmol), the title compound was obtained (100 mg, 74%) as a white solid. LC-MS: m/z $[M+H]^+$ = 360.

**14: 4-(4-Fluoro-3-(8-methoxy-3-(tetrahydro-2H-pyran-4-yl)-[1,2,4]triazolo[4,3-a]pyridin-7-yl)phenyl)-7-isopropyl-7H-imidazo [4,5-c] pyridazine**

Following the same experimental procedure as in Example 2, with starting materials of 4-(4-fluoro-3-(4,4,5,5-tetramethyl-1,3,2-dioxaborolan-2-yl)phenyl)-7-isopropyl-7H-imidazo[4,5-c]pyridazine (45 mg, 0.11 mmol) and 7-iodo-8-methoxy-3-(tetrahydro-2H-pyran-4-yl)-[1,2,4]triazolo[4,3-a]pyridine (50 mg, 0.14 mmol), the title compound (40 mg, 74.5%) was obtained.

$^1$H NMR (400MHz ,CHLOROFORM-d) 9.37 (s, 1 H), 8.37 - 8.31 (m, 2 H), 8.27 (dd, J = 3.2, 5.6 Hz, 1 H), 7.74 (d, J = 6.8 Hz, 1 H), 7.40 (t, J = 9.0 Hz, 1 H), 6.90 (d, J = 6.8 Hz, 1 H), 5.30 - 5.17 (m, 1 H), 4.50 (s, 3 H), 4.19 (d, J = 11.7 Hz, 2 H), 3.67 (t, J = 10.5 Hz, 2 H), 3.43 - 3.31 (m, 1 H), 2.33 - 2.22 (m, 2 H), 2.07 (d, J = 13.7 Hz, 2 H), 1.77 (d, J = 6.8 Hz, 6 H). LC-MS: m/z $[M+H]^+$ = 488.

**Example 15**

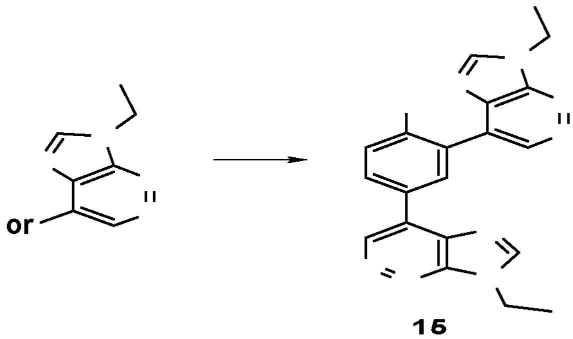

**15**

**15: 4,4'-(4-Fluoro-1,3-phenylene)bis(7-ethyl-7H-imidazo[4,5-c]pyridazine)**

Following the same experimental procedure as in Example 2, with starting materials of 7-ethyl-4-(4-fluoro-3-(4,4,5,5-tetramethyl-1,3,2-dioxaborolan-2-yl)phenyl)-7H-imidazo[4,5-c]pyridazine (100 mg, 0.3 mmol) and 4-chloro-7-ethyl-7H-imidazo[4,5-c]pyridazine (5 mg, 0.3 mmol), the title compound (17 mg, 16%) was obtained as a white solid.

$^1$H NMR (400 MHz, CHLOROFORM-d) 9.45 (br. s., 2 H) 8.84 - 9.02 (m, 1 H) 8.37 - 8.49 (m, 1 H) 8.30 (br. s., 2 H) 7.50 (br. s., 1 H) 4.49 - 4.69 (m, 4 H) 1.71 (q, J=7.01 Hz, 6 H). LC-MS: m/z [M+H]$^+$ = 389.

**Example 16**

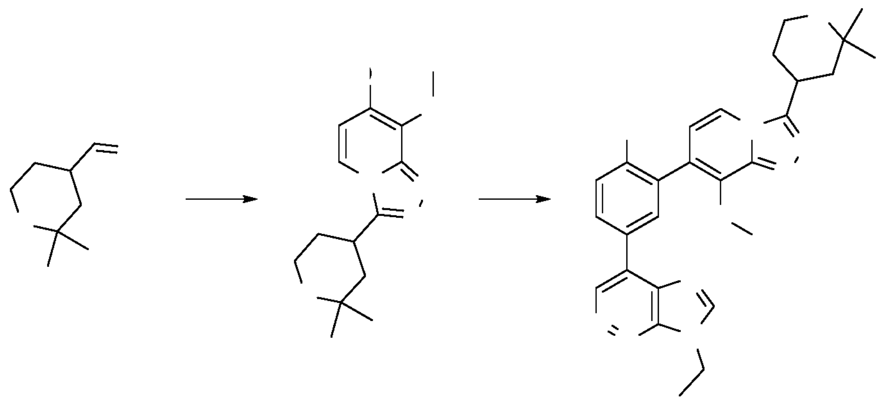

**16-1: 3-(2,2-Dimethyltetrahydro-2H-pyran-4-yl)-7-iodo-8-methoxy-[1,2,4] triazolo[4,3-a] pyridine**

Following the same experimental procedure as the synthesis method for 5-3 in Example 5, with starting materials of 2-hydrazineyl-4-iodo-3-methoxypyridine (300 mg, 1.132 mmol) and 2,2-dimethyltetrahydro-2H-pyran-4-carbaldehyde (161 mg, 1.132 mmol), the title compound (259 mg, 59%) was obtained as a brown solid. LC-MS: m/z [M+H]$^+$ = 388.

**16: 4-(3-(3-(2,2-Dimethyltetrahydro-2H-pyran-4-yl)-8-methoxy-[1,2,4]triazolo[4,3-a]pyridin-7-yl)-4-fluorophenyl)-7-ethyl-7H-imidazo[4,5-c]pyridazine**

Following the same experimental procedure as in Example 2, with starting materials of 7-ethyl-4-(4-fluoro-3-(4,4,5,5-tetramethyl-1,3,2-dioxaborolan-2-yl)phenyl)-7H-imidazo [4,5-c]pyridazine (50 mg, 0.13 mmol) and 3-(2,2-dimethyltetrahydro-2H-pyran-4-yl)-7-iodo-8-methoxy-[1,2,4]triazolo[4,3-a]pyridine (59 mg, 0.15 mmol), the title compound (34 mg, 53%) was obtained as a yellow solid.

$^1$H NMR (400MHz ,CHLOROFORM-d) 9.35 (s, 1 H), 8.32 (dd, J = 2.2, 7.1 Hz, 1 H), 8.29 - 8.23 (m, 2 H), 7.74 (d, J = 7.3 Hz, 1 H), 7.37 (t, J = 9.0 Hz, 1 H), 6.89 (d, J = 7.3 Hz, 1 H), 4.57 (q, J = 7.3 Hz, 2 H), 4.46 (s, 3 H), 3.97 - 3.84 (m, 2 H), 3.53 - 3.43 (m, 1 H), 2.16 - 2.07 (m, 2 H), 1.99 - 1.92 (m, 2 H), 1.67 (t, J = 7.3 Hz, 3 H), 1.39 (s, 3 H), 1.32 (s, 3 H). LC-MS: m/z [M+H]$^+$ = 502.

**Example 17**

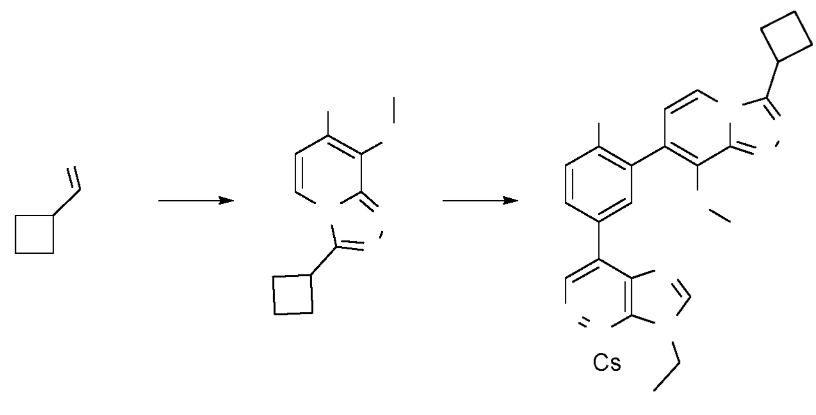

**17-1: 3-Cyclobutyl-7-iodo-8-methoxy-[1,2,4]triazolo[4,3-a]pyridine**

Following the same experimental procedure as the synthesis method for 5-3 in Example 5, with starting materials of 2-hydrazineyl-4-iodo-3-methoxypyridine (300 mg, 1.132 mmol) and cyclobutanecarbaldehyde (95 mg, 1.132 mmol), the title compound (262 mg, 70%) was obtained as a brown solid. LC-MS: m/z $[M+H]^+$ = 330.

**17: 4-(3-(3-Cyclobutyl-8-methoxy-[1,2,4]triazolo[4,3-a]pyridin-7-yl)-4-fluorophenyl)-7-ethyl-7H-imidazo [4,5-c] pyridazine**

Following the same experimental procedure as in Example 2, with starting materials of 7-ethyl-4-(4-fluoro-3-(4,4,5,5-tetramethyl-1,3,2-dioxaborolan-2-yl)phenyl)-7H-imidazo[4,5-c]pyridazine (60 mg, 0.152 mmol) and 3-cyclobutyl-7-iodo-8-methoxy-[1,2,4]triazolo[4,3-a]pyridine (50 mg, 0.152 mmol), the title compound (34 mg, 50%) was obtained as a yellow solid.

$^1$H NMR (400MHz, CHLOROFORM-d) d = 9.37 (s, 1 H), 8.35 - 8.31 (m, 1 H), 8.30 - 8.23 (m, 2 H), 7.58 (d, J = 6.8 Hz, 1 H), 7.38 (t, J = 9.0 Hz, 1 H), 6.85 (d, J = 7.3 Hz, 1 H), 4.62 - 4.55 (m, 2 H), 4.48 (s, 3 H), 3.88 (quin, J = 8.4 Hz, 1 H), 2.76 - 2.54 (m, 4 H), 2.29 - 2.09 (m, 2 H), 1.68 (t, J = 7.3 Hz, 3 H). LC-MS: m/z $[M+H]^+$ = 444.

**Example 18**

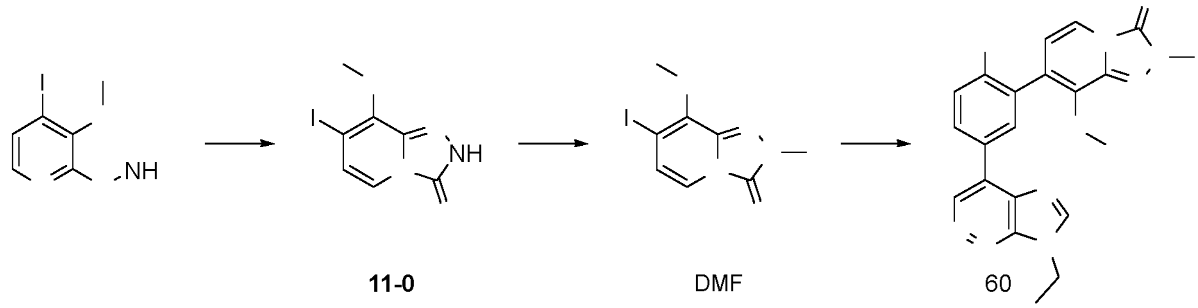

11-0

DMF

60

**18-1: 7-Iodo-8-methoxy-[1,2,4]triazolo[4,3-a]pyridin-3(2H)-one**

2-Hydrazineyl-4-iodo-3-methoxypyridine (450 mg, 1.698 mmol) and carbonyldiimidazole (550 mg, 3.400 mmol) were added to acetonitrile (4 mL), and the mixture was allowed to react at 85 °C for 2 h. The reaction mixture was filtered under vacuum, and the resulting solid was washed with water. The mixture was then filtered under vacuum to obtain the title compound (370 mg, 75%). LC-MS: m/z $[M+H]^+$ = 292.

**18-2: 7- Iodo-8-methoxy-2-methyl- [1,2,4]triazolo [4,3-a] pyridin-3(2H)-one**

7-Iodo-8-methoxy-[1,2,4]triazolo[4,3-a]pyridin-3(2H)-one (60 mg, 0.206 mmol), iodomethane (32 mg, 0.227 mmol), and cesium carbonate (134 mg, 0.412 mmol) were added to acetonitrile (1 mL), and the mixture was stirred at room temperature for 30 min. The reaction mixture was filtered under vacuum and concentrated. The residue was subjected to preparative thin-layer chromatography (petroleum ether/ethyl acetate = 1/1) to obtain the title compound (39 mg, 62%) as a white solid. LC-MS: m/z $[M+H]^+$ = 306.

**18: 7-(5-(7-Ethyl-7H-imidazo[4,5-c]pyridazin-4-yl)-2-fluorophenyl)-8-methoxy-2-methyl-[1,2,4] triazolo [4,3-a] pyridin-3(2H)-one**

Following the same experimental procedure as in Example 2, with starting materials of 7-ethyl-4-(4-fluoro-3-(4,4,5,5-tetramethyl-1,3,2-dioxaborolan-2-yl)phenyl)-7H-imidazo [4,5-c]pyridazine (42 mg, 0.115 mmol) and 3-(4-fluoro-1-methylpiperidin-4-yl)-7-iodo-8-methoxy-[1,2,4]triazolo[4,3-a]pyridine (35 mg, 0.115 mmol), the title compound (13 mg, 27%) was obtained as a yellow solid.

$^1$H NMR (400MHz, CHLOROFORM-d) 9.36 (s, 1 H), 8.32 - 8.25 (m, 3 H), 7.62 (d, J = 6.8 Hz, 1 H), 7.38 (t, J = 9.0 Hz, 1 H), 6.56 (d, J = 6.8 Hz, 1 H), 4.59 (q, J = 7.2 Hz, 2 H), 4.14 (s, 3 H), 3.73 (s, 3 H), 1.69 (t, J = 7.1 Hz, 3 H). LC-MS: m/z $[M+H]^+$ = 420.

**Example 19**

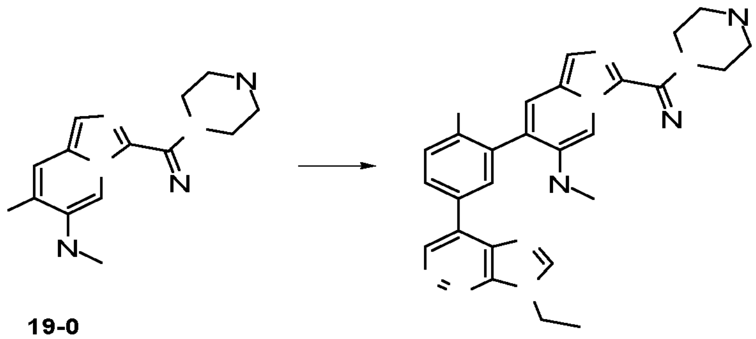

**19-0**

**19: (7-(5-(7-Ethyl-7H-imidazo[4,5-c]pyridazin-4-yl)-2-fluorophenyl)-6-methoxyimidazo[1,5-a]pyridin-3-yl)(morpholino)methanone**

7-Ethyl-4-(4-fluoro-3-(4,4,5,5-tetramethyl-1,3,2-dioxaborolan-2-yl)phenyl)-7H-imidazo[4,5-c]pyridazine (60 mg, 0.15 mmol), (7-bromo-6-methoxyimidazo[1,5-a]pyridin-3-yl)(morpholino)methanone (50 mg, 0.15 mmol), [1,1'-bis(diphenylphosphino)ferrocene]palladium dichloride dichloromethane complex (CAS: 95464-05-4, 12 mg, 0.02 mmol), and cesium carbonate (200 mg, 0.3 mmol) were successively added to 5 mL of dioxane and 0.5 mL of water, and then the mixture was stirred at 95 °C for 2 h. The reaction mixture was filtered and concentrated. The residue was purified by a preparative plate (dichloromethane:methanol = 20: 1) to obtain the title compound (24 mg, 33%) as a white solid.

$^1$H NMR (400 MHz, CHLOROFORM-d) 9.38 (s, 1 H) 9.06 (s, 1 H) 8.29 (s, 3 H) 7.51 - 7.65 (m, 2 H) 7.37 (t, J=8.80 Hz, 1 H) 4.59 (q, J=7.34 Hz, 4H) 3.89 (s, 3 H) 3.84 (br. s., 6 H) 1.70 (t, J=7.34 Hz, 3 H). LC-MS: m/z $[M+H]^+$ = 502.

Example 20

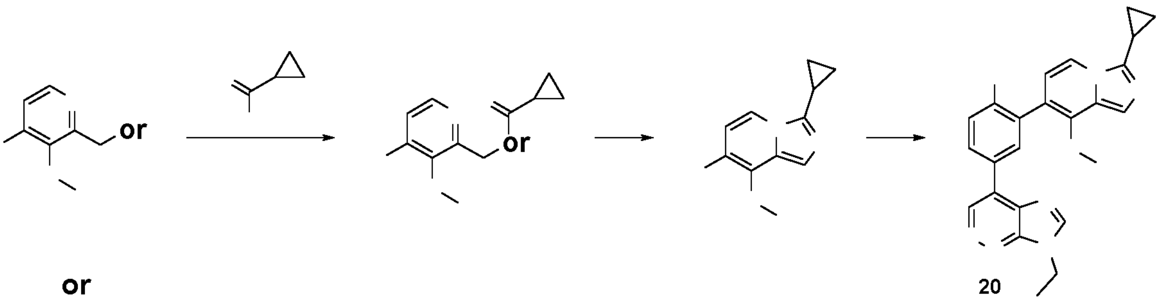

**or**

**20**

**20-1: N-((4-Chloro-3-methoxypyridin-2-yl)methyl)cyclopropanecarboxamide**

(4-Chloro-3-methoxypyridin-2-yl)methanamine (900 mg, 5.2 mmol) and potassium carbonate (2.2 g, 15.7 mmol) were dissolved in acetonitrile (20 mL). Cyclopropyl chloride (816 mg, 7.8 mmol) was then added in an ice-water bath, and the mixture was stirred at room temperature for 2 h. The reaction mixture was directly concentrated to dryness by rotary evaporation. The residue was separated by column chromatography (petroleum ether:ethyl acetate = 1:1) to obtain a yellow solid (300 mg, yield: 24%). LC-MS: m/z $[M+H]^+$= 241.

**20-2: 7-Chloro-3-cyclopropyl-8-methoxyimidazo[1,5-a]pyridine**

N-((4-Chloro-3-methoxypyridin-2-yl)methyl)cyclopropanecarboxamide (200 mg, 0.83 mmol), a Burgess reagent (500 mg, 2.1 mmol), and dichloromethane (5 mL) were added into a reaction flask, and the mixture was stirred at room temperature for 2 h and then directly concentrated to dryness by rotary evaporation. The residue was purified by column chromatography (petroleum ether:ethyl acetate = 1:1) to obtain a yellow oily liquid (110 mg, yield: 60%). LC-MS: m/z $[M+H]^+$ = 223.

**20: 4-(3-(3-Cyclopropyl-8-methoxyimidazo[1,5-a]pyridin-7-yl)-4-fluorophenyl)-7-ethyl-7H-imidazo[4,5-c]pyridazine**

7-Chloro-3-cyclopropyl-8-methoxyimidazo[1,5-a]pyridine (50 mg, 0.23 mmol), 7-ethyl-4-(4-fluoro-3-(4,4,5,5-tetramethyl-1,3,2-dioxaborolan-2-yl)phenyl)-7H-imidazo[4,5-c]pyridazine (97 mg, 0.34 mmol), [1,1-bis(di-tert-butylphosphino)ferrocene]palladium(II) dichloride (15 mg, 0.023 mmol), and cesium carbonate (150 mg, 0.46 mmol) were added to 1,4-dioxane/water (5 mL/1 mL). The mixture was allowed to react under microwave irradiation at 100 °C for 2 h under an argon atmosphere. The mixture was then filtered, and the filtrate was concentrated to dryness by rotary evaporation. The residue was subjected to prep-HPLC and lyophilized to obtain the title product (26 mg, yield: 27%).

$^1H$ NMR (400 MHz, DMSO-d6) 9.57 (s, 1H), 8.87 (s, 1H), 8.57 (dd, J = 7.2, 2.3 Hz, 1H), 8.52-8.36 (m, 1H), 8.20 (d, J = 7.2 Hz, 1H), 7.62-7.45 (m, 2H), 6.74 (d, J = 7.2 Hz, 1H), 4.52 (q, J = 7.2 Hz, 2H), 3.95 (s, 3H), 2.41-2.30 (m, 1H), 1.56 (t, J = 7.3 Hz, 3H), 1.15-1.03 (m, 2H), 1.00-0.84 (m, 2H). LC-MS: m/z $[M+H]^+$ = 429.

**Example 21**

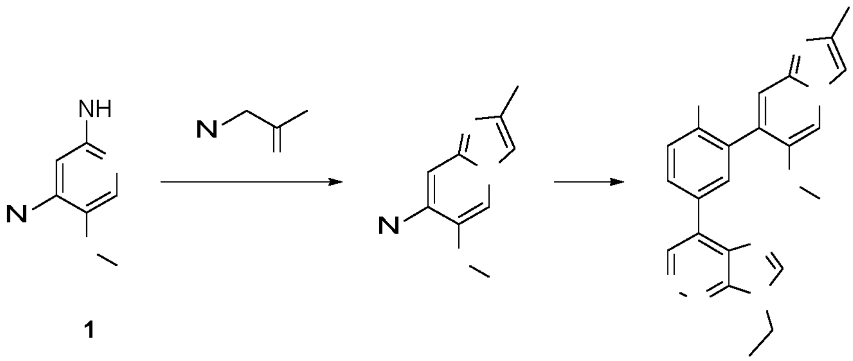

1

7-Chloro-6-methoxy-2-methylimidazo[1,2-a]pyridine

4-Chloro-5-methoxypyridin-2-amine (20 mg, 0.13 mmol) was added to ethanol (2 mL), and then chloroacetone (35 mg, 0.38 mmol) and sodium carbonate (40 mg, 0.38 mmol) were added. The mixture was heated to 100 °C with microwave irradiation and allowed to react for 1 h, then filtered, and concentrated. The residue was purified by column chromatography to obtain the title product (5 mg, yield: 20%). LC-MS: m/z $[M+H]^+$ = 197.

7-Ethyl-4-(4-fluoro-3-(6-methoxy-2-methylimidazo[1,2-a]pyridin-7-yl)phenyl)-7H-imidazo[4,5-c]pyridazine

7-Chloro-6-methoxy-2-methylimidazo[1,2-a]pyridine (72 mg, 0.367 mmol), (5-(7-ethyl-7H-imidazo[4,5-c]pyridazin-4-yl)-2-fluorophenyl)boronic acid (105 mg, 0.367 mmol), [1,1-bis(di-*tert*-butylphosphino)ferrocene]palladium(II) dichloride (24 mg, 0.0367 mmol), and cesium carbonate (239 mg, 0.735 mmol) were added to 1,4-dioxane/water (7.2 mL/0.8 mL). The mixture was allowed to react at 95 °C for 2 h under an argon atmosphere. 10 mL of water was added, and the mixture was extracted with ethyl acetate (20 mL × 2). The organic phase was dried over anhydrous sodium sulfate and concentrated. The residue was purified by prep-HPLC (0.5% NH3·H2O-acetonitrile) to obtain a white solid (40 mg, yield: 31%).

$^1H$ NMR (400 MHz, $CDCl_3$) 9.38 (s, 1H), 8.40-8.16 (m, 3H), 7.71 (s, 2H), 7.43-7.29 (m, 2H), 4.58 (q, J = 7.3 Hz, 2H), 3.84 (s, 3H), 2.51 (s, 3H), 1.69 (t, J = 7.3 Hz, 3H). LC-MS: m/z $[M+H]^+$ = 403.

**Example 22**

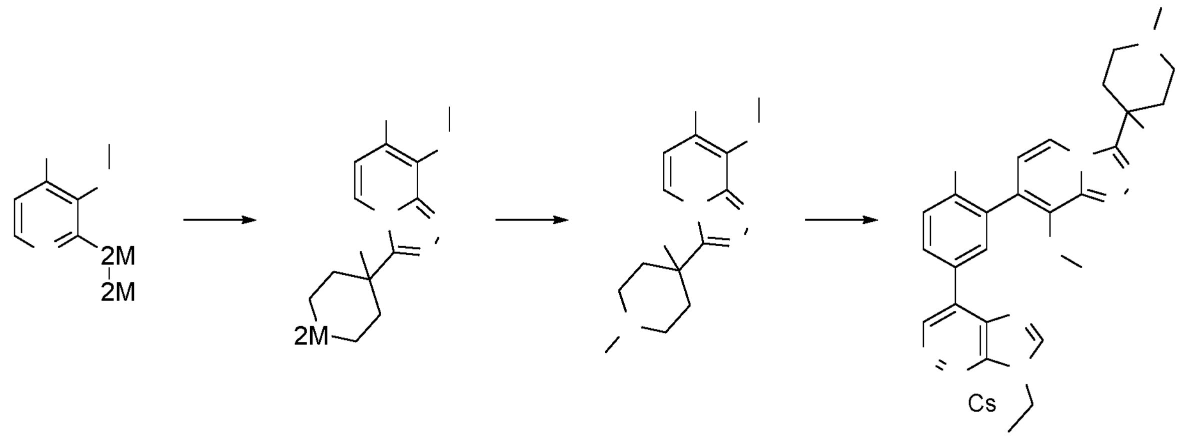

22-1: 3-(4-Fluoropiperidin-4-yl)-7-iodo-8-methoxy-[1,2,4]triazolo[4,3-a]pyridine

2-Hydrazineyl-4-iodo-3-methoxypyridine (300 mg, 0.822 mmol) and *tert*-butyl 4-fluoro-4-formylpiperidine-1-carboxylate (285 mg, 1.233 mmol) were added to ethanol (2 mL), and the mixture was stirred at 25 °C for 2 h. Copper bromide (37 mg, 0.164 mmol) and potassium monopersulfate (606 mg, 0.986 mmol) were added to the reaction mixture, and the mixture was stirred at 25 °C for 1.5 h. The reaction mixture was filtered under vacuum and concentrated. The residue was dissolved in dichloromethane (5 mL). A hydrochloric acid-ethyl acetate solution (4 mol/L, 2 mL) was added to the reaction mixture, and the mixture was stirred at 25 °C for 1 h. The reaction mixture was concentrated and evaporated to dryness. The residue was subjected to preparative thin-layer chromatography (dichloromethane/methanol = 15/1) to obtain the title compound (179 mg, 61%) as a yellow solid. LC-MS: m/z $[M+H]^+$ = 377.

**22-2: 3-(4-Fluoro-1-methylpiperidin-4-yl)-7-iodo-8-methoxy-[1,2,4]triazolo[4,3-a]pyridine**

3-(4-Fluoropiperidin-4-yl)-7-iodo-8-methoxy-[1,2,4]triazolo[4,3-a]pyridine (179 mg, 0.500 mmol) and an aqueous formaldehyde solution (60 mg, 1 mmol) were added to tetrahydrofuran (1 mL), and the mixture was stirred at 25 °C for 5 min. Sodium cyanoborohydride (63 mg, 1 mmol) was added to the reaction mixture, and the mixture was stirred at 25 °C for 1 h. The reaction mixture was filtered under vacuum and concentrated. The residue was subjected to preparative thin-layer chromatography (dichloromethane/methanol = 20/1) to obtain the title compound (79 mg, 42%) as a brown solid. LC-MS: m/z $[M+H]^+$ = 391.

**22: 7-Ethyl-4-(4-fluoro-3-(3-(4-fluoro-1-methylpiperidin-4-yl)-8-methoxy-[1,2,4]triazolo[4,3-a]pyridin-7-yl)phenyl)-7H-imidazo[4,5-c]pyridazine**

Following the same experimental procedure as in Example 2, with starting materials of 7-ethyl-4-(4-fluoro-3-(4,4,5,5-tetramethyl-1,3,2-dioxaborolan-2-yl)phenyl)-7H-imidazo [4,5-c]pyridazine (71 mg, 0.195 mmol) and 3-(4-fluoro-1-methylpiperidin-4-yl)-7-iodo-8-methoxy-[1,2,4]triazolo[4,3-a]pyridine (76 mg, 0.195 mmol), the title compound (6 mg, 6%) was obtained as a pale yellow solid.

$^1H$ NMR (400MHz, CHLOROFORM-d) 9.38 (s, 1 H), 8.36 (d, J = 6.8 Hz, 1 H), 8.28 (s, 2 H), 8.15 (d, J = 6.8 Hz, 1 H), 7.41 (t, J = 9.0 Hz, 1 H), 6.93 (d, J = 7.3 Hz, 1 H), 4.59 (q, J = 7.3 Hz, 2 H), 4.48 (s, 3 H), 3.04 (br. s., 2 H), 2.88 - 2.57 (m, 6 H), 2.53 (s, 3 H), 1.70 (br. s., 3 H). LC-MS: m/z $[M+H]^+$ = 505.

**Example 23**

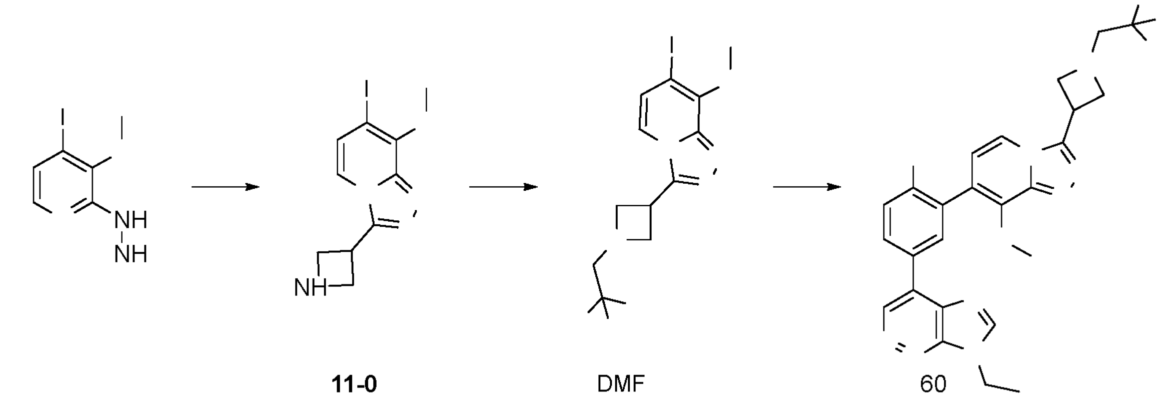

11-0 DMF 60

**23-1: 3-(Azetidin-3-yl)-7-iodo-8-methoxy-[1,2,4]triazolo[4,3-a]pyridine**

Following the same experimental procedure as the synthesis method for 22-1 in Example 22, with starting materials of 2-hydrazineyl-4-iodo-3-methoxypyridine (250 mg, 0.94 mmol) and *tert*-butyl 3-formylazetidine-1-carboxylate (349 mg, 1.89 mmol), the title compound (136 mg, 44%) was obtained as a brown solid. LC-MS: m/z $[M+H]^+$ = 331.

**23-2: 7-Iodo-8-methoxy-3-(1-(2,2,2-trifluoroethyl)azetidin-3-yl)-[1,2,4]triazolo[4,3-a]pyridine**

3-(Azetidin-3-yl)-7-iodo-8-methoxy-[1,2,4]triazolo[4,3-a]pyridine (136 mg, 0.41 mmol), 2,2,2-trifluoroethyl trifluoromethanesulfonate (113 mg, 0.50 mmol), and potassium carbonate (114 mg, 0.82 mmol) were added to acetonitrile (3 mL), and the mixture was allowed to react at 50 °C for 2 h. The reaction mixture was filtered under vacuum and concentrated. The residue was subjected to preparative thin-layer chromatography (dichloromethane/methanol = 30/1) to obtain the title compound (70 mg, 41%) as a yellow gelatinous solid. LC-MS: m/z $[M+H]^+$ = 413.

**23: 7-Ethyl-4-(4-fluoro-3-(8-methoxy-3-(1-(2,2,2-trifluoroethyl)azetidin-3-yl)-[1,2,4]triazolo[4,3-a]pyridin-7-yl) phenyl)-7H-imidazo[4,5-c]pyridazine**

Following the same experimental procedure as in Example 2, with starting materials of 7-ethyl-4-(4-fluoro-3-(4,4,5,5-tetramethyl-1,3,2-dioxaborolan-2-yl)phenyl)-7H-imidazo[4,5-c]pyridazine (62 mg, 0.17 mmol) and 7-iodo-8-methoxy-3-(1-(2,2,2-trifluoroethyl)azetidin-3-yl)-[1,2,4]triazolo[4,3-a]pyridine (70 mg, 0.17 mmol), the title compound (22 mg, 25%) was obtained as a pale yellow solid.

$^1H$ NMR (400MHz ,CHLOROFORM-d) 9.36 (s, 1 H), 8.34 (d, J = 5.9 Hz, 1 H), 8.28 (s, 2 H), 7.98 (d, J = 6.8 Hz, 1 H), 7.39 (t, J = 9.0 Hz, 1 H), 6.93 (d, J = 6.8 Hz, 1 H), 4.58 (q, J = 7.3 Hz, 2 H), 4.46 (s, 3 H), 4.27 (quin, J = 7.5 Hz, 1 H), 4.09 (t, J = 7.6 Hz, 2 H), 3.88 (t, J = 7.1 Hz, 2 H), 3.17 (q, J = 9.3 Hz, 2 H), 1.68 (t, J = 7.3 Hz, 3 H). LC-MS: m/z $[M+H]^+$ = 527.

## Example 24

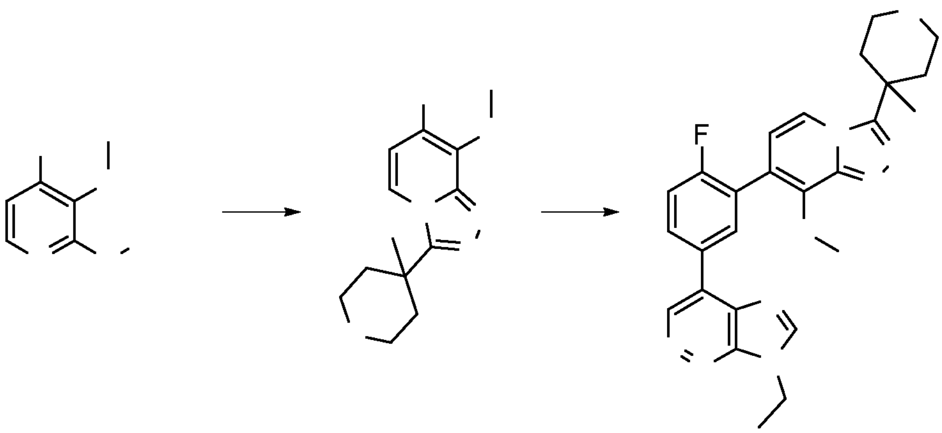

**24-1: 7-Iodo-8-methoxy-3-(4-methyltetrahydro-2H-pyran-4-yl)-[1,2,4] triazolo[4,3-a] pyridine**

Following the same experimental procedure as the synthesis method for 5-3 in Example 5, with starting materials of 2-hydrazineyl-4-iodo-3-methoxypyridine (100 mg, 0.38 mmol) and 4-methyltetrahydro-2H-pyran-4-carbaldehyde (96 mg, 0.75 mmol), the title compound (113 mg, 80%) was obtained as a brownish-red solid. LC-MS: m/z $[M+H]^+$ = 374.

**24: 7-Ethyl-4-(4-fluoro-3-(8-methoxy-3-(4-methyltetrahydro-2H-pyran-4-yl)-[1,2,4]triazolo[4,3-a]pyridin-7-yl) phenyl)-7H-imidazo[4,5-c]pyridazine**

Following the same experimental procedure as in Example 2, with starting materials of 7-ethyl-4-(4-fluoro-3-(4,4,5,5-tetramethyl-1,3,2-dioxaborolan-2-yl)phenyl)-7H-imidazo[4,5-c]pyridazine (54 mg, 0.15 mmol) and 7-iodo-8-methoxy-3-(4-methyltetrahydro-2H-pyran-4-yl)-[1,2,4]triazolo[4,3-a]pyridine (55 mg, 0.15 mmol), the title compound (19 mg, 27%) was obtained as a yellow solid.

$^1$H NMR (400MHz ,CHLOROFORM-d) 9.38 (s, 1 H), 8.35 (d, J = 5.9 Hz, 1 H), 8.28 (s, 2 H), 7.94 (d, J = 7.3 Hz, 1 H), 7.40 (t, J = 9.0 Hz, 1 H), 6.87 (d, J = 6.8 Hz, 1 H), 4.59 (q, J = 7.3 Hz, 2 H), 4.49 (s, 3 H), 3.93 - 3.85 (m, 2 H), 3.83 - 3.73 (m, 2 H), 2.60 (d, J = 14.2 Hz, 2 H), 2.00 - 1.91 (m, 2 H), 1.69 (br. s., 3 H), 1.59 (s, 3 H). LC-MS: m/z $[M+H]^+$ = 488.

**Example 25**

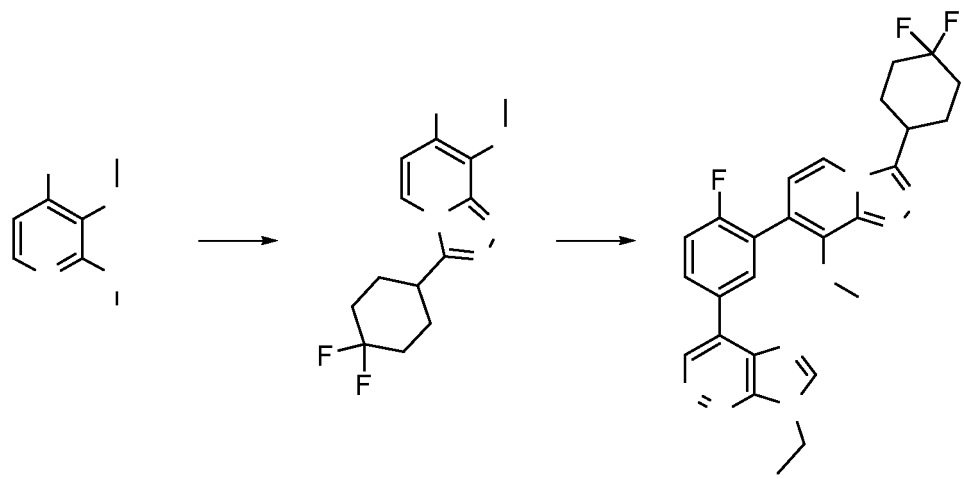

**25-1: 3-(4,4-Difluorocyclohexyl)-7-iodo-8-methoxy-[1,2,4]triazolo[4,3-a]pyridine**

Following the same experimental procedure as the synthesis method for 5-3 in Example 5, with starting materials of 2-hydrazineyl-4-iodo-3-methoxypyridine (100 mg, 0.38 mmol) and 4,4-difluorocyclohexane-1-carbaldehyde (112 mg, 0.75 mmol), the title compound (100 mg, 67%) was obtained as a brownish-red solid. LC-MS: m/z $[M+H]^+$ = 394.

**25: 4-(3-(3-(4,4-Difluorocyclohexyl)-8-methoxy-[1,2,4]triazolo[4,3-a]pyridin-7-yl)-4-fluorophenyl)-7-ethyl-7H-imidazo[4,5-c] pyridazine**

Following the same experimental procedure as in Example 2, with starting materials of 7-ethyl-4-(4-fluoro-3-(4,4,5,5-tetramethyl-1,3,2-dioxaborolan-2-yl)phenyl)-7H-imidazo [4,5-c]pyridazine (52 mg, 0.14 mmol) and 3-(4,4-difluorocyclohexyl)-7-iodo-8-methoxy-[1,2,4]triazolo[4,3-a]pyridine (55 mg, 0.14 mmol), the title compound (13 mg, 20%) was obtained as a pale yellow solid.

$^1$H NMR (400MHz ,CHLOROFORM-d) 9.38 (br. s., 1 H), 8.34 (dd, J = 2.0, 6.8 Hz, 1 H), 8.30 - 8.25 (m, 2 H), 7.71 (d, J = 6.8 Hz, 1 H), 7.39 (t, J = 9.0 Hz, 1 H), 6.92 (d, J = 7.3 Hz, 1 H), 4.61 - 4.56 (m, 2 H), 4.49 (s, 3 H), 3.25 (br. s., 1 H), 2.39 (d, J = 9.3 Hz, 2 H), 2.29 - 2.20 (m, 4 H), 1.88 - 1.78 (m, 2 H), 1.69 (t, J = 7.3 Hz, 3 H). LC-MS: m/z $[M+H]^+$ = 508.

**Example 26**

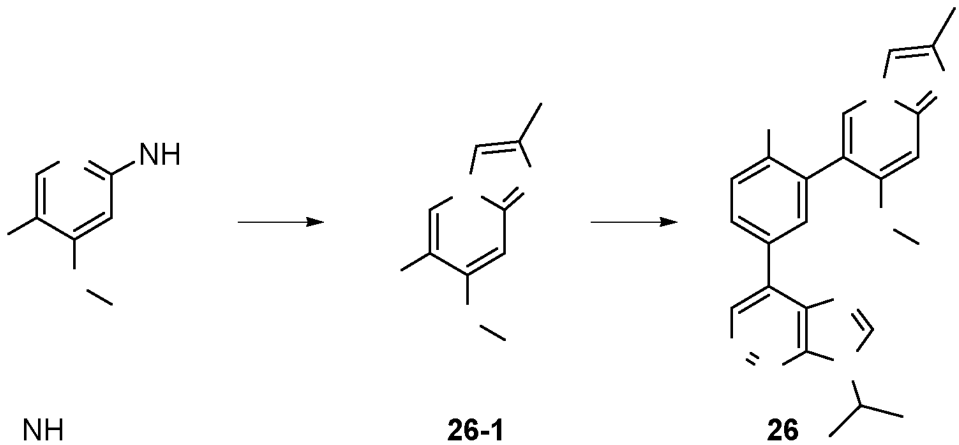


NH 26-1 26

**26-1: 6-Bromo-7-methoxy-2-methylimidazo[1,2-a]pyridine**

5-Bromo-4-methoxypyridin-2-amine (200 mg, 1 mmol) and bromoacetone (274 mg, 2 mmol) were added to anhydrous EtOH (5 mL), and the mixture was stirred at 90 °C overnight. The reaction mixture was concentrated. The residue was then separated and purified by a preparative plate (petroleum ether/ethyl acetate = 1/1) to obtain the title compound (120 mg, 50%).

**26: 4-(4-Fluoro-3-(7-methoxy-2-methylimidazo[1,2-a]pyridin-6-yl)phenyl)-7-isopropyl-7H-imidazo [4,5-c] pyridazine**

Following the same experimental procedure as in Example 2, with starting materials of 6-bromo-7-methoxy-2-methylimidazo[1,2-a]pyridine (24.2 mg, 0.1 mmol) and 7-isopropyl-4-(4-fluoro-3-(4,4,5,5-tetramethyl-1,3,2-dioxaborolan-2-yl)phenyl)-7H-imidazo[4,5-c]pyridazine (40 mg, 0.1 mmol), the title compound (17 mg, 42%) was obtained.

$^1$H NMR (400MHz, DMSO-d6) 9.55 - 9.51 (s, 1 H), 8.93 - 8.89 (s, 1 H), 8.64 - 8.41 (m, 4 H), 7.60 - 7.49 (m, 2 H), 5.12 - 5.06 (m, 1 H), 3.87 - 3.78 (m, 3 H), 2.39 - 2.24 (m, 3 H), 1.66 (d, J = 6.8 Hz, 6 H). LC-MS: m/z[M+H]$^+$= 417.

**Example 27**

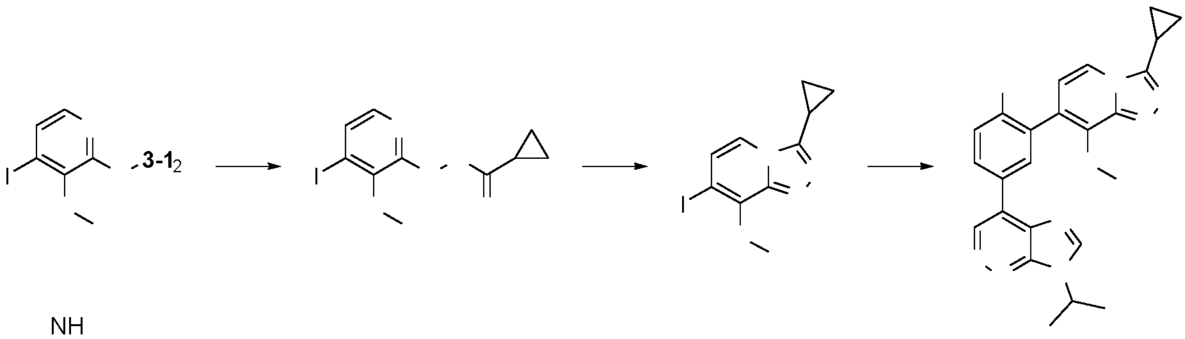


NH

**27-1: N'-(4-Iodo-3-methoxypyridin-2-yl)cyclopropanecarbohydrazide**

Following the same experimental procedure as the synthesis method for 3-3 in Example 3, with starting materials of 2-hydrazineyl-4-iodo-3-methoxypyridine (200 mg, 0.76 mmol) and cyclopropanecarboxylic acid (65 mg, 0.76 mmol), the title product (190.1 mg, white solid) was obtained with a yield of 76%. LC-MS: m/z [M+H]$^+$ = 334.

**27-2: 3-Cyclopropyl-7-iodo-8-methoxy-[1,2,4]triazolo[4,3-a]pyridine**

Following the same experimental procedure as the synthesis method for 3-4 in Example 3, with the starting material of N'-(4-iodo-3-methoxypyridin-2-yl)cyclopropanecarbohydrazide (150.0 mg, 0.45 mmol), the title product (60.1

mg, white solid) was obtained with a yield of 32%. LC-MS: m/z $[M+H]^+$ = 316.

**27: 4-(3-(3-Cyclopropyl-8-methoxy-[1,2,4]triazolo[4,3-a]pyridin-7-yl)-4-fluorophenyl)-7-isopropyl-7H-imidazo [4,5-c]pyridazine**

Following the same experimental procedure as in Example 2, with starting materials of 3-cyclopropyl-7-iodo-8-methoxy-[1,2,4]triazolo[4,3-a]pyridine (100.0 mg, 0.32 mmol) and (2-fluoro-5-(7-isopropyl-7H-imidazo[4,5-c]pyridazin-4-yl)phenyl)boronic acid (142.8 mg,0.48 mmol), the title compound (62.1 mg, white solid) was obtained with a yield of 37%.
$^1$H NMR (400 MHz, DMSO-d6) 9.57 (s, 1H), 8.94 (s, 1H), 8.65-8.48 (m, 2H), 8.40 (d, J = 7.0 Hz, 1H), 7.59 (t, J = 9.2 Hz, 1H), 7.07 (d, J = 7.0 Hz, 1H), 5.13 (m, 1H), 4.31 (s, 3H), 2.44 (m, 1H), 1.68 (d, J = 6.7 Hz, 6H), 1.27-0.98 (m, 4H). LC-MS: m/z $[M+H]^+$ = 444.

**Example 28**

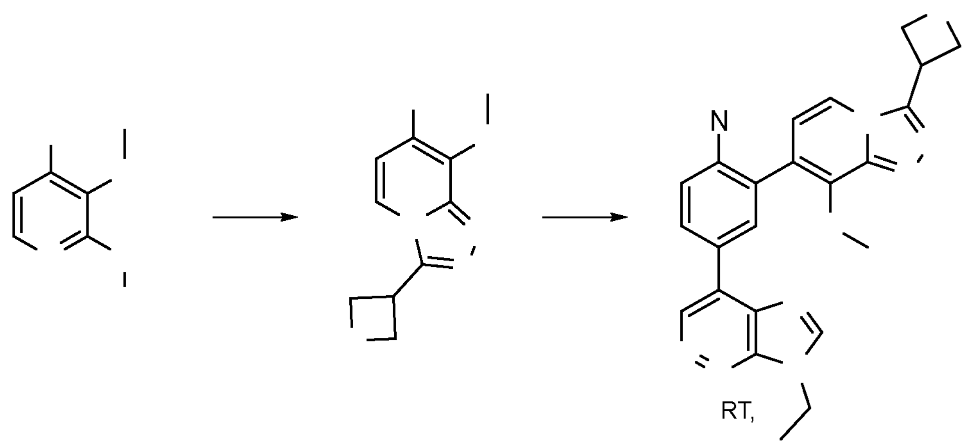


**28-1: 7-Iodo-8-methoxy-3-(oxetan-3-yl)-[1,2,4]triazolo[4,3-a]pyridine**

Following the same experimental procedure as the synthesis method for 5-3 in Example 5, with starting materials of 2-hydrazineyl-4-iodo-3-methoxypyridine (200 mg, 0.754 mmol) and oxetane-3-carbaldehyde (207 mg, 3.77 mmol), the title compound (151 mg, 61%) was obtained as a brown solid. LC-MS: m/z $[M+H]^+$ = 332.

**28: 7-Ethyl-4-(4-fluoro-3-(8-methoxy-3-(oxetan-3-yl)-[1,2,4]triazolo[4,3-a]pyridin-7-yl)phenyl)-7H-imidazo[4,5-c] pyridazine**

Following the same experimental procedure as in Example 2, with starting materials of 7-ethyl-4-(4-fluoro-3-(4,4,5,5-tetramethyl-1,3,2-dioxaborolan-2-yl)phenyl)-7H-imidazo[4,5-c]pyridazine (56 mg, 0.151 mmol) and 7-iodo-8-methoxy-3-(oxetan-3-yl)-[1,2,4]triazolo[4,3-a]pyridine (50 mg, 0.151 mmol), the title compound (26 mg, 39%) was obtained as a yellow solid.
$^1$H NMR (400MHz, CHLOROFORM-d) d = 9.38 (br. s., 1 H), 8.39 - 8.24 (m, 3 H), 7.92 - 7.85 (m, 1 H), 7.40 (t, J = 9.3 Hz, 1 H), 6.96 (d, J = 4.9 Hz, 1 H), 5.22 (d, J = 7.3 Hz, 4 H), 4.79 (br. s., 1 H), 4.63 - 4.55 (m, 2 H), 4.49 (d, J = 2.9 Hz, 3 H), 1.69 (br. s., 3 H). LC-MS: m/z $[M+H]^+$ = 446.

**Example 29**

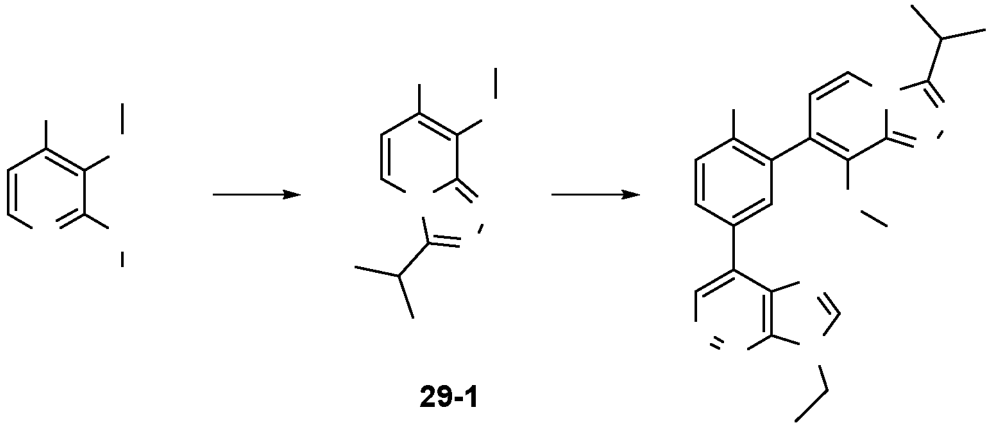

29-1

**29-1: 7-Iodo-8-methoxy-3-isopropyl-[1,2,4]triazolo[4,3-a]pyridine**

Following the same experimental procedure as the synthesis method for 5-3 in Example 5, with starting materials of 2-hydrazineyl-4-iodo-3-methoxypyridine (200 mg, 0.754 mmol) and isobutyraldehyde (271 mg, 3.77 mmol), the title compound (109 mg, 46%) was obtained as a brown solid. LC-MS: m/z $[M+H]^+$ = 318.

**29: 7-Ethyl-4-(4-fluoro-3-(8-methoxy-3-isopropyl-[1,2,4]triazolo[4,3-a]pyridin-7-yl)phenyl)-7H-imidazo[4,5-c] pyridazine**

Following the same experimental procedure as in Example 2, with starting materials of 7-ethyl-4-(4-fluoro-3-(4,4,5,5-tetramethyl-1,3,2-dioxaborolan-2-yl)phenyl)-7H-imidazo [4,5-c]pyridazine (58 mg, 0.158 mmol) and 7-iodo-8-methoxy-3-isopropyl-[1,2,4]triazolo[4,3-a]pyridine (50 mg, 0.158 mmol), the title compound (17 mg, 25%) was obtained as a yellow solid.

$^1$H NMR (400MHz, CHLOROFORM-d) 9.38 (br. s., 1 H), 8.37 - 8.23 (m, 3 H), 7.72 (d, J = 6.4 Hz, 1 H), 7.43 - 7.35 (m, 1 H), 6.89 (d, J = 6.8 Hz, 1 H), 4.59 (d, J = 7.3 Hz, 2 H), 4.48 (br. s., 3 H), 3.39 (d, J = 5.9 Hz, 1 H), 1.70 (d, J = 7.8 Hz, 3 H), 1.57 (d, J = 5.9 Hz, 6 H). LC-MS: m/z $[M+H]^+$ = 432.

**Example 30**

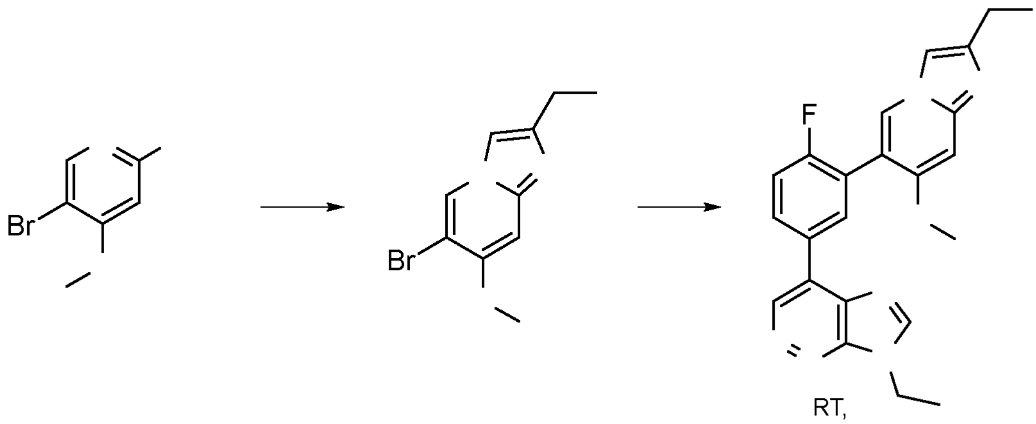


**30-1: 6-Bromo-2-ethyl-7-methoxyimidazo[1,2-a]pyridine**

5-Bromo-4-methoxypyridin-2-amine (200 mg, 0.99 mmol) and bromobutanone (747 mg, 4.95 mmol) were successively added to anhydrous ethanol (5 mL), and the mixture was stirred at 90 °C for 16 h. The reaction mixture was concentrated. The residue was separated by preparative thin-layer chromatography (petroleum ether/acetone = 4/1) to obtain the title compound (150 mg, 60%) as an oily liquid. LC-MS: m/z $[M+H]^+$ = 255, 257.

**30: 7-Ethyl-4-(3-(2-ethyl-7-methoxyimidazo[1,2-a]pyridin-6-yl)-4-fluorophenyl)-7H-imidazo[4,5-c]pyridazine**

Following the same experimental procedure as in Example 2, with starting materials of 6-bromo-2-ethyl-7-methoxyimidazo[1,2-a]pyridine (50 mg, 0.20 mmol) and 7-ethyl-4-(4-fluoro-3-(4,4,5,5-tetramethyl-1,3,2-dioxaborolan-2-yl)phenyl)-7H-imidazo[4,5-c]pyridazine (74 mg, 0.20 mmol), the title compound (10 mg, 12%) was obtained as a brown solid.

$^1$H NMR (400MHz, CHLOROFORM-d) 9.37 (s, 1 H), 8.28 (br. s., 3 H), 8.02 (br. s., 1 H), 7.36 (t, J = 8.8 Hz, 1 H), 7.21 (br. s., 1 H), 7.05 (br. s., 1 H), 4.62 - 4.55 (m, 2 H), 3.87 (br. s., 3H), 2.82 (d, J = 7.8 Hz, 2 H), 1.69 (t, J = 7.1 Hz, 3 H), 1.34 (d, J = 10.3 Hz, 3 H). LC-MS: m/z [M+H]$^+$ = 417.

**Example 31**

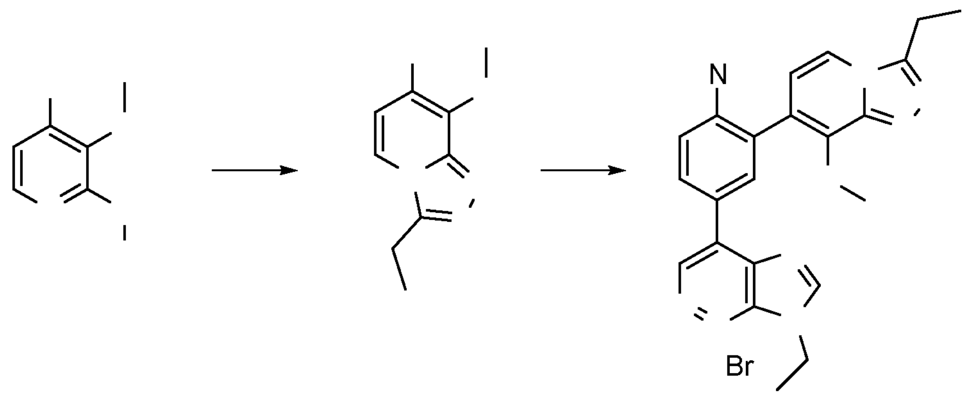

**31-1: 7-Iodo-8-methoxy-3-ethyl-[1,2,4]triazolo[4,3-a]pyridine**

Following the same experimental procedure as the synthesis method for 5-3 in Example 5, with starting materials of 2-hydrazineyl-4-iodo-3-methoxypyridine (200 mg, 0.75 mmol) and propionaldehyde (219 mg, 3.77 mmol), the title compound (147 mg, 64%) was obtained as a brown solid. LC-MS: m/z [M+H]$^+$ = 304.

**31: 7-Ethyl-4-(4-fluoro-3-(8-methoxy-3-ethyl-[1,2,4]triazolo[4,3-a]pyridin-7-yl)phenyl)-7H-imidazo[4,5-c] pyridazine**

Following the same experimental procedure as in Example 2, with starting materials of 7-ethyl-4-(4-fluoro-3-(4,4,5,5-tetramethyl-1,3,2-dioxaborolan-2-yl)phenyl)-7H-imidazo [4,5-c]pyridazine (60 mg, 0.165 mmol) and 7-iodo-8-methoxy-3-ethyl-[1,2,4]triazolo[4,3-a]pyridine (50 mg, 0.165 mmol), the title compound (23 mg, 33%) was obtained as a yellow solid.

$^1$H NMR (400MHz, CHLOROFORM-d) 9.37 (br. s., 1 H), 8.38 - 8.22 (m, 3 H), 7.68 (d, J = 4.4 Hz, 1 H), 7.38 (t, J = 9.0 Hz, 1 H), 6.89 (d, J = 5.9 Hz, 1 H), 4.58 (d, J = 7.3 Hz, 2 H), 4.46 (br. s., 3 H), 3.13 (d, J = 7.3 Hz, 2 H), 1.72 - 1.66 (m, 3 H), 1.56 - 1.51 (m, 3 H). LC-MS: m/z [M+H]$^+$ = 418.

**Example 32**

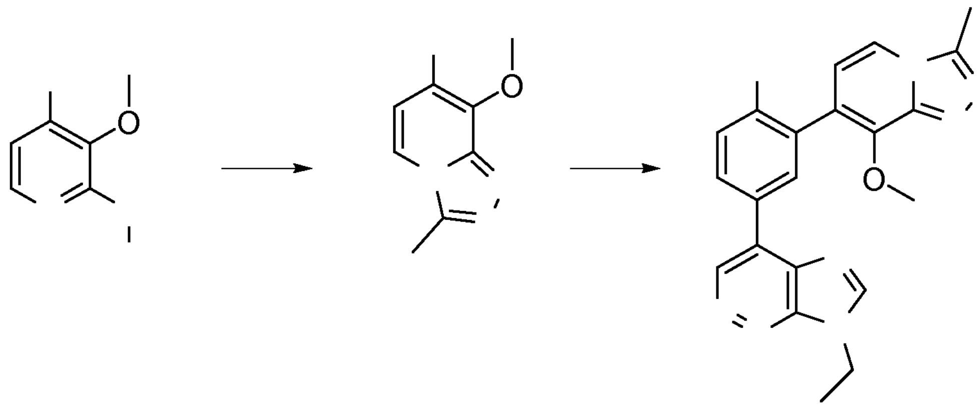

32-1: 7-Iodo-8-methoxy-3-methyl-[1,2,4]triazolo[4,3-a]pyridine

Following the same experimental procedure as the synthesis method for 5-3 in Example 5, with starting materials of 2-hydrazineyl-4-iodo-3-methoxypyridine (200 mg, 0.75 mmol) and an acetaldehyde-tetrahydrofuran solution (0.75 mL, 3.77 mmol), the title compound (140 mg, 64%) was obtained as a brown solid. LC-MS: m/z $[M+H]^+$ = 290.

**32: 7-Ethyl-4-(4-fluoro-3-(8-methoxy-3-methyl-[1,2,4]triazolo[4,3-a]pyridin-7-yl)phenyl)-7H-imidazo[4,5-c]pyridazine**

Following the same experimental procedure as in Example 2, with starting materials of 7-ethyl-4-(4-fluoro-3-(4,4,5,5-tetramethyl-1,3,2-dioxaborolan-2-yl)phenyl)-7H-imidazo [4,5-c]pyridazine (67 mg, 0.182 mmol) and 7-iodo-8-methoxy-3-methyl-[1,2,4]triazolo[4,3-a]pyridine (50 mg, 0.182 mmol), the title compound (27 mg, 37%) was obtained as a yellow solid.

$^1$H NMR (400MHz, CHLOROFORM-d) 9.38 (s, 1 H), 8.34 (d, J = 6.4 Hz, 1 H), 8.28 (s, 2 H), 7.66 (d, J = 6.8 Hz, 1 H), 7.39 (t, J = 9.0 Hz, 1 H), 6.91 (d, J = 6.8 Hz, 1 H), 4.58 (q, J = 7.0 Hz, 2 H), 4.47 (s, 3 H), 2.79 (s, 3 H), 1.69 (br. s., 3 H). LC-MS: m/z $[M+H]^+$ = 404.

**Example 33**

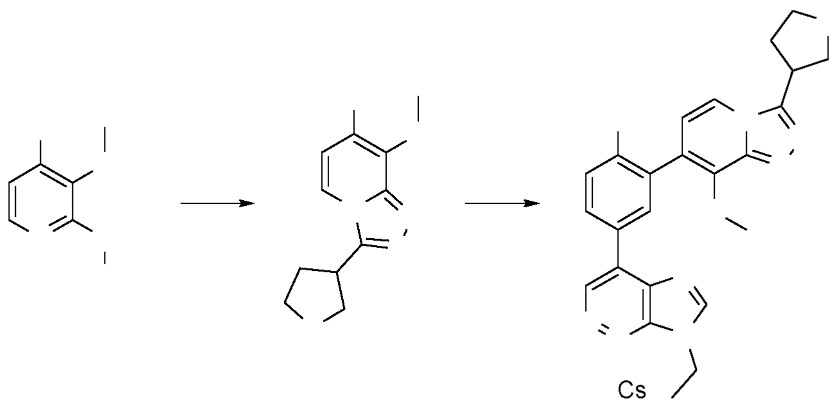

**33-1: 7-Iodo-8-methoxy-3-(tetrahydrofuran-3-yl)-[1,2,4]triazolo[4,3-a]pyridine**

Following the same experimental procedure as the synthesis method for 5-3 in Example 5, with starting materials of 2-hydrazineyl-4-iodo-3-methoxypyridine (100 mg, 0.38 mmol) and tetrahydrofuran-3-carbaldehyde (50 mg, 0.38 mmol), the title compound (140 mg, 100%) was obtained as a white solid. LC-MS: m/z $[M+H]^+$ = 346.

**33: 7-Ethyl-4-(4-fluoro-3-(8-methoxy-3-(tetrahydrofuran-3-yl)-[1,2,4]triazolo[4,3-a]pyridin-7-yl)phenyl)-7H-imidazo[4,5-c]pyridazine**

Following the same experimental procedure as in Example 2, with starting materials of 7-ethyl-4-(4-fluoro-3-(4,4,5,5-tetramethyl-1,3,2-dioxaborolan-2-yl)phenyl)-7H-imidazo [4,5-c]pyridazine (60 mg, 0.17 mmol) and 7-iodo-8-methoxy-3-(tetrahydrofuran-3-yl)-[1,2,4]triazolo[4,3-a]pyridine (50 mg, 0.17 mmol), the title compound (47 mg, 60%) was obtained as a yellow solid.

$^1H$ NMR (400 MHz, CHLOROFORM-d) 9.39 (s, 1 H) 8.35 (d, J=6.36 Hz, 1 H) 8.29 (s, 2 H) 7.83 (d, J=6.85 Hz, 1 H) 7.38 - 7.43 (m, 1 H) 6.93 (d,J=6.85 Hz, 1 H) 4.59 (q, J=6.85 Hz, 2 H) 4.49 (s, 3 H) 4.30 (d, J=8.31 Hz, 1 H) 4.23 (dd, J=15.41, 7.09 Hz, 2 H) 3.91 - 4.08 (m, 2 H) 2.53 (d, J=6.85 Hz, 2H) 1.69 - 1.72 (m, 3 H). LC-MS: m/z $[M+H]^+$= 460.

**Example 34**

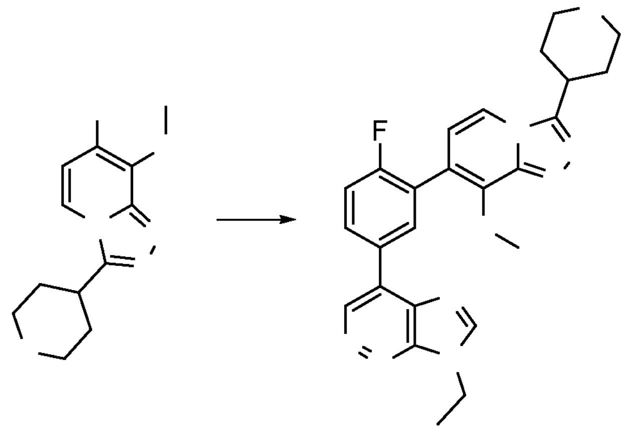

**34: 7-Ethyl-4-(4-fluoro-3-(8-methoxy-3-(tetrahydro-2H-pyran-4-yl)-[1,2,4]triazolo[4,3-a]pyridin-7-yl)phenyl)-7H-imidazo[4,5-c]pyridazine**

Following the same experimental procedure as in Example 2, with starting materials of 7-ethyl-4-(4-fluoro-3-(4,4,5,5-tetramethyl-1,3,2-dioxaborolan-2-yl)phenyl)-7H-imidazo [4,5-c]pyridazine (70 mg, 0.2 mmol) and 7-iodo-8-methoxy-3-(tetrahydro-2H-pyran-4-yl)-[1,2,4]triazolo[4,3-a]pyridine (50 mg, 0.2 mmol), the title compound (47 mg, 50%) was obtained as a brown solid.

$^1H$ NMR (400 MHz, CHLOROFORM-d) 9.39 (s, 1 H) 8.35 (d, J=5.87 Hz, 1 H) 8.29 (s, 2 H) 7.75 (d, J=6.85 Hz, 1 H) 7.40 (t, J=9.05 Hz, 1 H) 6.91(d, J=6.85 Hz, 1 H) 4.59 (q, J=7.34 Hz, 2 H) 4.50 (s, 3 H) 4.19 (d, J=11.25 Hz, 2 H) 3.67 (t, J=11.00 Hz, 2 H) 3.37 (t, J=11.00 Hz, 1 H) 2.20 - 2.35 (m, 2 H)2.08 (d, J=13.21 Hz, 2 H) 1.68 (br. s., 3 H). LC-MS: m/z $[M+H]^+$ = 474.

**Example 35**

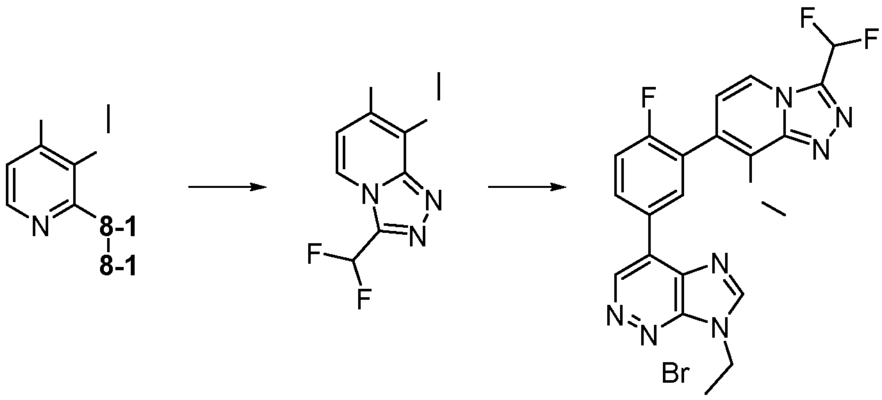

8-1

8-1

**35-1: 3-(Difluoromethyl)-7-iodo-8-methoxy-[1,2,4]triazolo[4,3-a]pyridine**

2-Hydrazineyl-4-iodo-3-methoxypyridine (160 mg, 0.57 mmol) was dissolved in 2 mL of difluoroacetic anhydride, and the resulting solution was heated at reflux overnight. The solution was then diluted with water, neutralized with a sodium bicarbonate solution to neutrality, extracted with dichloromethane, and concentrated. The residue was purified by a preparative plate (petroleum ether:ethyl acetate = 1:1) to obtain the title compound (150 mg, 82%) as a white solid. LC-MS: m/z $[M+H]^+$ = 326.

**35: 4-(3-(3-(Difluoromethyl)-8-methoxy-[1,2,4]triazolo[4,3-a]pyridin-7-yl)-4-fluorophenyl)-7-ethyl-7H-imidazo [4,5-c] pyridazine**

Following the same experimental procedure as in Example 2, with starting materials of 7-ethyl-4-(4-fluoro-3-(4,4,5,5-tetramethyl-1,3,2-dioxaborolan-2-yl)phenyl)-7H-imidazo [4,5-c]pyridazine (80 mg, 0.22 mmol) and 3-(difluoromethyl)-7-iodo-8-methoxy-[1,2,4]triazolo[4,3-a]pyridine (75 mg, 0.22 mmol), the title compound (39 mg, 41%) was obtained as a white solid.

$^1H$ NMR (400 MHz, CHLOROFORM-d) 9.38 (s, 1 H) 8.38 (d, J=6.85 Hz, 1 H) 8.29 (s, 2 H) 8.18 (d, J=6.85 Hz, 1 H) 7.42 (t, J=9.05 Hz, 1 H) 7.30(br. s., 1 H) 7.08 (d, J=6.85 Hz, 1 H) 4.59 (q, J=7.17 Hz, 2 H) 4.50 (s, 3 H) 1.67 - 1.70 (m, 3 H). LC-MS: m/z $[M+H]^+$ = 440.

**Example 36**

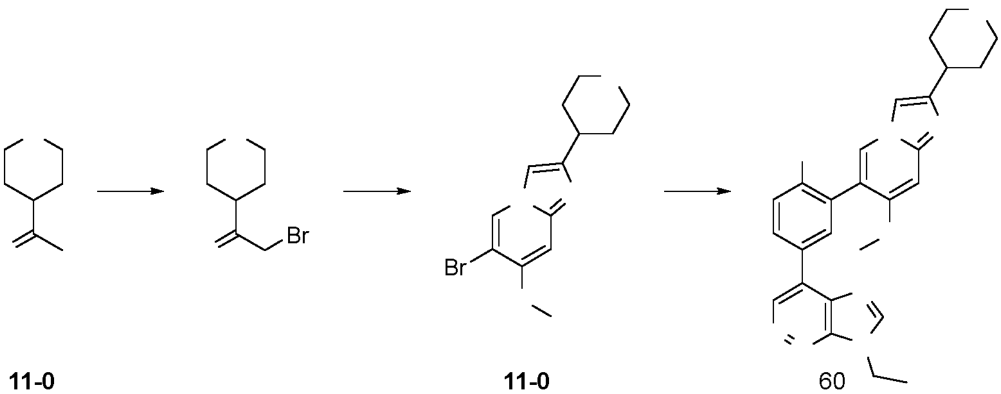

**11-0** **11-0** 60

**36-1: 2-Bromo-1-(tetrahydro-2H-pyran-4-yl)ethanone**

1-(Tetrahydro-2H-pyran-4-yl)ethanone (1000 mg, 7.8 mmol) was added to methanol (5 mL), and the mixture was cooled to 0 °C. Liquid bromine (0.4 mL, 7.8 mmol) was then added, and the mixture was stirred at 0 °C for 45 min and then stirred at room temperature for another 45 min. Sulfuric acid (2.7 mL, 30 mmol) was then added, and the mixture was stirred at room temperature overnight. The reaction mixture was quenched with an aqueous sodium bisulfite solution. Ethyl acetate (300 mL) was then added, and the ethyl acetate was washed with water (200 mL × 3), then dried, and concentrated

to obtain the title compound (600 mg, 37%).

**36-2: 6-Bromo-7-methoxy-2-(tetrahydro-2H-pyran-4-yl)imidazo[1,2-a]pyridine**

5-Bromo-4-methoxypyridin-2-amine (200 mg, 1 mmol) and 2-bromo-1-(tetrahydro-2H-pyran-4-yl)ethanone (600 mg, 3 mmol) were added to anhydrous ethanol (10 mL). The mixture was stirred at 80 °C overnight. The reaction mixture was directly subjected to thin-layer chromatography (petroleum ether/ethyl acetate = 3/1) to obtain the title compound (300 mg, 98%). LC-MS: m/z $[M+H]^+$ = 311.

**36: 7-Ethyl-4-(4-fluoro-3-(7-methoxy-2-(tetrahydro-2H-pyran-4-yl)imidazo[1,2-a]pyridin-6-yl)phenyl)-7H-imidazo[4,5-c]pyridazine**

Following the same experimental procedure as in Example 2, with starting materials of 6-bromo-7-methoxy-2-(tetrahydro-2H-pyran-4-yl)imidazo[1,2-a]pyridine (80 mg, 0.26 mmol) and 7-ethyl-4-(4-fluoro-3-(4,4,5,5-tetramethyl-1,3,2-dioxaborolan-2-yl)phenyl)-7H-imidazo[4,5-c]pyridazine (50 mg, 0.13 mmol), the title compound (16 mg, 25%) was obtained.

$^1$H NMR (400 MHz, CHLOROFORM-d) 1.70 (d,J=7.34 Hz, 3 H) 2.07 (br. s., 4 H) 3.08 (br. s., 1 H) 3.59 (br. s., 2 H) 3.89 (br. s., 3 H) 4.07 (br. s., 2 H) 4.58 (d,J=6.85 Hz, 2 H) 7.12 (br. s., 1 H) 7.22 (br. s., 1 H) 7.35 - 7.41 (m, 1 H) 8.06 (br. s., 1 H) 8.28 (br. s., 3 H) 9.37 (s, 1 H). LC-MS: m/z $[M+H]^+$ = 473.

**Example 37**

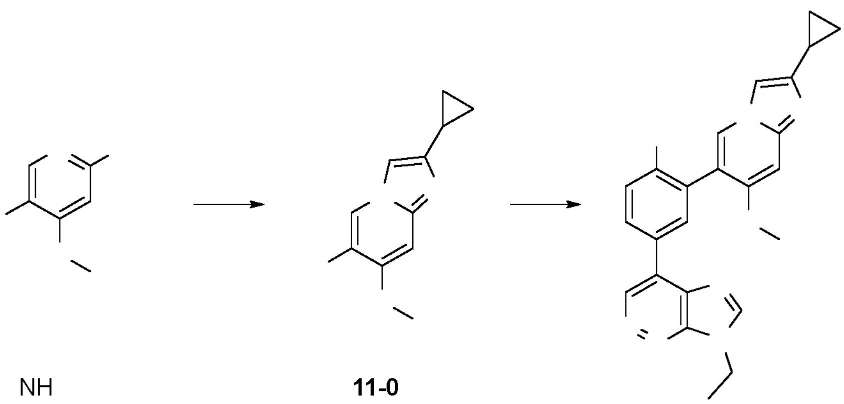

NH 11-0

**37-1: 6-Bromo-2-cyclopropyl-7-methoxyimidazo[1,2-a]pyridine**

5-Bromo-4-methoxypyridin-2-amine (2000 mg, 9.85 mmol) and 2-bromo-1-cyclopropylethanone (3211 mg, 19.7 mmol) were added to anhydrous ethanol (20 mL). The tube was sealed, and the mixture was stirred at 90 °C overnight. The reaction mixture was directly subjected to column chromatography (petroleum ether/ethyl acetate = 3/1) to obtain the title compound (1900 mg, 72%). LC-MS: m/z $[M+H]^+$ = 267.

**37: 4-(3-(2-Cyclopropyl-7-methoxyimidazo[1,2-a]pyridin-6-yl)-4-fluorophenyl)-7-ethyl-7H-imidazo[4,5-c]pyridazine**

Following the same experimental procedure as in Example 2, with starting materials of 6-bromo-2-cyclopropyl-7-methoxyimidazo[1,2-a]pyridine (150 mg, 0.56 mmol) and 7-ethyl-4-(4-fluoro-3-(4,4,5, 5-tetramethyl-1,3,2-dioxaborolan-2-yl)phenyl)-7H-imidazo[4,5-c]pyridazine (249 mg, 0.67 mmol), the title compound (83 mg, 34%) was obtained.

$^1$H NMR (400 MHz, DMSO-d6) 0.78 - 0.91 (m, 4 H) 1.54 (s, 3 H) 1.94 - 2.00 (m, 1 H) 3.79 (s, 3 H) 4.46 - 4.54 (m, 2 H) 6.96 (s, 1 H) 7.54 (s, 2 H) 8.11- 8.16 (m, 1 H) 8.47 (s, 1 H) 8.50 - 8.56 (m, 1 H) 8.85 (s, 1 H) 9.55 (s, 1 H). LC-MS: m/z $[M+H]^+$ = 429.

**Example 38**

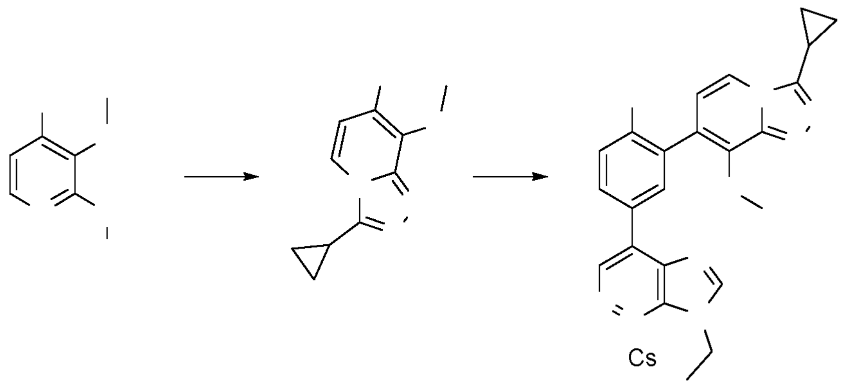

**38-1: 3-Cyclopropyl-7-iodo-8-methoxy-[1,2,4]triazolo[4,3-a]pyridine**

Following the same experimental procedure as the synthesis method for 5-3 in Example 5, with starting materials of 2-hydrazineyl-4-iodo-3-methoxypyridine (200 mg, 0.76 mmol) and cyclopropanecarboxaldehyde (53 mg, 0.76 mmol), the title compound (160 mg, 67%) was obtained as a white solid. LC-MS: m/z $[M+H]^+$ = 316.

**38: 4-(3-(3-Cyclopropyl-8-methoxy-[1,2,4]triazolo[4,3-a]pyridin-7-yl)-4-fluorophenyl)-7-ethyl-7H-imidazo[4,5-c]pyridazine**

Following the same experimental procedure as in Example 2, with starting materials of 7-ethyl-4-(4-fluoro-3-(4,4,5,5-tetramethyl-1,3,2-dioxaborolan-2-yl)phenyl)-7H-imidazo [4,5-c]pyridazine (70 mg, 0.2 mmol) and 3-cyclopropyl-7-iodo-8-methoxy-[1,2,4]triazolo[4,3-a]pyridine (50 mg, 0.2 mmol), the title compound (43 mg, 50%) was obtained as a yellow solid.

$^1$H NMR (400 MHz, CHLOROFORM-d) $\delta$ ppm 9.38 (s, 1 H) 8.34 (d, J=5.87 Hz, 1 H) 8.29 (br. s., 2 H) 7.91 (d, J=6.85 Hz, 1 H) 7.39 (t, J=9.05 Hz, 1 H) 6.92 (d, J=6.85 Hz, 1 H) 4.59 (q, J=6.85 Hz, 2 H) 4.38 - 4.52 (m, 3 H) 2.08 (br. s., 1 H) 1.70 (t, J=6.85 Hz, 3 H) 1.21 (d, J=7.83 Hz, 2 H) 0.88 (br. s, 2 H). LC-MS: m/z $[M+H]^+$ = 430.

**Example 39**

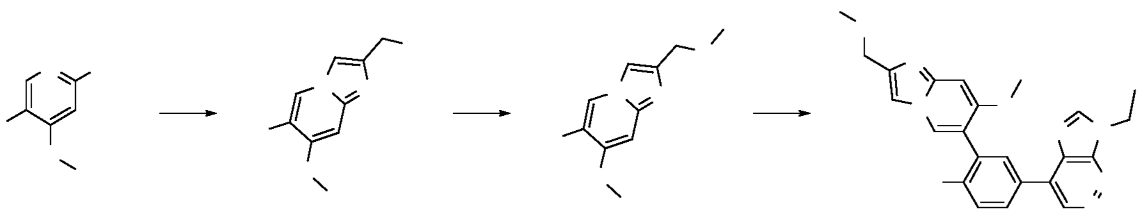

**20**

**39-1: 6-Bromo-2-(bromomethyl)-7-methoxyimidazo[1,2-a]pyridine**

5-Bromo-4-methoxypyridin-2-amine (300 mg, 1.48 mmol) and dibromoacetone (635 mg, 2.95 mmol) were added to anhydrous ethanol (4 mL), and the mixture was stirred at 90 °C for two hours. The reaction mixture was concentrated. The residue was separated and purified directly by a preparative plate (petroleum ether/ethyl acetate = 1/1) to obtain the title compound (210 mg, 45%). LC-MS: m/z $[M+H]^+$ = 321.

**39-2: 6-Bromo-7-methoxy-2-(methoxymethyl)imidazo[1,2-a]pyridine**

6-Bromo-2-(bromomethyl)-7-methoxyimidazo[1,2-a]pyridine (200 mg, 0.63 mmol) and sodium methoxide (0.5 mL, 4 mmol) were added to methanol (4 mL), and the mixture was stirred at room temperature for two hours. The reaction mixture was concentrated. The residue was separated and purified directly by a preparative plate (petroleum ether/ethyl acetate = 1/1) to obtain the title compound (35 mg, 21%). LC-MS: m/z $[M+H]^+$ = 271, 273.

**39: 7-Ethyl-4-(4-fluoro-3-(7-methoxy-2-(methoxymethyl)imidazo[1,2-a]pyridin-6-yl)phenyl)-7H-imidazo [4,5-c] pyridazine**

Following the same experimental procedure as in Example 2, with starting materials of 6-bromo-7-methoxy-2-(methoxymethyl)imidazo[1,2-a]pyridine (35 mg, 0.13 mmol) and 7-ethyl-4-(4-fluoro-3-(4,4,5,5-tetramethyl-1,3,2-dioxaborolan-2-yl)phenyl)-7H-imidazo[4,5-c]pyridazine (50 mg, 0.13 mmol), the title compound (21 mg, 38%) was obtained.

$^1$H NMR (400MHz, CDCl3) δ 9.37 (s, 1 H), 8.31 - 8.22 (m, 3 H), 8.05 (s, 1 H), 7.45 (s, 1 H), 7.40 - 7.33 (m, 1 H), 7.00 (br. s., 1 H), 4.65 - 4.54 (m, 4 H), 3.87 (s, 3 H), 3.48 (br. s., 3 H), 1.69 - 1.67 (m, 3 H). LC-MS: m/z [M+H]$^+$ = 433.

## Example 40

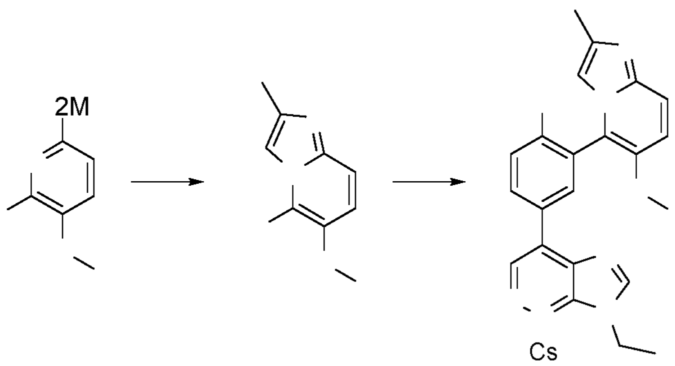

**40-1: 5-Bromo-6-methoxy-2-methylimidazo[1,2-a]pyridine**

6-Bromo-5-methoxypyridin-2-amine (90 mg, 0.45 mmol) and bromoacetone (120 mg, 0.88 mmol) were added to anhydrous ethanol (10 mL), and the mixture was allowed to react at 90 °C for 16 h and then concentrated. The residue was purified by a preparative plate (petroleum ether:ethyl acetate = 1:2) to obtain a yellow oil (35 mg, yield: 32%). LC-MS: m/z [M+H]$^+$ = 241, 243.

**40: 7-Ethyl-4-(4-fluoro-3-(6-methoxy-2-methylimidazo[1,2-a]pyridin-5-yl)phenyl)-7H-imidazo[4,5-c]pyridazine**

Following the same experimental procedure as in Example 2, with starting materials of 7-ethyl-4-(4-fluoro-3-(4,4,5,5-tetramethyl-1,3,2-dioxaborolan-2-yl)phenyl)-7H-imidazo [4,5-c]pyridazine (54 mg, 0.15 mmol) and 5-bromo-6-methoxy-2-methylimidazo[1,2-a]pyridine (35 mg, 0.15 mmol), a brown solid (25 mg, yield: 41%) was obtained.

$^1$H NMR (400MHz, CHLOROFORM-d) 9.38 (s, 1 H), 8.43 (br. s., 2 H), 8.28 (s, 2 H), 7.50 (t, J = 9.0 Hz, 1 H), 7.36 - 7.28 (m, 1 H), 7.11 (br. s., 1 H), 4.61 - 4.56 (m, 2 H), 3.85 (br. s., 3 H), 2.46 - 2.32 (m, 3 H), 1.71 - 1.67 (m, 3 H). LC-MS: m/z [M+H]$^+$ = 403.

## Example 41

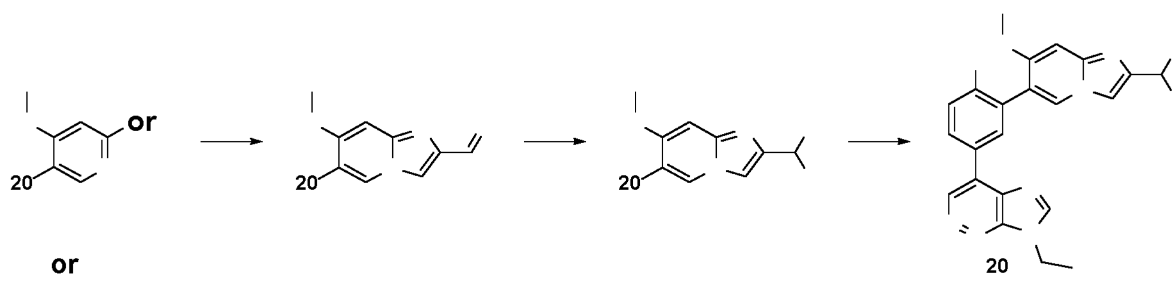

**41-1: 6-Bromo-7-methoxyimidazo[1,2-a]pyridine-2-carbaldehyde**

5-Bromo-4-methoxypyridin-2-amine (610 mg, 3.0 mmol) and 1,1,3-trichloroacetone (720 mg, 4.5 mmol) were added to 1,2-dimethoxyethane (2 mL), and the mixture was stirred at room temperature for 24 h. The reaction mixture was filtered under vacuum, and the resulting white solid was dissolved in ethanol (4 mL). The resulting solution was allowed to react at 95 °C for 24 h. The reaction mixture was concentrated and added to dichloromethane (5 mL) and trifluoroacetic acid (0.5 mL), and the mixture was stirred at room temperature for one hour. The reaction mixture was concentrated. The residue was subjected to preparative thin-layer chromatography (dichloromethane/methanol = 10/1) to obtain the title compound (60 mg, 7.8%). LC-MS: m/z $[M+H]^+$ = 255, 257.

**41-2: 6-Bromo-2-(difluoromethyl)-7-methoxyimidazo[1,2-a]pyridine**

6-Bromo-7-methoxyimidazo[1,2-a]pyridine-2-carbaldehyde (60 mg, 0.235 mmol) was added to dichloromethane (2 mL), and (diethylamino)sulfur trifluoride (0.05 mL) was added to the reaction mixture. The mixture was stirred at room temperature for 1 h. The reaction mixture was quenched with 0.5 mL and concentrated. The residue was subjected to preparative thin-layer chromatography (petroleum ether/ethyl acetate/dichloromethane = 3/1/0.1) to obtain the title compound (17 mg, 26%). LC-MS: m/z $[M+H]^+$ = 277, 279.

**41: 4-(3-(2-(Difluoromethyl)-7-methoxyimidazo[1,2-a]pyridin-6-yl)-4-fluorophenyl)-7-ethyl-7H-imidazo[4,5-c] pyridazine**

Following the same experimental procedure as in Example 19, with starting materials of 6-bromo-2-(difluoromethyl)-7-methoxyimidazo[12-a]pyridine (9 mg, 0.032 mmol) and 7-ethyl-4-(4-fluoro-3-(4,4,5,5-tetramethyl-1,3,2-dioxaborolan-2-yl)phenyl)-7H-imidazo[4,5-c]pyridazine (12 mg, 0.032 mmol), the title compound (7 mg, 50%) was obtained.

$^1$H NMR (400MHz, CHLOROFORM-d) d = 9.38 (s, 1 H), 8.29 (br. s., 3 H), 8.10 (s, 1 H), 7.69 (s, 1 H), 7.38 (s, 1 H), 7.05 - 6.67 (m, 2 H), 4.59 (q, J = 7.0 Hz, 2 H), 3.88 (s, 3 H), 1.70 (br. s., 3 H). LC-MS: m/z $[M+H]^+$ = 439.

**Example 42**

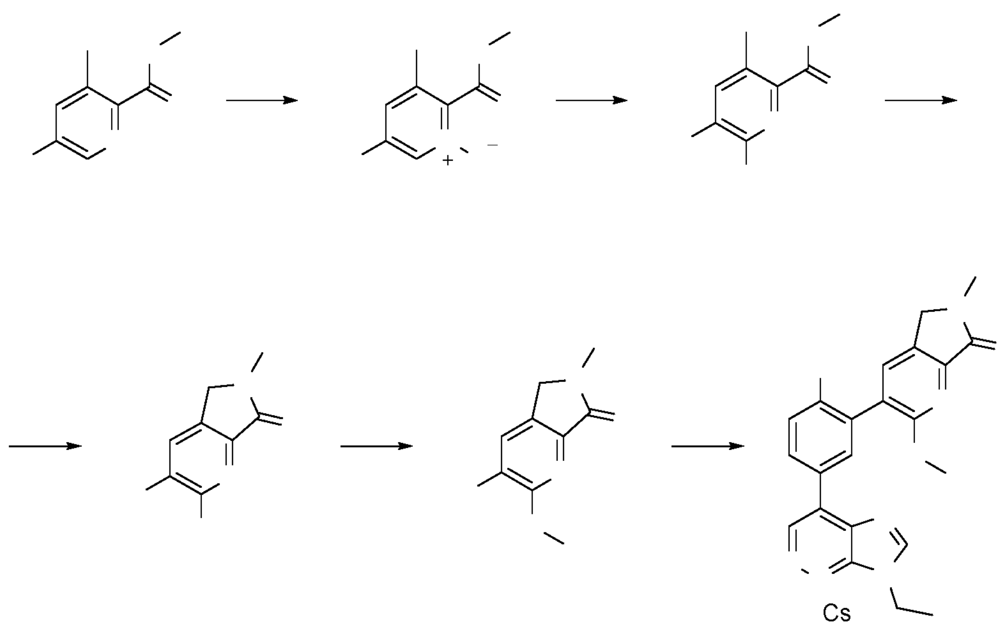

**42-1: 5-Bromo-2-(methoxycarbonyl)-3-methylpyridine 1-oxide**

Methyl 5-bromo-3-methylpicolinate (1.2 g, 5.22 mmol) and *m*-chloroperoxybenzoic acid (3 g, 17.44 mmol) were added to dichloromethane (100 mL), and the mixture was stirred at room temperature for 16 h and then concentrated. The residue was subjected to column chromatography (petroleum ether:ethyl acetate = 5:1) to obtain the title compound (1.06 g, yield: 83%) as a white solid. LC-MS: m/z $[M+H]^+$ = 246, 248.

**42-2: Methyl 5-bromo-6-chloro-3-methylpicolinate**

5-Bromo-2-(methoxycarbonyl)-3-methylpyridine 1-oxide (1.05 g, 4.30 mmol) and triethylamine (5 g, 49.5 mmol) were added to dichloromethane (100 mL). Oxalyl chloride (2.5 g, 20.5 mmol) was added dropwise in an ice bath, and the mixture was stirred at room temperature for 1 h and filtered. The mother liquor was concentrated. The residue was subjected to column chromatography (petroleum ether:ethyl acetate = 5:1) to obtain the title compound (0.52 g, yield: 44%) as a pale yellow solid. LC-MS: m/z $[M+H]^+$ = 264, 266.

**42-3: Methyl 5-bromo-3-(bromomethyl)-6-chloropicolinate**

Methyl 5-bromo-6-chloro-3-methylpicolinate (0.52 g, 1.90 mmol), NBS (0.48 g, 2.7 mmol), and AIBN (30 mg) were added to carbon tetrachloride (10 mL), and the mixture was stirred at 65 °C for 16 h and then concentrated. The residue was purified by a preparative plate (petroleum ether:ethyl acetate = 5:1) to obtain a mixture containing the title compound and the starting materials (0.5 g, 1: 1 crude product). LC-MS: m/z $[M+H]^+$ = 342, 344, 346.

**42-4: 3-Bromo-2-chloro-6-methyl-5,6-dihydro-7H-pyrrolo[3,4-b]pyridin-7-one**

Methyl 5-bromo-3-(bromomethyl)-6-chloropicolinate (0.5 g, crude product), methylamine hydrochloride (200 mg, 3 mmol), and triethylamine (0.5 g, 4.95 mmol) were added to acetonitrile (10 mL), and the mixture was stirred at room temperature for 2 h. A saturated ammonium chloride solution (100 mL) was then added, and the mixture was extracted with ethyl acetate (3 × 100 mL). The mother liquor was concentrated. The residue was purified by a preparative plate to obtain the title compound (200 mg) as a white solid. LC-MS: m/z $[M+H]^+$ = 261, 263.

**42-5: 3-Bromo-2-methoxy-6-methyl-5,6-dihydro-7H-pyrrolo[3,4-b]pyridin-7-one**

3-Bromo-2-chloro-6-methyl-5,6-dihydro-7H-pyrrolo[3,4-b]pyridin-7-one (120 mg, 0.46 mmol) and sodium phosphate (250 mg, 1.52 mmol) were added to methanol (5 mL), and the mixture was stirred at 60 °C for 16 h and filtered. The mother liquor was concentrated. The residue was purified by a preparative plate to obtain the title compound (17 mg, 14%) as a white solid. LC-MS: m/z $[M+H]^+$ = 257, 259.

**42: 3-(5-(7-Ethyl-7H-imidazo[4,5-c]pyridazin-4-yl)-2-fluorophenyl)-2-methoxy-6-methyl-5,6-dihydro-7H-pyrrolo[3,4-b]pyridin-7-one**

Following the same experimental procedure as in Example 2, with starting materials of 7-ethyl-4-(4-fluoro-3-(4,4,5,5-tetramethyl-1,3,2-dioxaborolan-2-yl)phenyl)-7H-imidazo [4,5-c]pyridazine (25 mg, 0.07 mmol) and 3-bromo-2-methoxy-6-methyl-5,6-dihydro-7H-pyrrolo[3,4-b]pyridin-7-one (15 mg, 0.06 mmol), a brown solid (12 mg, yield: 48%) was obtained.

$^1H$ NMR (400MHz ,CHLOROFORM-d) 9.37 (s, 1 H), 8.28 (s, 3 H), 7.78 (s, 1 H), 7.37 (t, J = 9.3 Hz, 1 H), 4.67 - 4.52 (m, 2 H), 4.38 (s, 2 H), 4.10 (s, 3 H), 3.28 (s, 3 H), 1.71 - 1.68 (m, 3 H). LC-MS: m/z $[M+H]^+$ = 419.

**Example 43**

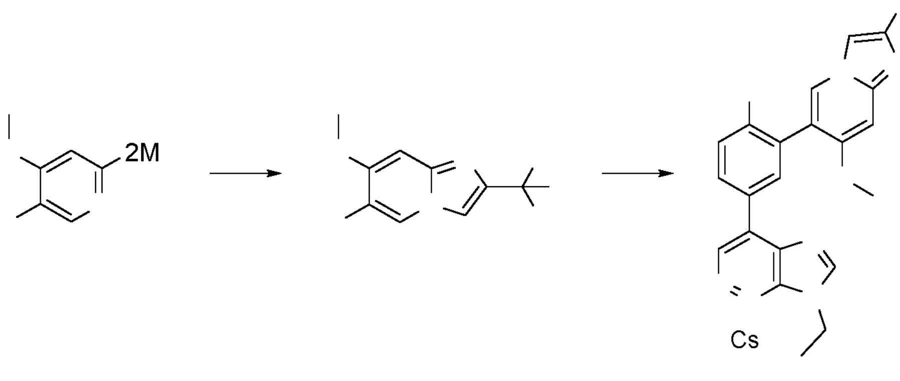

**43-1: 6-Bromo-7-methoxy-2-(trifluoromethyl)imidazo[1,2-a]pyridine**

5-Bromo-4-methoxypyridin-2-amine (202 mg, 1.0 mmol) was added to a mixed solvent of ethanol (1.5 mL) and 1,4-dioxane (0.5 mL). 3-Chloro-1,1,1-trifluoroacetone (161 mg, 1.1 mmol) was slowly added to the reaction mixture, and the mixture was stirred at room temperature for 6 h. Triethylamine (202 mg, 2.0 mmol) was then added to the reaction mixture, and the mixture was allowed to react at 95 °C overnight. The reaction mixture was filtered under vacuum and concentrated. The residue was subjected to preparative thin-layer chromatography (petroleum ether/ethyl acetate = 20/1) to obtain the title compound (50 mg, 16.9%). LC-MS: m/z $[M+H]^+$=295, 297.

**43: 7-Ethyl-4-(4-fluoro-3-(7-methoxy-2-(trifluoromethyl)imidazo[1,2-a]pyridin-6-yl)phenyl)-7H-imidazo [4,5-c] pyridazine**

Following the same experimental procedure as in Example 19, with starting materials of 6-bromo-7-methoxy-2-(trifluoromethyl)imidazo[1,2-a]pyridine (50 mg, 0.170 mmol) and 7-ethyl-4-(4-fluoro-3-(4,4,5,5-tetramethyl-1,3,2-dioxaborolan-2-yl)phenyl)-7H-imidazo[4,5-c]pyridazine (58 mg, 0.155 mmol), the title compound (32 mg, 45%) was obtained.

$^1$H NMR (400MHz, CHLOROFORM-d) 9.36 (br. s., 1 H), 8.27 (br. s., 3 H), 8.09 (s, 1 H), 7.76 (s, 1 H), 7.36 (t, J = 8.6 Hz, 1 H), 7.01 (s, 1 H), 4.57 (d, J = 7.3 Hz, 2 H), 3.86 (s, 3 H), 1.68 (br. s., 3 H). LC-MS: m/z $[M+H]^+$ = 457.

**Example 44**

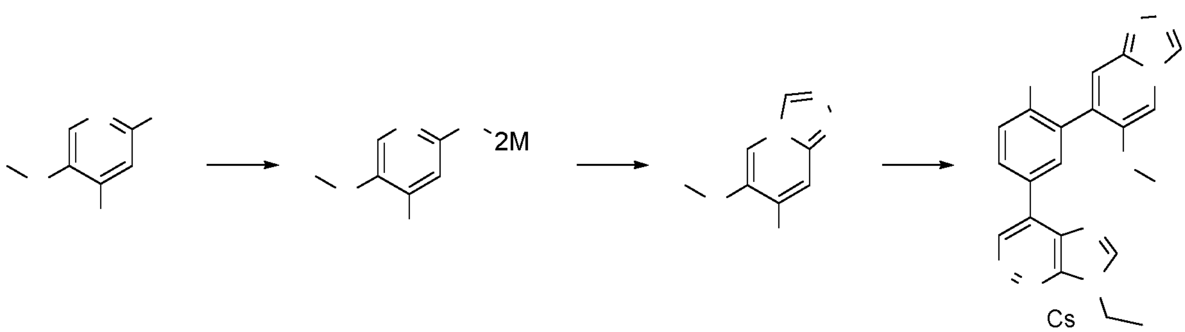


**44-1: 4-Bromo-2-hydrazineyl-5-methoxypyridine**

4-Bromo-2-fluoro-5-methoxypyridine (560 mg, 2.72 mmol) and hydrazine hydrate (1360 mg, 27.20 mmol) were added to ethanol (10 mL), and the mixture was stirred at 80 °C for 16 h. The reaction mixture was concentrated. The residue was separated by preparative thin-layer chromatography (ethyl acetate) to obtain the title compound (110 mg, 20%) as a yellow oily liquid. LC-MS: m/z $[M+H]^+$ = 218, 220.

**44-2: 7-Bromo-6-methoxy-[1,2,4]triazolo[4,3-a]pyridine**

4-Bromo-2-hydrazineyl-5-methoxypyridine (110 mg, 0.50 mmol) was added to formic acid (5 mL), and the mixture was stirred at 110 °C for 16 h. The reaction mixture was filtered and then concentrated. The residue was separated by preparative thin-layer chromatography (dichloromethane/methanol = 20/1) to obtain the title compound (30 mg, 26%) as a white solid. LC-MS: m/z $[M+H]^+$ =228, 230.

**44: 7-Ethyl-4-(4-fluoro-3-(6-methoxy-[1,2,4]triazolo[4,3-a]pyridin-7-yl)phenyl)-7H-imidazo [4,5-c] pyridazine**

Following the same experimental procedure as in Example 2, with starting materials of 7-bromo-6-methoxy-[1,2,4]triazolo[4,3-a]pyridine (30 mg, 0.13 mmol) and 7-ethyl-4-(4-fluoro-3-(4,4,5,5-tetramethyl-1,3,2-dioxaborolan-2-yl)phenyl)-7H-imidazo[4,5-c]pyridazine (48 mg, 0.13 mmol), the title compound (3 mg, 6%) was obtained as a yellow solid.

$^1$H NMR (400MHz, CHLOROFORM-d) 9.37 (s, 1 H), 8.83 (s, 1 H), 8.34 (d, J = 6.4 Hz, 1 H), 8.28 (s, 2 H), 7.82 (s, 1 H), 7.71 (s, 1 H), 7.37 (t, J = 9.0 Hz, 1 H), 4.58 (q, J = 7.3 Hz, 2 H), 3.86 (s, 3 H), 1.69 (t, J = 7.3 Hz, 3 H). LC-MS: m/z $[M+H]^+$ = 390.

**Example 45**

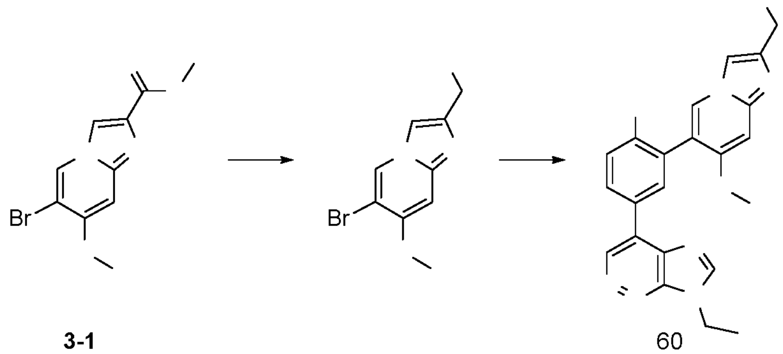

3-1 60

**45-1: (6-Bromo-7-methoxyimidazo[1,2-a]pyridin-2-yl)methanol**

Methyl 6-bromo-7-methoxyimidazo[1,2-a]pyridine-2-carboxylate (800 mg, 2.81 mmol) was added to dichloromethane (4 mL). A solution of diisobutylaluminium hydride in n-hexane (1 M, 4 mL) was then added, and the mixture was stirred at room temperature for 16 h. The reaction mixture was filtered and then concentrated. The residue was separated by preparative thin-layer chromatography (dichloromethane/methanol = 10/1) to obtain the title compound (190 mg, 26%) as a yellow solid. LC-MS: m/z $[M+H]^+$ = 257, 259.

**45: (6-(5-(7-Ethyl-7H-imidazo[4,5-c]pyridazin-4-yl)-2-fluorophenyl)-7-methoxyimidazo[1,2-a]pyridin-2-yl)methanol**

Following the same experimental procedure as in Example 2, with starting materials of (6-bromo-7-methoxyimidazo[1,2-a]pyridin-2-yl)methanol (50 mg, 0.19 mmol) and 7-ethyl-4-(4-fluoro-3-(4,4,5,5-tetramethyl-1,3,2-dioxaborolan-2-yl)phenyl)-7H-imidazo[4,5-c]pyridazine (70 mg, 0.19 mmol), the title compound (15 mg, 18%) was obtained as a brown solid.

$^1H$ NMR (400MHz, CHLOROFORM-d) 9.23 (s, 1 H), 8.32 (s, 1 H), 8.20 - 8.10 (m, 3 H), 7.45 (s, 1 H), 7.30 (t, J = 9.3 Hz, 1 H), 7.20 - 6.98 (m, 1 H), 4.67 (s, 2 H), 4.50 (q, J = 7.2 Hz, 2 H), 3.80 (s, 3 H), 1.60 (t, J = 7.3 Hz, 3 H). LC-MS: m/z $[M+H]^+$ = 419.

**Example 46**

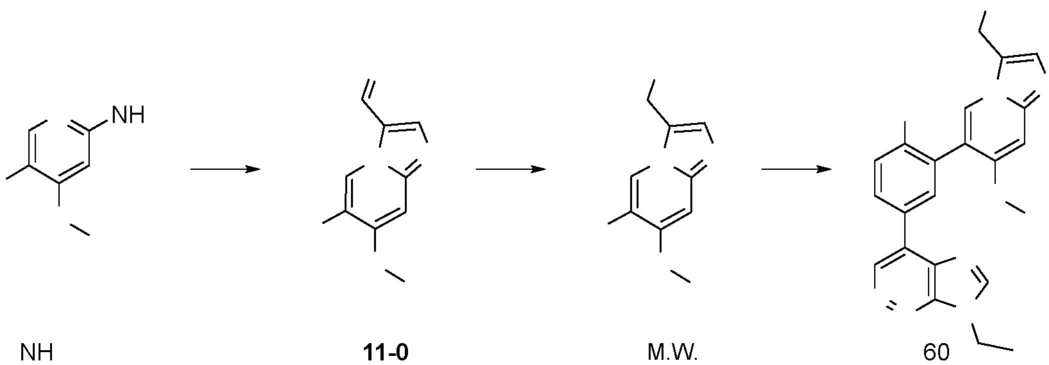

NH 11-0 M.W. 60

**46-1: 6-Bromo-7-methoxyimidazo[1,2-a]pyridine-3-carbaldehyde**

5-Bromo-4-methoxypyridin-2-amine (300 mg, 1.48 mmol) and 2-bromomalonaldehyde (335 mg, 2.22 mmol) were added to ethanol (10 mL), and the mixture was stirred at 80 °C for 0.5 h. Sodium bicarbonate (249 mg, 2.96 mmol) was then added, and the mixture was stirred at 80 °C for 16 h. The reaction mixture was filtered and then concentrated. The

residue was separated by preparative thin-layer chromatography (ethyl acetate) to obtain the title compound (120 mg, 25%) as a yellow solid. LC-MS: m/z $[M+H]^+$ = 255, 257.

**46-2: 6-Bromo-7-methoxyimidazo[1,2-a]pyridine-3-methanol**

6-Bromo-7-methoxyimidazo[1,2-a]pyridine-3-carbaldehyde (120 mg, 0.47 mmol) was added to dichloromethane (4 mL). A solution of diisobutylaluminium hydride in *n*-hexane (1 M, 4 mL) was then added, and the mixture was stirred at room temperature for 16 h. The reaction mixture was filtered and then concentrated. The residue was separated by preparative thin-layer chromatography (dichloromethane/methanol = 10/1) to obtain the title compound (40 mg, 33%) as a white solid. LC-MS: m/z $[M+H]^+$ = 257, 259.

**46: (6-(5-(7-Ethyl-7H-imidazo[4,5-c]pyridazin-4-yl)-2-fluorophenyl)-7-methoxyimidazo[1,2-a]pyridin-3-yl)methanol**

Following the same experimental procedure as in Example 2, with starting materials of 6-bromo-7-methoxyimidazo[1,2-a]pyridine-3-methanol (40 mg, 0.16 mmol) and 7-ethyl-4-(4-fluoro-3-(4,4,5,5-tetramethyl-1,3,2-dioxaborolan-2-yl)phenyl)-7H-imidazo[4,5-c]pyridazine (59 mg, 0.16 mmol), the title compound (6 mg, 8%) was obtained as a brown solid.

$^1$H NMR (400MHz, DMSO-d6) 9.57 - 9.54 (m, 1 H), 8.87 - 8.84 (m, 1 H), 8.59 - 8.53 (m, 1 H), 8.53 - 8.47 (m, 2 H), 7.58 - 7.53 (m, 1 H), 7.52 - 7.49 (m, 1 H), 7.26 - 7.15 (m, 1 H), 5.33 - 5.26 (m, 1 H), 4.79 - 4.75 (m, 2 H), 4.53 - 4.47 (m, 2 H), 3.86 (s, 3 H), 1.54 (s, 3 H). LC-MS: m/z $[M+H]^+$ = 419.

**Example 47**

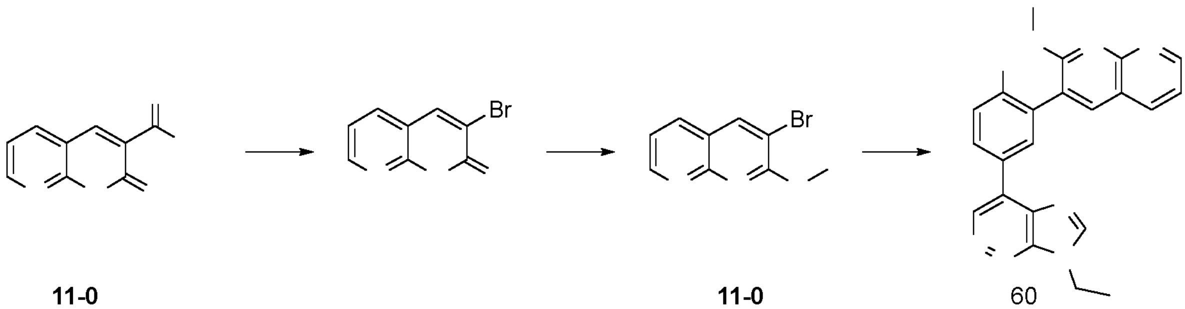

**11-0** **11-0** 60

**47-1: 3-Bromo-1,8-naphthyridin-2(1H)-one**

2-Oxo-1,2-dihydro-1,8-naphthyridine-3-carboxylic acid (490 mg, 2.58 mmol) was added to N,N-dimethylformamide (10 mL). Liquid bromine (2 g, 12.9 mmol) and pyridine (5 mL) were added, and the mixture was stirred at 100 °C for 1 h. A saturated aqueous sodium bisulfite solution (5 mL) was added dropwise to the reaction mixture. The mixture was then extracted with ethyl acetate (3 × 200 mL), and the ethyl acetate was washed three times with water and once with saturated brine, then dried over anhydrous sodium sulfate, and concentrated to obtain a crude product. The crude product was purified by thin-layer chromatography (petroleum ether/ethyl acetate = 4/1) to obtain the title compound (140 mg, 21%). LC-MS: m/z $[M+H]^+$ = 225.

**47-2: 3-Bromo-2-methoxy-1,8-naphthyridine**

3-Bromo-1,8-naphthyridin-2(1H)-one (140 mg, 0.63 mmol) was added to dichloromethane/methanol (5 mL/2 mL). (Trimethylsilyl)diazomethane (2 mL) was then added, and the mixture was stirred at room temperature overnight. The reaction mixture was concentrated directly. The residue was then purified by thin-layer chromatography (petroleum ether/ethyl acetate = 4/1) to obtain the title compound (40 mg, 27%). LC-MS: m/z $[M+H]^+$ = 239.

**47: 3-(5-(7-Ethyl-7H-imidazo[4,5-c]pyridazin-4-yl)-2-fluorophenyl)-2-methoxy-1,8-naphthyridine**

Following the same experimental procedure as in Example 19, with starting materials of 7-ethyl-4-(4-fluoro-3-(4,4,5,5-tetramethyl-1,3,2-dioxaborolan-2-yl)phenyl)-7H-imidazo [4,5-c]pyridazine (74 mg, 0.2 mmol) and 3-bromo-2-methoxy-1,8-naphthyridine (40 mg, 0.17 mmol), the title compound (38 mg, 48%) was obtained.

$^1H$ NMR (400 MHz, CHLOROFORM-d) 9.39 (s, 1 H) 8.66 (d, J=3.42 Hz, 1 H) 8.43 (d, J=4.89 Hz, 1 H) 8.29 (br. s., 1 H) 8.27 (s, 1 H) 7.95 (d, J=6.36 Hz, 1 H) 7.90 (s, 1 H) 7.39 (t, J=9.05 Hz, 1H) 7.23 (d, J=7.83 Hz, 1 H) 4.58 (q, J=7.34 Hz, 2 H) 3.94 (s, 3 H) 1.69 (t, J=7.34 Hz, 3 H). LC-MS: m/z $[M+H]^+$ = 401.

**Example 48**

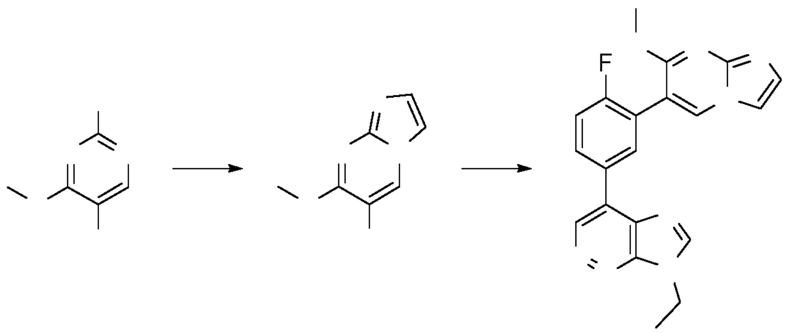

**48-1: 6-Bromo-7-methoxyimidazo[1,2-a]pyrimidine**

5-Bromo-4-methoxypyrimidin-2-amine (203 mg, 1.0 mmol) and chloroacetaldehyde (94 mg, 1.2 mmol) were added to ethanol (2 mL), and the mixture was stirred at room temperature overnight. Sodium bicarbonate (168 mg, 2.0 mmol) was added to the reaction mixture, and the mixture was allowed to react at 60 °C for 2 h. The reaction mixture was filtered under vacuum and concentrated. The residue was subjected to preparative thin-layer chromatography (dichloromethane/methanol = 20/1) to obtain the title compound (57 mg, 25%). LC-MS: m/z $[M+H]^+$ = 228, 230.

**48: 7-Ethyl-4-(4-fluoro-3-(7-methoxyimidazo[1,2-a]pyrimidin-6-yl)phenyl)-7H-imidazo[4,5-c]pyridazine**

Following the same experimental procedure as in Example 19, with starting materials of 6-bromo-7-methoxyimidazo[1,2-a]pyrimidine (57 mg, 0.251 mmol) and 7-ethyl-4-(4-fluoro-3-(4,4,5,5-tetramethyl-1,3,2-dioxaborolan-2-yl)phenyl)-7H-imidazo[4,5-c]pyridazine (92 mg, 0.251 mmol), the title compound (50 mg, 52%) was obtained.

$^1H$ NMR (400MHz, CHLOROFORM-d) 9.37 (s, 1 H), 8.31 (d, J = 16.6 Hz, 4 H), 7.57 (br. s., 1 H), 7.44 - 7.34 (m, 2 H), 4.59 (q, J = 7.3 Hz, 2 H), 4.08 (s, 3 H), 1.72 - 1.68 (m, 3 H). LC-MS: m/z $[M+H]^+$ = 390.

**Example 49**

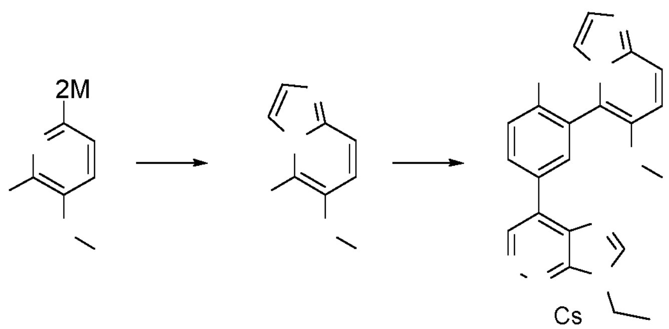

**49-1: 5-Bromo-6-methoxyimidazo[1,2-a]pyridine**

6-Bromo-5-methoxypyridin-2-amine (200 mg, 0.99 mmol) and chloroacetaldehyde (40%, 290 mg, 1.49 mmol) were added to anhydrous methanol (10 mL), and the mixture was stirred at room temperature for 1 h. Sodium bicarbonate (200 mg, 2.38 mmol) was then added, and the mixture was allowed to react at 60 °C for 16 h and then concentrated. The

residue was purified by a preparative plate (petroleum ether:ethyl acetate = 3:1) to obtain a pale yellow solid (130 mg, yield: 58%). LC-MS: m/z $[M+H]^+$ = 227, 229.

**49: 7-Ethyl-4-(4-fluoro-3-(6-methoxyimidazo[1,2-a]pyridin-5-yl)phenyl)-7H-imidazo[4,5-c]pyridazine**

Following the same experimental procedure as in Example 2, with starting materials of 7-ethyl-4-(4-fluoro-3-(4,4,5,5-tetramethyl-1,3,2-dioxaborolan-2-yl)phenyl)-7H-imidazo [4,5-c]pyridazine (36 mg, 0.1 mmol) and 5-bromo-6-methoxyimidazo[1,2-a]pyridine (23 mg, 0.1 mmol), a brown solid (7 mg, yield: 18%) was obtained.

$^1$H NMR (400MHz, CHLOROFORM-d) 9.38 (s, 1 H), 8.47 - 8.39 (m, 2 H), 8.28 (s, 1 H), 7.84 - 7.59 (m, 1 H), 7.50 (t, J = 8.8 Hz, 1 H), 7.41 - 7.26 (m, 2 H), 7.25 - 6.97 (m, 1 H), 4.58 (q, J = 7.3 Hz, 2 H), 3.85 (s, 3 H), 1.70 - 1.65 (m, 3 H). LC-MS: m/z $[M+H]^+$ = 389.

**Example 50**

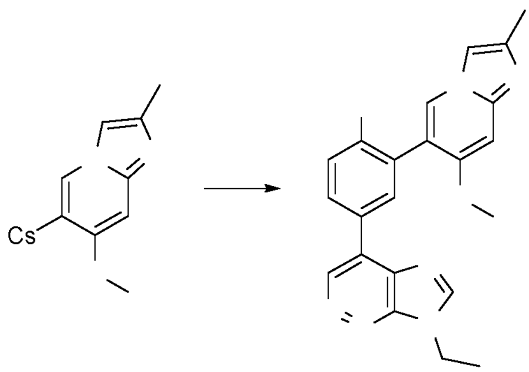

**50: 7-Ethyl-4-(4-fluoro-3-(7-methoxy-2-methylimidazo[1,2-a]pyridin-6-yl)phenyl)-7H-imidazo[4,5-c]pyridazine**

Following the same experimental procedure as in Example 2, with starting materials of 6-bromo-7-methoxy-2-methylimidazo[1,2-a]pyridine (30 mg, 0.12 mmol) and 7-ethyl-4-(4-fluoro-3-(4,4,5,5-tetramethyl-1,3,2-dioxaborolan-2-yl) phenyl)-7H-imidazo[4,5-c]pyridazine (44 mg, 0.12 mmol), the title compound (13 mg, 27%) was obtained as a white solid.

$^1$H NMR (400MHz, CHLOROFORM-d) 9.35 (s, 1 H), 8.32 - 8.16 (m, 3 H), 7.98 (s, 1 H), 7.34 (t, J = 8.8 Hz, 1 H), 7.20 (s, 1 H), 6.90 (br. s., 1 H), 4.57 (q, J = 7.3 Hz, 2 H), 3.93 - 3.74 (m, 3 H), 2.47 - 2.33 (m, 3 H), 1.68 (t, J = 7.1 Hz, 3 H). LC-MS: m/z $[M+H]^+$ = 403.

**Example 51**

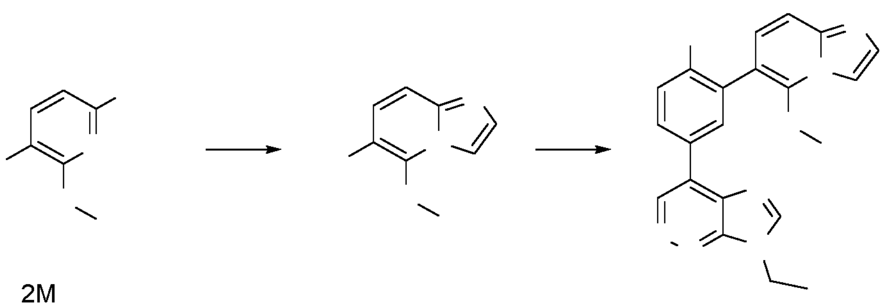

2M

**51-1: 6-Bromo-5-methoxyimidazo[1,2-a]pyridine**

5-Bromo-6-methoxypyridin-2-amine (200 mg, 0.99 mmol) and chloroacetaldehyde (40%, 290 mg, 1.49 mmol) were added to anhydrous methanol (10 mL), and the mixture was stirred at room temperature for 1 h. Sodium bicarbonate (200 mg, 2.38 mmol) was then added, and the mixture was allowed to react at 70 °C for 16 h and then concentrated. The

residue was purified by a preparative plate (petroleum ether:ethyl acetate = 3:1) to obtain a pale yellow solid (70 mg, yield: 31%). LC-MS: m/z $[M+H]^+$ = 227, 229.

**51: 7-Ethyl-4-(4-fluoro-3-(5-methoxyimidazo[1,2-a]pyridin-6-yl)phenyl)-7H-imidazo[4,5-c]pyridazine**

Following the same experimental procedure as in Example 2, with starting materials of 7-ethyl-4-(4-fluoro-3-(4,4,5,5-tetramethyl-1,3,2-dioxaborolan-2-yl)phenyl)-7H-imidazo [4,5-c]pyridazine (36 mg, 0.1 mmol) and 6-bromo-5-methoxyimidazo[1,2-a]pyridine (23 mg, 0.1 mmol), a brown solid (4 mg, yield: 10%) was obtained.

$^1H$ NMR (400MHz, CHLOROFORM-d) 9.39 (s, 1 H), 8.41 (d, J = 4.9 Hz, 1 H), 8.29 (br. s., 2 H), 7.76 (br. s., 2 H), 7.49 - 7.27 (m, 3 H), 4.68 - 4.50 (m, 2 H), 3.84 (s, 3 H), 1.69 (br. s., 3 H). LC-MS: m/z $[M+H]^+$ = 389.

**Example 52**

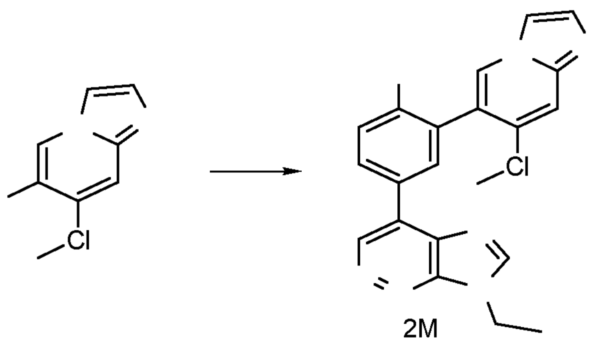


**52: 7-Ethyl-4-(4-fluoro-3-(7-methoxyimidazo[1,2-a]pyridin-6-yl)phenyl)-7H-imidazo[4,5-c]pyridazine**

Following the same experimental procedure as in Example 2, with starting materials of 7-ethyl-4-(4-fluoro-3-(4,4,5,5-tetramethyl-1,3,2-dioxaborolan-2-yl)phenyl)-7H-imidazo [4,5-c]pyridazine (50 mg, 0.14 mmol) and 6-bromo-7-methoxyimidazo[1,2-a]pyridine (30 mg, 0.14 mmol), the title compound (8 mg, 15%) was obtained as a yellow solid.

$^1H$ NMR (400 MHz, DMSO-d6) δ 9.57 (s, 1 H) 8.87 (s, 1 H) 8.67 (s, 1 H) 8.48 - 8.61 (m, 2 H) 7.82 (br. s., 1 H) 7.46 - 7.65 (m, 2 H) 7.17 (br. s., 1 H)4.51 (q, J=6.85 Hz, 2 H) 3.84 (s, 3 H) 1.55 (t, J=7.09 Hz, 3 H). LC-MS: m/z $[M+H]^+$= 389.

**Example 53**

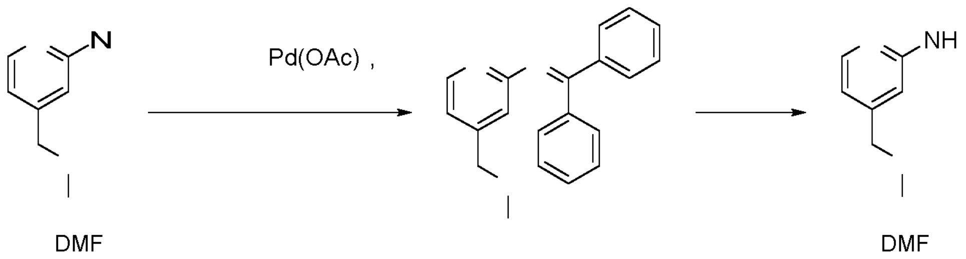

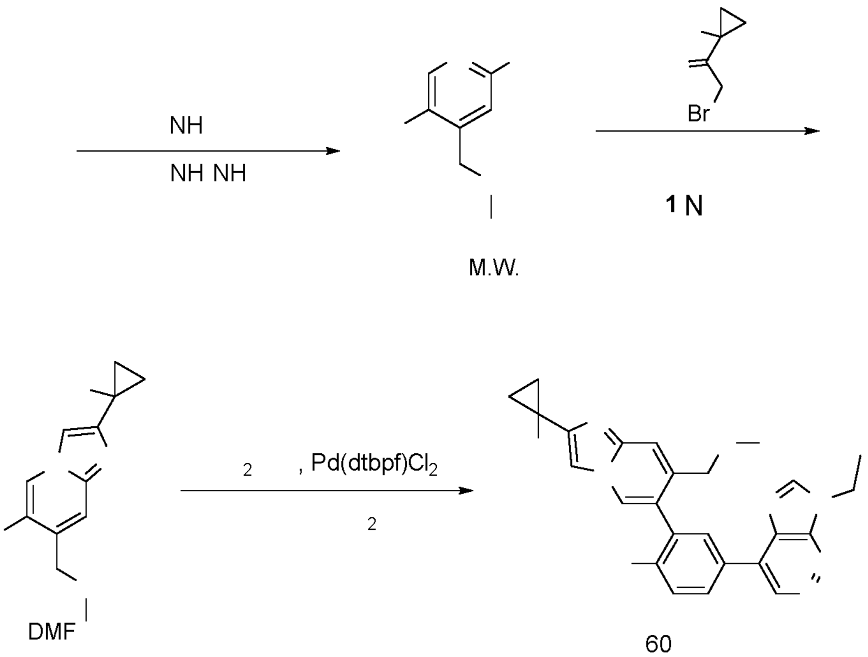


60

**53-1: N-(4-(Methoxymethyl)pyridin-2-yl)-1,1-diphenylmethanimine**

2-Chloro-4-(methoxymethyl)pyridine (2.87 g, 18.28 mmol), benzhydrylamine (3.3 g, 18.28 mmol), (R)-(+)-2,2'-bis(diphenylphosphino)-1,1'-binaphthyl (1.10 g, 1.82 mmol), and cesium carbonate (8.30 g, 25.59 mmol) were added to a 1,4-dioxane solution (50 mL), and the mixture was purged three times with argon, then heated to 80 °C and allowed to react for 15 h. The reaction mixture was cooled to room temperature, poured into ice water (40 mL), extracted with ethyl acetate (50 mL × 3), and washed with a saturated aqueous sodium chloride solution (50 mL × 3). The organic phase was dried and concentrated. The residue was purified by prep-HPLC to obtain a yellow oily liquid (3.4 g, 61.8%). LC-MS: m/z $[M+1]^+$: 303.

**53-2: 4-(Methoxymethyl)pyridin-2-amine**

N-(4-(Methoxymethyl)pyridin-2-yl)-1,1-diphenylmethanimine (3.3 g, 11 mmol) was dissolved in a tetrahydrofuran solution (50 mL). A solution of 1,4-dioxane in hydrochloric acid (17 mL) was then added, and the mixture was allowed to react at room temperature for 2 h. After the reaction was completed, the reaction mixture was adjusted to pH = about 7 with saturated sodium bicarbonate (30 mL), extracted with ethyl acetate (50 mL × 3), and washed with a saturated aqueous sodium chloride solution (50 mL × 3). The organic phase was dried and concentrated. The residue was purified by prep-HPLC to obtain a white solid (800 mg, 53.3%). LC-MS: m/z $[M+1]^+$: 139.

**53-3: 5-Bromo-4-(methoxymethyl)pyridin-2-amine**

5-Bromo-4-(methoxymethyl)pyridin-2-amine (260 mg, 1.88 mmol) and ammonium acetate (14.5 mg, 0.188 mmol) were added to an acetonitrile solution (25 mL). NBS (167.7 mg, 0.94 mmol) was added in batches at a temperature of 0 °C. The ice-water bath was removed, and the mixture was allowed to react at room temperature for 40 min. Dichloromethane (20 mL) was added to dissolve, and the resulting solution was adjusted to pH = about 7 with saturated sodium bicarbonate (40 mL) and extracted with dichloromethane (50 mL × 4). The organic phase was dried and concentrated. The residue was purified by prep-HPLC to obtain a white solid (220 mg, 40%). LC-MS: m/z $[M+1]^+$: 219.

**53-4: 6-Bromo-2-(1-fluorocyclopropyl)-7-(methoxymethyl)imidazo[1,2-a]pyridine**

5-Bromo-4-(methoxymethyl)pyridin-2-amine (524 mg, 2.5 mmol), 2-chloro-1-(1-fluorocyclopropyl)ethan-1-one (670 mg, 5 mmol), and cesium carbonate (1.6 g, 5 mmol) were dissolved in an ethanol solution (10.0 mL). The resulting solution was heated to 100 °C in a microwave reactor and allowed to react for 1 h. The reaction mixture was purified by prep-HPLC to obtain a white solid (100 mg, 26%). LC-MS: m/z $[M+1]^+$ = 300.

**53: 7-Ethyl-4-(4-fluoro-3-(2-(1-fluorocyclopropyl)-7-(methoxymethyl)imidazo [1,2-a] pyridin-6-yl)phenyl)-7H-imidazo [4,5-c] pyridazine**

With starting materials of 6-bromo-2-(1-fluorocyclopropyl)-7-(methoxymethyl) imidazo[1,2-a]pyridine (50.0 mg, 0.17 mmol) and (5-(7-ethyl-7H-imidazo[4,5-c]pyridazin-4-yl)-2-fluorophenyl)boronic acid (105 mg, 0.36 mmol), the title compound (20 mg, 26%) was obtained as an off-white solid.

$^1$H NMR (400MHz, CHLOROFORM-d) d = 9.39 (s, 1 H), 8.30 (d, J = 10.8 Hz, 3 H), 8.13 (br. s., 1 H), 7.81 (br. s., 1 H), 7.70 (s, 1 H), 7.40 (t, J = 8.6 Hz, 1 H), 4.59 (q, J = 7.0 Hz, 2 H), 4.38 (s, 2 H), 3.31 (s, 3 H), 1.70 (t, J = 7.1 Hz, 3H), 1.54 (d, J = 19.6 Hz, 2 H), 1.26 (d, J = 14.5 Hz, 2 H). LC-MS: m/z $[M+1]^+$ = 461.

**Example 54**

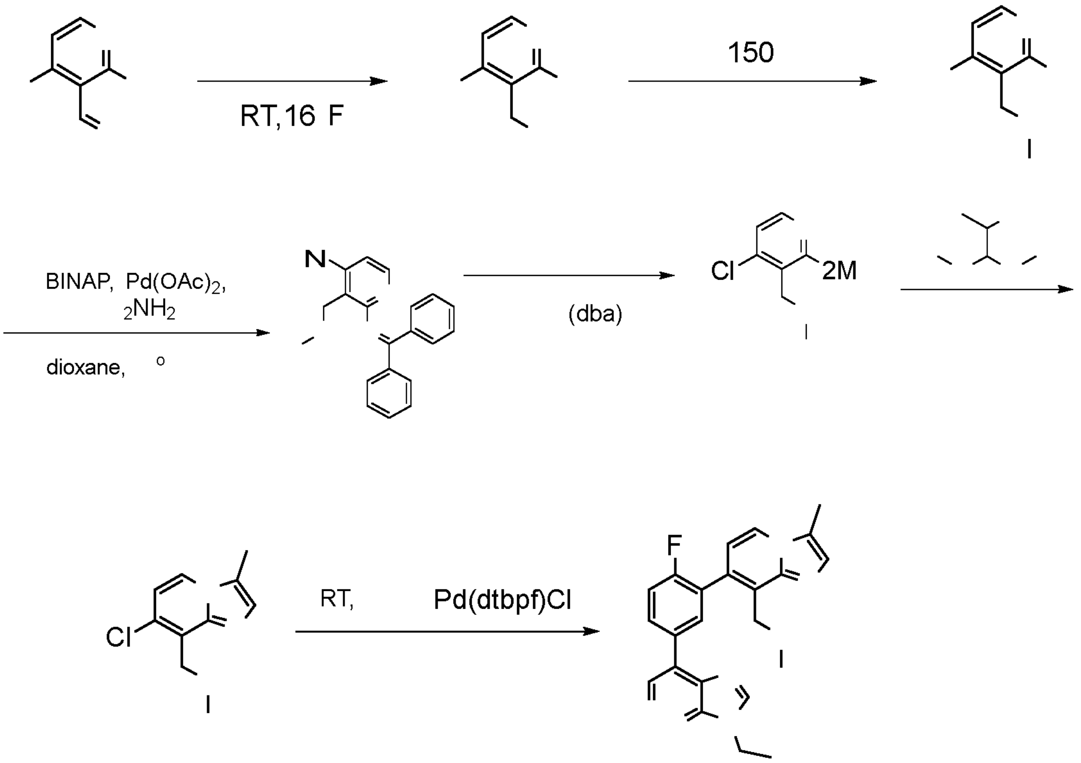


**54-1: (2,4-Dichloropyridin-3-yl)methanol**

2,4-Dichloronicotinaldehyde (5 g, 28.6 mmol) was dissolved in 50 mL of ethanol under a nitrogen atmosphere. The solution was cooled to 0 °C, and sodium borohydride (1.4 g, 37.2 mmol) was slowly added at this temperature. The mixture was warmed to room temperature and allowed to react for 16 h, and then added to an ice aqueous ammonium chloride solution. The mixture was extracted with dichloromethane (100 mL × 3). The organic phase was concentrated to obtain (2,4-dichloropyridin-3-yl)methanol (4.8 g, 94.1%) as a yellow solid. LC-MS: m/z $[M+H]^+$ = 178.

**54-2: 2,4-Dichloro-3-(methoxymethyl)pyridine**

(2,4-Dichloropyridin-3-yl)methanol (4.8 g, 27 mmol) was dissolved in 50 mL of N,N-dimethylformamide under a nitrogen atmosphere. The solution was cooled to 0 °C, and NaH (2.2 g, 54 mmol) was slowly added at this temperature. The mixture was allowed to react in an ice bath for half an hour. Iodomethane (4.6 g, 32.4 mmol) was then added, and the mixture was allowed to react at room temperature for 16 h. After the reaction was completed, the reaction mixture was added to 100 mL of ice water, extracted with ethyl acetate (100 mL × 3), and back-washed with water (50 mL × 2). The organic phase was concentrated to dryness by rotary evaporation to obtain 2,4-dichloro-3-(methoxymethyl)pyridine (2.3 g, 44.5%) as a pale yellow liquid. 1H NMR (400 MHz, CDCl3) 8.16 (d, J = 5.1 Hz, 1H), 7.23 (d, J = 5.2 Hz, 1H), 4.64 (s, 2H), 3.38 (s, 3H). LC-MS: m/z $[M+H]^+$ = 192.

**54-3: N-(4-Chloro-3-(methoxymethyl)pyridin-2-yl)-1,1-diphenylmethanimine**

2,4-Dichloro-3-(methoxymethyl)pyridine (1 g, 5.8 mmol) and benzhydrylamine (1.1 g, 5.8 mmol) were dissolved in 15 mL under a nitrogen atmosphere. 2,2'-Bis(diphenylphosphino)-1,1'-binaphthyl (361 mg, 0.58 mmol) and cesium carbonate (2.83 g, 8.7 mmol) were then added. Finally, palladium acetate (130 mg. 0.58 mmol) was added, and the mixture was allowed to react at 90 °C for 16 h. After the reaction was completed, the reaction mixture was added to 60 mL of water and extracted with ethyl acetate (50 mL × 3). The organic phase was concentrated to dryness by rotary evaporation. The residue was subjected to column chromatography (petroleum ether/ethyl acetate = 10/1). The organic phase was concentrated to dryness by rotary evaporation to obtain N-(4-chloro-3-(methoxymethyl)pyridin-2-yl)-1,1-diphenylmethanimine (700 mg, 40%) as a yellow oily liquid. LC-MS: m/z $[M+H]^+$ = 337.

**54-4: 4-Chloro-3-(methoxymethyl)pyridin-2-amine**

N-(4-Chloro-3-(methoxymethyl)pyridin-2-yl)-1,1-diphenylmethanimine (0.7 g, 2.1 mmol) was added to 10 mL of tetrahydrofuran under a nitrogen atmosphere, and then 8 mL of a hydrochloric acid-dioxane solution was added. The mixture was allowed to react at room temperature for 16 h. After the reaction was completed, the reaction mixture was added to 20 mL of a sodium bicarbonate solution and extracted with ethyl acetate (30 mL × 3). The organic phase was concentrated to dryness by rotary evaporation. The residue was subjected to column chromatography (petroleum ether/ethyl acetate = 2/1) to obtain 4-chloro-3-(methoxymethyl)pyridin-2-amine (220 mg, 20%) as a white solid.

$^1$HNMR (400 MHz, CDCl3) 7.85 (d, J = 5.7 Hz, 1H), 6.71 (d, J = 5.8 Hz, 1H), 5.72 (s, 2H), 4.69 (s, 2H), 3.37 (s, 3H). LC-MS: m/z $[M+H]^+$ = 173.

**54-5: 7-Chloro-8-(methoxymethyl)-3-methylimidazo[1,2-a]pyridine**

4-Chloro-3-(methoxymethyl)pyridin-2-amine (50 mg, 0.29 mmol) and 1,1-dimethoxy-2-bromopropane (265 mg, 1.45 mmol) were dissolved in 2 mL of ethanol under a nitrogen atmosphere, and then 0.2 mL of a hydrochloric acid-methanol solution was added. The mixture was allowed to react under microwave irradiation at 120 °C for 2 h. The mixture was then directly concentrated to dryness by rotary evaporation for use in the next step to obtain 7-chloro-8-(methoxymethyl)-3-methylimidazo[1,2-a]pyridine (70 mg, 100%) as a white solid. LC-MS: m/z $[M+H]^+$ = 211.

**54: 7-Ethyl-4-(4-fluoro-3-(8-(methoxymethyl)-3-methylimidazo[1,2-a]pyridin-7-yl)phenyl)-7H-imidazo[4,5-c]pyridazine**

Following the same experimental procedure as in Example 2, with starting materials of 7-chloro-8-(methoxymethyl)-3-methylimidazo[1,2-a]pyridine (70 mg, 0.33 mmol) and (5-(7-ethyl-7H-imidazo[4,5-c]pyridazin-4-yl)-2-fluorophenyl)boronic acid (95 mg, 0.33 mmol), the title compound (16.1 mg, 11.6%) was obtained as a white solid.

$^1$HNMR (400 MHz, DMSO-d6) 9.54 (s, 1H), 8.87 (s, 1H), 8.61-8.53 (m, 2H), 8.35 (d, J = 7.1 Hz, 1H), 7.59 (t, J = 9.3 Hz, 1H), 7.46 (d, J = 0.7 Hz, 1H), 7.00 (d, J = 7.0 Hz, 1H), 4.72 (s, 2H), 4.52 (q, J = 7.3 Hz, 2H), 3.11 (d, J = 3.8 Hz, 3H), 2.53 (s, 3H), 1.64-1.47 (m, 3H). LC-MS: m/z $[M+H]^+$ = 417.

**Example 55 and Example 56**

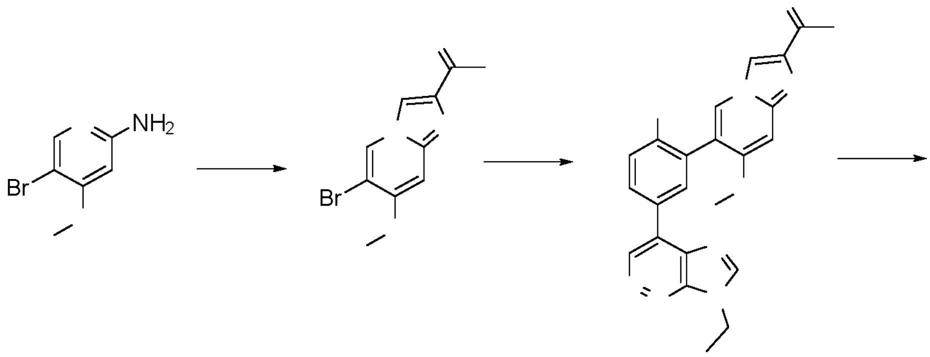

3-1 11-0

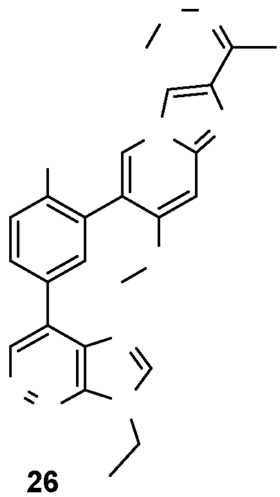

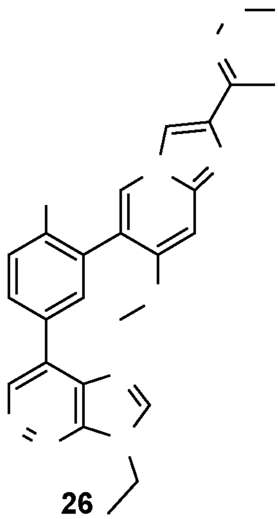

26 26

**55-1: 1-(6-Bromo-7-methoxyimidazo[1,2-a]pyridin-2-yl)ethan-1-one**

5-Bromo-4-methoxypyridin-2-amine (500 mg, 2.46 mmol) and 1-bromobutane-2,3-dione (406 mg, 2.46 mmol) were added to ethanol (10 mL), and the mixture was stirred at 90 °C overnight. The reaction mixture was directly subjected to thin-layer chromatography (petroleum ether/ethyl acetate = 1/1) to obtain the title compound (150 mg, 22.66%) as a yellow solid. LC-MS: m/z $[M+H]^+$ = 269.

**55-2: 1-(6-(5-(7-Ethyl-7H-imidazo[4,5-c]pyridazin-4-yl)-2-fluorophenyl)-7-methoxyimidazo[1,2-a]pyridin-2-yl) ethan-1-one**

Following the same experimental procedure as in Example 2, with starting materials of 1-(6-bromo-7-methoxyimidazo[1,2-a]pyridin-2-yl)ethan-1-1 (150 mg, 0.56 mmol) and (5-(7-ethyl-7H-imidazo[4,5-c]pyridazin-4-yl)-2-fluorophenyl)boronic acid (161 mg, 0.56 mmol), the title compound (100 mg, 41.29%) was obtained as a yellow solid. LC-MS: m/z $[M+H]^+$ = 431.

**55: (Z)-1-(6-(5-(7-Ethyl-7H-imidazo[4,5-c]pyridazin-4-yl)-2-fluorophenyl)-7-methoxyimidazo[1,2-a]pyridin-2-yl) ethan-1-one O-methyl oxime**

**56: (E)-1-(6-(5-(7-Ethyl-7H-imidazo[4,5-c]pyridazin-4-yl)-2-fluorophenyl)-7-methoxyimidazo[1,2-a]pyridin-2-yl) ethan-1-one O-methyl oxime**

1-(6-(5-(7-Ethyl-7H-imidazo[4,5-c]pyridazin-4-yl)-2-fluorophenyl)-7-methoxyimidazo[1,2-a]pyridin-2-yl) ethan-1-one (100 mg, 0.23 mmol) and methoxylamine hydrochloride (58 mg, 0.69 mmol) were added to ethanol (10 mL). The mixture was stirred at room temperature for 1 h. The reaction mixture was purified directly by thin-layer chromatography (dichloromethane/methanol = 20/1) to obtain compounds of Example 55 (6 mg, 5%) and Example 56 (15 mg, 13%) as yellow solids.

Example 55: $^1H$ NMR (400 MHz, DMSO-d6) ppm 1.50 - 1.57 (m, 3 H) 2.26 - 2.32 (m, 3 H) 3.80 - 3.85 (m, 3 H) 3.89 -

3.97 (m, 3 H) 4.47 - 4.54 (m, 2 H) 7.12 - 7.17 (m,1H) 7.52 - 7.58 (m, 1 H) 8.44 - 8.59 (m, 3 H) 8.65 - 8.71 (m, 1 H) 8.83 - 8.89 (m, 1 H) 9.52 - 9.60 (m, 1 H). LC-MS: m/z $[M+H]^+$ = 460.

Example 56: $^1H$ NMR (400 MHz, DMSO-d6) ppm 1.54 (t, J=7.34 Hz, 3H) 2.21 - 2.29 (m, 3H) 3.83 (s, 3H) 3.89 (s, 3H) 4.49 - 4.53 (m, 2H) 7.11 (s, 1H) 7.54 (t, J=8.80 Hz, 1H) 8.02 (s, 1H) 8.48 - 8.60 (m, 3H) 8.86 (s, 1H) 9.56 (s, 1H). LC-MS: m/z $[M+H]^+$ = 460.

**Example 57**

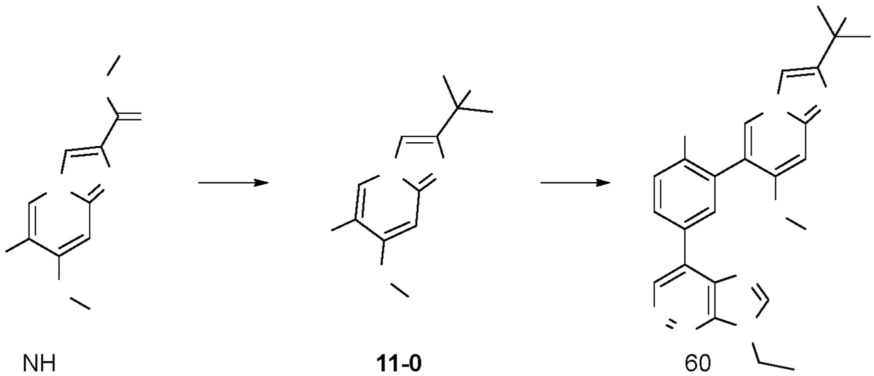

NH **11-0** 60

**57-1: 2-(6-Bromo-7-methoxy[1,2-a]pyridin-2-yl)propan-2-ol**

Methyl magnesium chloride (620 mg, 8.35 mmol) and methyl 6-bromo-7-methoxyimidazo[1,2-a]pyridine-2-carboxylate (500 mg, 1.67 mmol) were added to tetrahydrofuran (20 mL), and the mixture was stirred at room temperature for 1 h. The reaction mixture was adjusted to acidity with an aqueous hydrochloric acid solution (1 N), concentrated, then adjusted to alkalinity with a saturated aqueous sodium bicarbonate solution, and extracted with dichloromethane (50 mL × 3). The organic phase was washed with saturated brine (50 mL), dried over sodium sulfate, and concentrated. The residue was separated by preparative thin-layer chromatography (dichloromethane/methanol = 20/1) to obtain the title compound (130 mg, 27%). LC-MS: m/z $[M+H]^+$ = 285, 287.

**57: 2-(6-(5-(7-Ethyl-7H-imidazo[4,5-c]pyridazin-4-yl)-2-fluorophenyl)-7-methoxyimidazo[1,2-a]pyridin-2-yl)propan-2-ol**

Following the same experimental procedure as in Example 2, with starting materials of 2-(6-bromo-7-methoxy[1,2-a]pyridin-2-yl)propan-2-ol (120 mg, 0.42 mmol) and (5-(7-ethyl-7H-imidazo[4,5-c]pyridazin-4-yl)-2-fluorophenyl)boronic acid (120 mg, 0.42 mmol), the title compound (25 mg, 13%) was obtained.

$^1H$ NMR (400MHz, CHLOROFORM-d) 9.37 (s, 1 H), 8.31 - 8.23 (m, 3 H), 8.03 (s, 1 H), 7.40 - 7.33 (m, 2 H), 6.97 (s, 1 H), 4.59 (q, J = 7.3 Hz, 2 H), 3.86 (s, 3 H), 1.71 - 1.67 (m, 9 H). LC-MS: m/z $[M+H]^+$ = 447.

**Example 58**

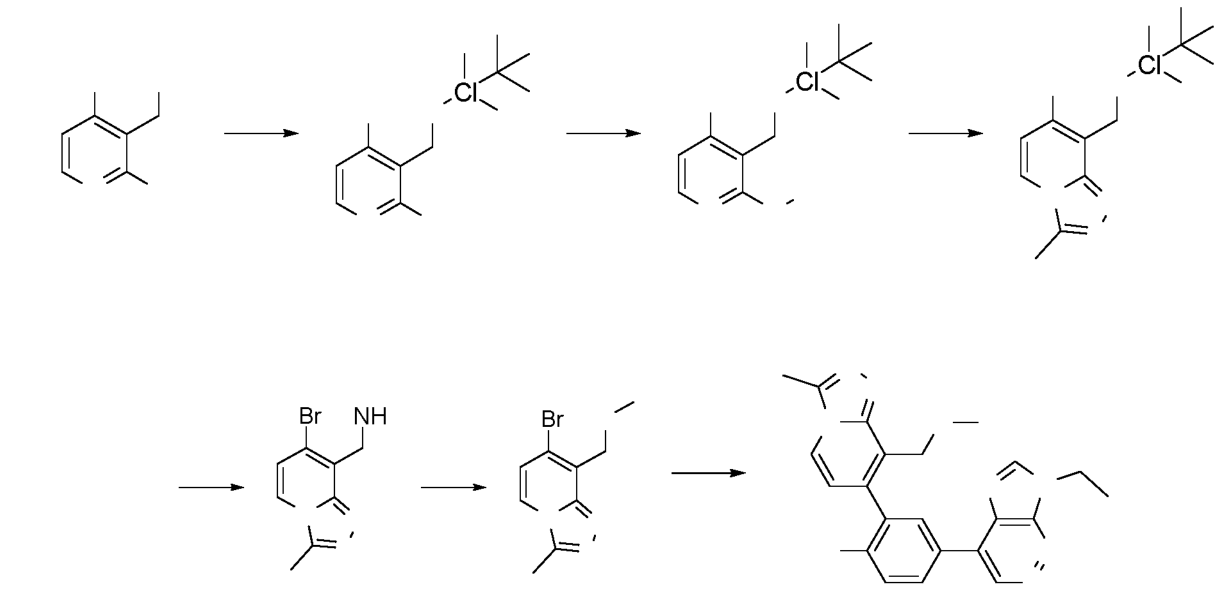

11-0 11-0 Cs

**58-1: 4-Bromo-3-(((*tert*-butyldimethylsilyl)oxy)methyl)-2-fluoropyridine**

(4-Bromo-2-fluoropyridin-3-yl)methanol (2.0 g, 9.71 mmol), *tert*-butyldimethylsilyl chloride (2.93 g, 19.42 mmol), and imidazole (1.32 g, 19.42 mmol) were added to dichloromethane (15 mL), and the mixture was stirred at room temperature for 30 min. The reaction mixture was subjected to column chromatography (petroleum ether/ethyl acetate = 20/1) to obtain the title compound (3.8 g, crude product) as a colorless oily liquid. LC-MS: m/z $[M+H]^+$ = 320, 322.

**58-2: 4-Bromo-3-(((*tert*-butyldimethylsilyl)oxy)methyl)-2-hydrazineylpyridine**

4-Bromo-3-(((*tert*-butyldimethylsilyl)oxy)methyl)-2-fluoropyridine (3.8 g, 11.86 mmol) and hydrazine hydrate (5.94 g, 118.6 mmol) were added to 1,4-dioxane (20 mL), and the mixture was allowed to react at 80 °C for 2 h. The reaction mixture was subjected to column chromatography (petroleum ether/ethyl acetate/aqueous ammonia = 20/1/0.05) to obtain the title compound (2.1 g, 53%) as a gray solid. LC-MS: m/z $[M+H]^+$ = 332, 334.

**58-3: 7-Bromo-8-(((*tert*-butyldimethylsilyl)oxy)methyl)-3-methyl-[1,2,4]triazolo[4,3-a]pyridine**

4-Bromo-3-(((*tert*-butyldimethylsilyl)oxy)methyl)-2-hydrazineylpyridine (1.03 g, 3.01 mmol) and an acetaldehyde-tetrahydrofuran solution (5 M, 1.6 mL) were added to 1,4-dioxane (6 mL), and the mixture was allowed to react at 80 °C for 1 h. Copper bromide (280 mg, 1.24 mmol) and potassium monopersulfate (2.29 g, 3.72 mmol) were added to the reaction mixture, and the mixture was allowed to react at room temperature for 4 h. The reaction mixture was filtered under vacuum. The residue was subjected to column chromatography (dichloromethane/methanol = 30/1) to obtain the title compound (262 mg, 23.54%) as a white solid. LC-MS: m/z $[M+H]^+$ = 356, 358.

**58-4: (7-Bromo-3-methyl-[1,2,4]triazolo[4,3-a]pyridin-8-yl)methanol**

7-Bromo-8-(((*tert*-butyldimethylsilyl)oxy)methyl)-3-methyl-[1,2,4]triazolo[4,3-a]pyridine (260 mg, 0.61 mmol) was added to dichloromethane (10 mL). A hydrochloric acid-dioxane solution (4 M, 2 mL) was added, and the mixture was stirred at room temperature for 20 min. The reaction mixture was concentrated. The residue was subjected to column chromatography (dichloromethane/methanol = 20/1) to obtain the title compound (160 mg, 96%) as a brown solid. LC-MS: m/z $[M+H]^+$ = 242, 244.

**58-5: 7-Bromo-8-(methoxymethyl)-3-methyl-[1,2,4]triazolo[4,3-a]pyridine**

(7-Bromo-3-methyl-[1,2,4]triazolo[4,3-a]pyridin-8-yl)methanol (160 mg, 0.66 mmol) was added to N,N-dimethylformamide (2 mL). Sodium hydride (52 mg, 1.32 mmol) was added to the reaction mixture, and the mixture was stirred at room temperature for 15 min. Iodomethane (190 mg, 1.31 mmol) was added to the reaction mixture, and the mixture was

allowed to react at room temperature for 30 min. The reaction mixture was subjected to preparative thin-layer chromatography (dichloromethane/methanol = 20/1) to obtain the title compound (143 mg, 85%). LC-MS: m/z $[M+H]^+$ = 256, 258.

**58: 7-Ethyl-4-(4-fluoro-3-(8-(methoxymethyl)-3-methyl-[1,2,4]triazolo[4,3-a]pyridin-7-yl)phenyl)-7H-imidazo[4,5-c]pyridazine**

Following the same experimental procedure as in Example 1, with starting materials of 7-bromo-3-methyl-8-(methoxymethyl)-[1,2,4]triazolo[4,3-a]pyridine (143 mg, 0.56 mmol) and (5-(7-ethyl-7H-imidazo[4,5-c]pyridazin-4-yl)-2-fluorophenyl)boronic acid (159 mg, 0.56 mmol), the title compound (82 mg, 35%) was obtained as a brown solid.

$^1$H NMR (400MHz, CHLOROFORM-d) 9.40 (s, 1 H), 8.43 (d, J = 5.9 Hz, 2 H), 8.28 (s, 1 H), 7.89 (d, J = 7.3 Hz, 1 H), 7.42 (t, J = 9.3 Hz, 1 H), 6.95 (d, J = 6.8 Hz, 1 H), 4.84 (s, 2 H), 4.59 (q, J = 7.3 Hz, 2 H), 3.41 (s, 3 H), 2.81 (s, 3 H), 1.70 (t, J = 7.3 Hz, 3 H). LC-MS: m/z $[M+H]^+$ = 418.

**Example 59**

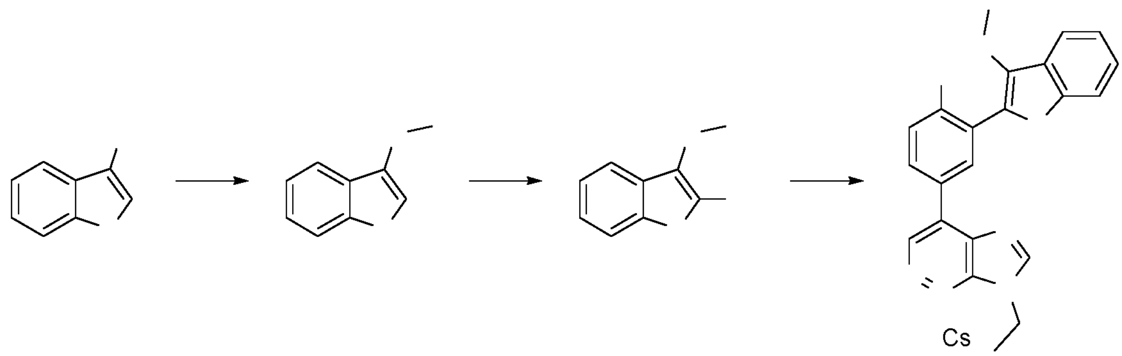


**59-1: 3-Methoxybenzo[b]thiophene**

3-Bromobenzo[b]thiophene (500 mg, 2.35 mmol), sodium methoxide (1267 mg, 23.5 mmol), copper oxide (93 mg, 1.18 mmol), cuprous iodide (22 mg, 0.12 mmol), and potassium iodide (19 mg, 0.12 mmol) were added to N,N-dimethylformamide (5 mL) and methanol (5 mL), and the mixture was allowed to react at 120 °C in a sealed tube overnight. Water (30 mL) was added to the reaction mixture, and the mixture was extracted with ethyl acetate. The organic phases were combined, washed with a saturated aqueous sodium chloride solution, dried over anhydrous sodium sulfate, and concentrated. The residue was subjected to column chromatography (pure petroleum ether) to obtain the title compound (308 mg, 80%) as a colorless oily liquid. LC-MS: m/z $[M+H]^+$ = 165.

**59-2: 2-Bromo-3-methoxybenzo[b]thiophene**

3-Methoxybenzo[b]thiophene (308 mg, 1.88 mmol) and N-bromosuccinimide (368 mg, 2.07 mmol) were added to dichloromethane (5 mL), and the mixture was stirred at 25 °C for 30 min. The reaction mixture was filtered under vacuum and concentrated. The residue was subjected to preparative thin-layer chromatography (pure petroleum ether) to obtain the title compound (311 mg, 68%) as a pink oily liquid. $^1$H NMR (400MHz, CHLOROFORM-d) d = 7.72 (d, J = 7.3 Hz, 1 H), 7.66 (d, J = 7.8 Hz, 1 H), 7.39 - 7.31 (m, 2 H), 4.05 (s, 3 H).

**59: 7-Ethyl-4-(4-fluoro-3-(3-methoxybenzo[b]thiophen-2-yl)phenyl)-7H-imidazo [4,5-c] pyridazine**

7-Ethyl-4-(4-fluoro-3-(4,4,5,5-tetramethyl-1,3,2-dioxaborolan-2-yl)phenyl)-7H-imidazo[4,5-c]pyridazine (59 mg, 0.16 mmol), 2-bromo-3-methoxybenzo[b]thiophene (97 mg, 0.4 mmol), cesium carbonate (104 mg, 0.32 mmol), and tetrakis(triphenylphosphine)palladium(0) (9 mg, 0.008 mmol) were added to 1,4-dioxane (2 mL) and water (0.2 mL), and the mixture was allowed to react at 100 °C for 4 h under an argon atmosphere. The reaction mixture was filtered under vacuum and concentrated. The residue was subjected to preparative thin-layer chromatography (petroleum ether/dichloromethane/methanol = 20/30/1) to obtain the title compound (6 mg, 9%) as a pale yellow solid.

$^1$H NMR (400MHz, CHLOROFORM-d) 9.43 (br. s., 1 H), 8.68 (d, J = 5.4 Hz, 1 H), 8.31 (br. s., 2 H), 7.90 - 7.76 (m, 2

H), 7.41 (br. s., 3 H), 4.60 (d, J = 7.3 Hz, 2 H), 3.93 (br. s., 3 H), 1.70 (t, J = 6.4 Hz, 3 H). LC-MS: m/z $[M+H]^+$= 405.

**Cell line construction and subculture**

An α subunit, a β subunit, and a γ subunit are essential for the formation of a complete functional $GABA_A$ receptor. In this example of the present disclosure, HEK293 cells stably expressing the human α1-$GABA_A$ receptor (α1-$GABA_A$R) and α2-$GABA_A$ receptor (α2-$GABA_A$R) were constructed and separately selected using the lipofection method (Felgner, P.L., et al. Proceedings of the National Academy of Sciences, 1987, 84: 7413-7417). The α1l-$GABA_A$R-HEK293 cell model simultaneously expressed the α1 subunit (protein sequence available in GenBank with an accession No.: NM_000806.5), the β3 subunit (protein sequence available in GenBank with an accession No.: NM_000814.5), and the γ2 subunit (protein sequence available in GenBank with an accession No.: NM_000816.3). The α2-$GABA_A$R-HEK293 cell model simultaneously expressed the α2 subunit (protein sequence available in GenBank with an accession No.: NM_000807.4), the β3 subunit (protein sequence available in GenBank with an accession No.: NM_000814.5), and the γ2 subunit (protein sequence available in GenBank with an accession No.: NM_000816.3).

The above cell lines were subcultured. The α2-$GABA_A$R-HEK293 cells were expanded for assay of the benzodiazepine (BZD) site affinity of compounds for $GABA_A$ receptors. During the passaging process, some of the α1-$GABA_A$R-HEK293 cells and α2-$GABA_A$R-HEK293 cells were suspended and plated onto slides pre-treated with Poly-D-Lysine for the electrophysiological test (the method referred to the patent CN 107344936 A, paragraph 0586).

**Affinity activity of the compounds of the present disclosure for α2-$GABA_A$ receptor**

The affinity of the compounds for the α2-$GABA_A$ receptor was determined by competing with $^3$H-flunitrazepam (isotope $^3$H-labelled flunitrazepam) for binding to the BZD site on the membrane proteins of HEK293 cells stably expressing the human α2-$GABA_A$ receptor.

Membrane preparation: The cells were suspended in 50 mM Tris-HCl buffer (pH = 7.4), homogenized 10 times (20 s for each time) with a homogenizer on ice, and then centrifuged at 4 °C at 1000 g for 10 min. The supernatant was taken. The above steps were repeated. The supernatant was centrifuged (33800 g; Thermo, rotor: A27-8×50) at 4 °C for 60 min. The pellet was resuspended in Tris buffer (50 mM Tris-HCl, 10 mM $MgCl_2$, 0.5 mM EDTA, and 10% glycerol). The protein content was determined (BCA (bicinchoninic acid) protein quantification assay based on the copper ion reduction method, with a BCA kit purchased from Pierce (Annoron (Beijing) Biotechnology Co., Ltd.)), and 1 mL aliquots were prepared and stored at -80 °C.

Radioligand competitive binding assay: This assay was performed in a 200 μL system (96-well plate) containing 100 μL of cell membranes. The concentration of$^3$H-flunitrazepam was 1 nM, and the concentrations of the test compounds were in the range of $1 \times 10^{-5}$-$10^{-6}$ M. Flumazenil was used as a control. To each low control (LC) well was added 1 μL of 2 mM flumazenil (the final concentration of 10 μM), and to each high control (HC) well was added 1 μL of dimethyl sulfoxide. The final concentration of the target membrane protein was 5 μg/well. All sample stock solutions of test compounds were 10 mM solutions in dimethyl sulfoxide. All the samples were diluted to a concentration of 0.2 mM with dimethyl sulfoxide, and then subjected to a 4-fold serial dilution to give a total of 8 working concentration gradients. The 96-well plate was sealed with a plate sealing membrane and then incubated on a shaker at room temperature for 1 h. Meanwhile, a GF/C filter plate was soaked in a plate soaking buffer (0.3% PEI (polyethyleneimine, purchased from: Sigma-Aldrich, model: P314), stored at 4 °C) for at least 0.5 h. After the binding incubation was completed, the cells were harvested onto the GF/C filter plate using a cell harvester and washed 4 times with a washing buffer (50 mM Tris-HCl, pH 7.4, stored at 4 °C). After drying in an oven at 50 °C for 1 h, the dried GF/C filter plate was bottom-sealed. The residual radioactivity on the filter membrane was detected by liquid scintillation counting. Scintillation fluid was added at 50 μL/well, and then the plate was sealed. Readings were taken using Microbeta$^2$ (microplate counter, purchased from: PerkinElmer, model: CNLL0153). The inhibitory activity of the test samples on the binding of $^3$H-flunitrazepam to the $GABA_A$ receptor membrane protein was calculated. The $IC_{50}$ values of the test samples were calculated by dose-response curve fitting (GraphPad Prism 5 software), and the Ki values of the samples were calculated from the $IC_{50}$ values, thereby assessing the binding capacity of the compounds to the α2-$GABA_A$ receptor BZD site.

Representative detection results obtained by the above method for determining the binding affinity of the compounds to the BZD site on the membrane proteins of human α2-$GABA_A$R-expressing HEK293 cells are shown in Table 1.

**Functional activity of the compounds of the present disclosure on different subtypes of $GABA_A$ receptors**

The positive regulation activity of the test drugs on the α1-$GABA_A$ receptor and the α2-$GABA_A$ receptor was detected by the electrophysiological method. The specific method was as follows:
Compound concentration setting: the final concentration of the compounds used for screening was 100 nM. For the cell

lines expressing the α1-$GABA_A$ receptor, the concentration of GABA was 0.05-0.07 μM (about $EC_{2-4}$); for the cell lines expressing the α2-$GABA_A$ receptor, the concentration of GABA was 0.10-0.11 μM (about $EC_{7-8}$). The electrophysiological test was performed using the whole-cell patch-clamp technique, which can refer to the method reported in the literature (Nickolls, S.A., et al. British Journal of Pharmacology, 2018, 175: 708-725). The components of the extracellular solution (ECS) for electrophysiology were as follows: 150 mM NaCl, 5 mM KC1, 2.5 mM $CaCl_2$, 1 mM $MgCl_2$, 10 mM HEPES, and 10 mM glucose (pH 7.4); the formulation of the intracellular solution (ICS) of electrodes for electrophysiology was as follows: 140 mM CsCl, 11 mM EGTA, 10 mM HEPES, 2 mM $CaCl_2$, 1 mM $MgCl_2$, 4 mM MgATP, and 2 mM TEA (pH 7.3). GABA (γ-aminobutyric acid) powder was prepared into a mother liquor with pure water, which was then diluted in ECS; the compounds were first prepared into 4 mM mother liquors with dimethyl sulfoxide and then gradually diluted to corresponding concentrations in GABA-ECS. The solutions were all prepared freshly prior to the electrophysiological test.

Electrophysiological signal acquisition was performed using an EPC 10 amplifier and PatchMaster software (HEKA) or an Axon700B amplifier and Clampex software (AXON). The recording electrode was made by pulling borosilicate glass, with an electrode resistance of 4-6 MΩ. Extracellular administration was performed using the ALA-VC-8PG™ system. A single, independently growing cell was selected. After the glass electrode formed a good seal with the cell, the membrane was ruptured to form the whole-cell configuration. The cell membrane potential was clamped at -60 mV and recorded in Gap-free mode. During the test, the extracellular solution was first applied extracellularly for about 20 s. After the baseline ($I_{prebaseline}$) stabilized, the extracellular solution was switched to GABA-ECS. At this time, the current caused by GABA ($I_{gaba}$) could be detected. After about 10-20 s, when the current stabilized, the extracellular solution was switched to a mixed solution of the compound and GABA-ECS. At this point, the current caused by both the compound and GABA ($I_{treatment}$) could be detected. Finally, the solution was switched to the extracellular solution, and the test was terminated after recording for 20-40 s. Only cells with a stable baseline, a control current magnitude ($I_{gaba}$-$I_{prebaseline}$) with an absolute value greater than 40 pA, and a relatively stable current within 10-20 s were used for the compound test.

The experimental results for the positive allosteric modulation activity of α1-$GABA_AR$ and α2-$GABA_AR$ were analyzed using PatchMaster v2x90.1 or PatchMaster v2x90.3 software (EPC 10 amplifier) and Clampex10.6 software (Axon700B amplifier). The current values at each stage were calculated based on the average value of the current after stabilization under the corresponding conditions. We defined the control current as $I_{gaba}$ - $I_{prebaseline}$, and the current after compound treatment as $I_{treatment}$ - $I_{prebaseline}$. The allosteric modulation activity of the compounds was expressed as a percentage, calculated by the following formula: functional activity = $[(I_{treatment} - I_{gaba})/(I_{gaba} - I_{prebaseline})] \times 100\%$. If the value is negative, it indicates that the test compound has a reverse allosteric modulation effect on the GABA receptor. If the value is positive, it indicates that the test compound has a positive allosteric modulation effect on the GABA receptor. For easy comparison, the positive compound CVL-865 was used as a reference for normalization, that is, the positive allosteric modulation activity = [the functional activity of the compound/the functional activity of the positive compound] × 100%. The test results for the positive allosteric modulation activity of the compounds on the α1-$GABA_A$ receptor and the α2-$GABA_A$ receptor are shown in Table 1.

### *In vitro* micronucleus assay

### 1. Guidelines for genotoxicity studies

The experimental design was based on the experimental purpose and followed the following experimental design guidelines:

(1) OECD Guidelines for the Testing of Chemicals 487, In Vitro Mammalian Cell Micronucleus Test, revised in 2016;
(2) ICH S2(R1): Guideline on Genotoxicity Testing and Data Interpretation for Pharmaceuticals Intended for Human Use, validated in November 2011.

### 2. Materials and methods

Experimental system and selection rationale: The experiment used Chinese hamster ovary cells (CHO-WBL cells) as an experimental system. The cell line has a karyotype containing 21 chromosomes and a growth cycle of 12 to 13 h. The CHO-WBL cells were originally derived from Dr. S. Wolff at the University of California in San Francisco, USA, then cloned in Dr. A. Bloom's laboratory at Columbia University in the City of New York, and subsequently cloned again by Dr. S. Galloway at Litton Bionetics laboratory in Maryland. The cell stock solution was stored in liquid nitrogen. The karyotype of each batch of cell stock solution was identified, and tests confirmed that it was free from mycoplasma contamination. The number of passages after cell cloning will not exceed 15. Chinese hamster ovary cells were chosen because the cell line is recommended for use in OECD Guidelines for the Testing of Chemicals 487 and has been proven to be sensitive to a variety of chromosome clastogens/aneugens (Marilyn J. *et al.,* 2006).

Medium and cell growth conditions: Chinese hamster ovary cells were cultured in McCoy's 5A complete medium

(McCoy's 5A complete medium supplemented with 10 vt% fetal bovine serum, 2 mM GlutaMAX™ medium, 100 units/mL penicillin, and 100 μg/mL streptomycin), and grown in a standard environment (high-humidity incubator with a temperature of 37 °C and a $CO_2$ concentration of 5%). The day before administration, CHO-WBL cells in exponential growth phase were plated at a density of $8 \times 10^3$ cells/400 μL McCoy's 5A complete medium/well onto eight-well cell culture slides.

Metabolic activation system: The *in vitro* metabolic activation system (Maron and Ames, 1983) included a rat liver microsomal homogenate induced by β-naphthoflavone and phenobarbital (referred to as S9) and an NADPH-generating system (i.e., NADP disodium and isocitric acid trisodium). S9 was purchased from CHI Scientific, Inc. and stored in a -75 °C refrigerator until use. Preparation method for S9 homogenate: Male Sprague Dawley rats were intraperitoneally injected with a single dose of β-naphthoflavone and phenobarbital, followed by tissue preparation.

Preparation of S9 metabolic activation system: The NADP disodium salt and the isocitric acid trisodium salt were first dissolved in a serum-free medium to prepare a core solution, which was then filtered through a 0.22 μm filter to sterilize. Immediately before use, the S9 homogenate was thawed, and the components of the S9 metabolic activation system were mixed in the proportions shown in Table 2 below.

Table 2 Components and proportions of S9 metabolic activation system

| Component | Final concentration in exposure medium |
|---|---|
| NADP disodium salt | 0.8 mg/mL |
| Isocitric acid trisodium salt | 1.5 mg/mL |
| S9 homogenate | 10 μL/mL |
| Note: the final concentration of S9 homogenate in the exposure medium was 1% (v/v). | |

Dose exploration experiment: In the dose exploration experiment, 8 administration concentrations were established in each administration treatment series. For each concentration of the test substance, 1 well of cells was set up. According to the ICH S2 (R1) guidelines, when not limited by the solubility of the test substance in the solvent/medium or the cytotoxicity of the test substance, the recommended maximum concentration is 1 mM or 0.5 mg/mL, whichever is lower. However, if limited by the solubility, the test maximum concentration is the minimum concentration at which a small amount of precipitate is visible in the medium under the microscope. To ensure that the upper solubility limit in the medium can be reached, up to 4 concentrations with visible precipitate can be tested.

Administration treatment: In the 3-hour administration series with S9 metabolic activation, the medium from each well was aspirated, and with reference to the ICH S2(R1) guidelines, a serum-free McCoy's 5A/S9 mixture medium containing the test substance/control at the appropriate concentration was added.

In the 3-hour administration series without metabolic activation, the medium from each well was aspirated, and a McCoy's 5A complete medium containing the test substance/control at the appropriate concentration was added.

Cells were incubated for 3 h under standard conditions. After the 3-hour incubation, the old medium from each well was aspirated and washed twice with a McCoy's 5A complete medium. AMcCoy's 5A complete medium containing cytochalasin B (the final concentration in medium of about 3 μg/mL) was added per well, and then the cells were cultured for 21 h until harvest.

For the 24-hour administration series without metabolic activation, the treatment was similar to that of the 3-hour administration series without metabolic activation, except that the cells were cultured with the test substance/control in the complete medium containing cytochalasin B (the final concentration in medium of about 3 μg/mL) for 24 h (± 30 min).

Slide preparation: At the time of harvest, the medium from the eight-well slides was poured off and blotted dry with absorbent paper. A hypotonic solution of 75 mM KCl was added per well. The cells were then fixed with two consecutive changes of fixative solution (anhydrous methanol:glacial acetic acid, 3:1 v/v), with each fixation lasting for 5 min, and then air-dried. The slides were stained with acridine orange and air-dried under light-protected conditions.

Cytotoxicity counting: The staining presented was examined under a low-power microscope to assess the analyzability of all wells on each slide (sufficient number of cells). Under a fluorescence microscope, at least 500 cells were analyzed per well. The numbers of mononuclear, binuclear, and multinuclear cells were counted separately to calculate the cytokinesis-block proliferation index (CBPI).

CBPI was used to assess the cytotoxicity of the test substance. The calculation formula for CBPI is as follows:

$$CBPI = \frac{\text{(The number of mononuclear cells + (2} \times \text{the number of binuclear cells) + (3} \times \text{the number of multinuclear cells)}}{\text{Total number of cells}}$$

$$\% \text{ cytotoxicity} = 100 - 100 \{(\text{CBPIT} - 1)/(\text{CBPIC} - 1)\}$$

where T = the test substance treatment culture group, and C = the vehicle control culture group.

When CBPI is 1, it equals 100% cytotoxicity.

Main micronucleus assay: In the micronucleus main assay, CHO-WBL cells were exposed to 3-8 concentrations of the test substance, with a positive control group (cyclophosphamide monohydrate) and a vehicle control group (dimethyl sulfoxide) treated in the same way as the solvent group, with 2 wells of cells for each dose group. In the test system without activation, the administration times were 3 and 24 h, and in the test system with S9 activation, the exposure time of the test substance was 3 h. The upper limit for testing the test substance depended on its solubility in the medium, but generally, it did not exceed the maximum concentration of 1 mM or 0.5 mg/mL, whichever was lower. When selecting the highest dose for micronucleus frequency analysis, the cytotoxicity of the test substance dose group should not exceed 50% too much compared with that of the corresponding vehicle control group. If the maximum concentration was not limited by cytotoxicity, to reach the upper solubility limit in the medium, multiple doses with visible precipitate could be tested, and a small amount of identifiable precipitate could be seen in the medium of the highest dose group.

Dose selection for micronucleus analysis: The dose selection for micronucleus analysis of CHO-WBL cells will depend on the cytotoxicity of the test substance. The highest dose selected for assessment will induce a cytotoxicity that does not exceed 50% too much. At least two additional groups with lower doses (in the range of moderately cytotoxic to non-cytotoxic, if applicable) were further analyzed. If the test substance was not cytotoxic, the dose at which the precipitate could be observed under a microscope at the end of the administration, or the maximum test concentration (1 mM or 0.5 mg/mL, whichever was lower) was selected as the maximum concentration for micronucleus analysis.

Micronucleus frequency counting: All slides selected for micronucleus analysis were labeled with randomly generated blind codes to reduce the subjectivity of reading the slides. The blind codes were made by personnel not involved in reading the slides. The blind code label on each slide included the following information: the experiment number, the treatment series, and a random number.

For each dose group, 2000 binuclear cells (1000 per well) were analyzed to count the frequency of binuclear cells containing micronuclei.

The binuclear cells should meet the following criteria to be counted:

a. The cell possesses an intact cell membrane. b. The two daughter nuclei are equal in size.

The criteria for judging micronuclei are as follows:

a. The micronucleus has a similar fluorescence intensity to the main nucleus. b. The micronucleus should be free in the cytoplasm. c. The micronucleus has a smooth edge and a diameter less than or equal to one-third of the main nucleus diameter.

The slides were discarded before signing the experimental report.

Data

Data report: The cytotoxicity of the administration group was judged and presented based on its comparison with the cytotoxicity of the corresponding vehicle control. The micronucleus frequency for each well and each dose group in the main micronucleus assay was also presented.

Statistical analysis: Fisher's exact probability method was used to compare the significance of the difference in the frequency of binuclear cells with micronuclei between the administration group and the corresponding vehicle control group. When making multiple comparisons, the P-value was corrected by Bonferroni. When comparing multiple groups of data, the Cochran-Armitage test was used to detect dose-response relationships.

When $P \leq 0.05$, the difference was considered significant.

Criteria for experimental validity: An acceptable genotoxicity test must meet the following criteria: the frequency of micronucleus cells in the vehicle/negative control must be comparable with historical negative control data; there should be at least three concentrations that can be analyzed; the proportion of micronucleus cells in the positive control should be statistically significantly higher ($p \leq 0.05$) than that in the parallel vehicle/negative control group.

Criteria for judgment of experimental results: On the premise that the experiment is valid, according to the guidelines of ICH S2(R1), the experimental results should be judged with reference to the results of micronucleus rates under different administration treatment conditions (3-hour treatment with S9, 3-hour treatment without S9, 24-hour treatment without S9) and at different concentrations of the test substance. Referring to the criteria for judging the experimental results, a comprehensive analysis should be conducted to judge the results of *in vitro* micronucleus assay of

the test substance as negative, positive, or suspected positive, with negative results being preferred. The specific criteria for assessing the experimental results are as follows:

Positive: a test substance was considered positive if it met the following criteria: a significant increase in the frequency of micronucleus cells is observed in one or more dose groups compared with the parallel vehicle control group; the increase in micronucleus frequency has a dose-response relationship; the frequency of micronucleus cells in one or more dose groups exceeds the range of historical negative data in the laboratory.

Negative: if none of the above three points were not met, the test substance can be considered negative.

Suspected positive: if a test substance only partially met the above three points, it should be judged scientifically. Evidence of concentration-related effects was considered useful, but was not necessary when assessing positive results. Biological relevance will be considered, such as the consistency of responses within the concentration range and between concentrations, as well as (if applicable) the consistency of responses between experiments, or effects that only occur at high or extremely strong cytotoxic concentrations.

The results for the affinity activity of each compound of the present disclosure for the α2-$GABA_A$ receptor and the positive allosteric modulation activity thereof on α1 and α2-$GABA_A$ receptors are shown in Table 3 below, and the results of the *in vitro* micronucleus assay are shown in Table 4 below.

It should be noted that the advantage of the compounds described in the present disclosure lies in their higher selectivity for the receptors, not just in the absolute activity of the drugs. At the same time, considering that the absolute value of the functional activity of the compounds may vary across different detection systems, which is not comparable, therefore, for the convenience of comparison, the experiments in the present disclosure were conducted in the same detection system to detect the controls and the compounds involved simultaneously, and the positive compound CVL-865 was used as a reference for normalization, that is, the positive allosteric modulation activity = [the functional activity of the compound/the functional activity of the positive compound] × 100%. The specific results are shown in Table 3 below.

Table 3 Affinity activity of some compounds for α2-$GABA_A$ receptor and positive allosteric modulation activity thereof on α1 and α2-$GABA_A$ receptors

| Serial number | α2 Ki (unit: nM) | Positive allosteric modulation activity on α1-$GABA_A$R | Positive allosteric modulation activity on α2-$GABA_A$R |
|---|---|---|---|
| CVL-865 | 2.23 | 100% | 100% |
| 1 | 10.5 | 70% | 129% |
| 6 | | -16% | 118% |
| 7 | | -20% | 88% |
| 12 | 5.79 | 32% | 104% |
| 16 | 8.17 | -56% | 95% |
| 17 | 2.85 | -49% | 100% |
| 27 | 6.05 | 4% | 110% |
| 28 | 5.12 | 19% | 126% |
| 29 | 6.60 | -51% | 156% |
| 30 | 12.04 | 6% | 143% |
| 32 | 2.89 | -39% | 102% |
| 34 | 8.13 | 25% | 95% |
| 36 | 11.76 | 49% | 89% |
| 37 | 6.39 | 74% | 79% |
| 38 | 6.53 | -14% | 94% |
| 41 | 0.11 | 59% | 178% |
| 49 | 16.46 | 44% | 82% |
| 50 | 9.89 | 29% | 103% |
| 52 | 8.07 | 19% | 99% |
| 54 | 2.96 | 47% | 100% |

(continued)

| Serial number | $\alpha$2 Ki (unit: nM) | Positive allosteric modulation activity on $\alpha$1-$GABA_AR$ | Positive allosteric modulation activity on $\alpha$2-$GABA_AR$ |
|---|---|---|---|
| 58 | 11.4 | 40% | 123% |

The compounds in the examples of the present disclosure have better selectivity compared with the reference compound CVL-865, maintaining appropriate affinity and electrophysiological activity for the $\alpha$2-$GABA_A$ receptor while reducing the electrophysiological activity for the $\alpha$1-$GABA_A$ receptor to less than 70% of that of CVL-865, so as to achieve smaller side effects while maintaining the *in vivo* efficacy.

Table 4 Results of *in vitro* micronucleus assay

| Example | Structure | Results of *in vitro* micronucleus assay |
|---|---|---|
| Patent WO2014091368A1 example 4 (CVL-865) | 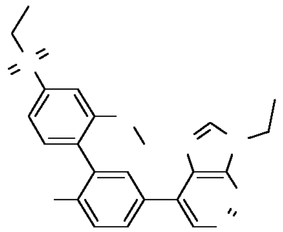 | Positive |
| Patent WO2015189744A1, example 1 | 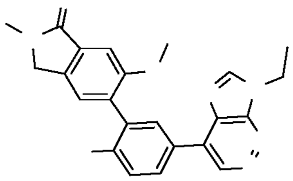 | Positive |
| Example 37 | 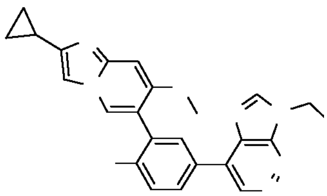 | Negative |

As can be seen from the results of *in vitro* micronucleus assay, the compounds of the present disclosure have better genotoxic safety and better druggability.

Obviously, the above examples are provided solely for the purpose of illustration and are not intended to limit the embodiments. Various variations and modifications can be made by those of ordinary skill in the art on the basis of the above description. It is neither necessary nor possible to exhaustively list all the embodiments here. The obvious variations or modifications derived therefrom still fall within the protection scope of the present disclosure.

## Claims

1. A compound of formula (1), a stereoisomer thereof, a tautomer thereof, a prodrug thereof, a pharmaceutically acceptable salt thereof, an amorphous material thereof, an isotopic variant thereof, a polymorph thereof, or a solvate thereof:

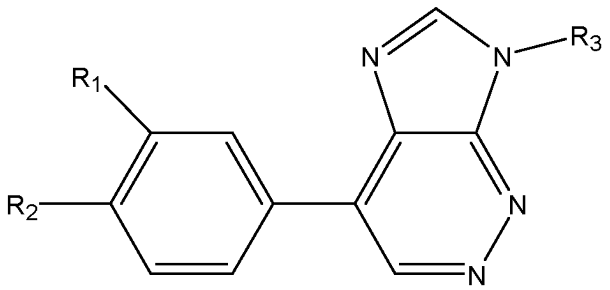

Formula (1)

wherein

$R_1$ is a fused group formed by a substituted or unsubstituted heterocyclic ring and a heterocyclic ring;
$R_2$ is selected from H, halogen, OH, C1-C6 alkoxy or CN;
$R_3$ is selected from H, substituted or unsubstituted linear or branched C1-C6 alkyl or substituted or unsubstituted substituted C3-C6 cycloalkyl.

**2.** The compound according to claim 1, wherein two heterocyclic rings forming the fused group of $R_1$ are independently a 5- to 7-membered saturated or unsaturated monocyclic group comprising 1 to 3 ring heteroatoms selected from N, O or S.

**3.** The compound according to claim 1 or 2, wherein $R_1$ has a structure of formula (2), (3), (4), (5), (6) or (7):

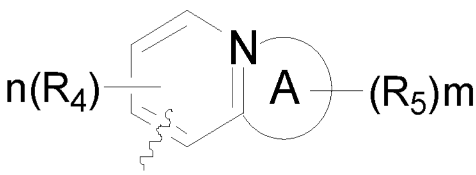

(2)

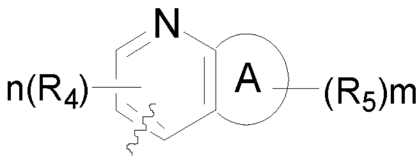

(3)

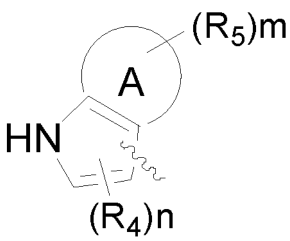

(4)

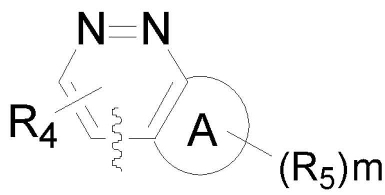

(5)

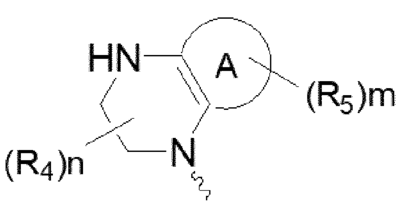

(6)

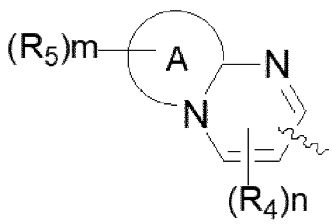

;

(7)

wherein

$R_4$ is selected from hydrogen, C1-C6 alkyl, C3-C6 cycloalkyl, C1-C6 alkoxy, C1-C6 alkylthio or C1-C6 alkylsulfonyl, wherein the above groups are optionally unsubstituted or each independently substituted with 1-4 groups each selected from at least one of halogen, hydroxyl, $C_1$-$C_3$ alkyl, halo $C_1$-$C_3$ alkyl, $C_1$-$C_3$ alkoxy or halo $C_1$-$C_3$ alkoxy;

$R_5$ is selected from hydrogen, halogen, hydroxyl, oxo, C1-C6 alkylacyl, C1-C6 alkylamido, C1-C6 alkoxyimino, C1-C6 alkyl, C2-C6 alkenyl, C2-C6 alkynyl, C3-C6 cycloalkyl, C1-C6 alkoxy, C6-C10 aryl, 5- to 10-membered heteroaryl comprising 1-3 heteroatoms and C1-C6 non-aromatic heterocyclic ring comprising 1-3 heteroatoms, wherein the above groups are optionally unsubstituted or each independently substituted with 1-4 groups each selected from at least one of halogen, hydroxyl, C1-C3 alkyl, halo C1-C3 alkyl, C1-C3 alkoxy, halo C1-C3 alkoxy or 3- to 7-membered heterocycloalkyl comprising 1-3 heteroatoms of N or O;

ring A refers to a 5- to 7-membered saturated or partially unsaturated monocyclic group comprising N; m is 1, 2 or 3, and n is 1 or 2; ∿∿∿ represents a linking site.

4. The compound according to claim 3, wherein $R_4$ is selected from hydrogen, methyl, ethyl, *n*-propyl, isopropyl, *n*-butyl, isobutyl, *tert*-butyl, vinyl, ethynyl, cyclopropyl, cyclobutyl, methoxy, ethoxy, *n*-propoxy, $-CH_2-O-CH_3$, piperidine, methyloxime, methylsulfonyl or ethylsulfonyl, wherein the above groups are optionally unsubstituted or each independently substituted with C1-C3 alkoxy or halogen.

5. The compound according to claim 3 or 4, wherein $R_5$ is selected from hydrogen, F, Cl, Br, hydroxyl, formyl, acetyl, formamido, acetamido, methoxyimino, ethoxyimino, methyl, ethyl, isopropyl, cyclopropyl, cyclobutyl, cyclopentyl, cyclohexyl, methoxy, ethoxy, phenyl, benzyl, tetrahydrofuryl, tetrahydropyranyl, hexahydropyridyl, oxetane and azetidine; the above groups are optionally unsubstituted or each independently substituted with 1-4 groups each selected from at least one of F, Cl, Br, hydroxyl, methoxy, methyl, tetrahydropyrrole, morpholinyl or $-CH_2-CF_3$.

6. The compound according to claim 5, wherein $R_5$ is selected from H, methyl, ethyl, isopropyl,

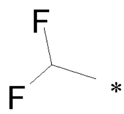, 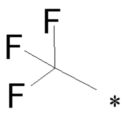, 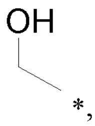, 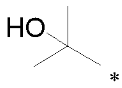, 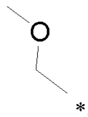,

cyclopropyl, fluorocyclopropyl,

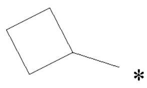, 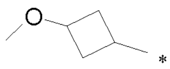, 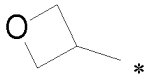, 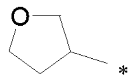, 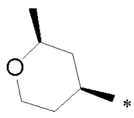, 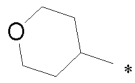, 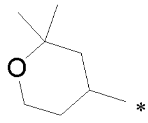,

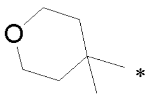, 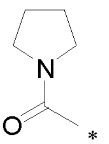, 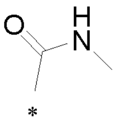, 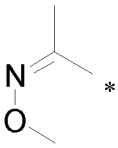, 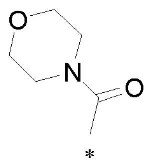, 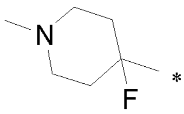, 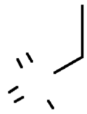,

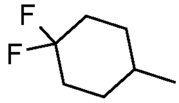 or 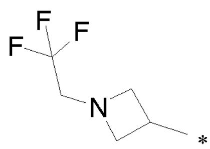.

7. The compound according to any one of claims 3 to 6, wherein ring A is a 5- to 6-membered saturated or unsaturated monocyclic group comprising 1, 2 or 3 ring heteroatoms of N; wherein

when $R_1$ has the structure of formula (2) or (7), ring A has the following structure:

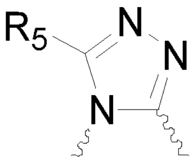, 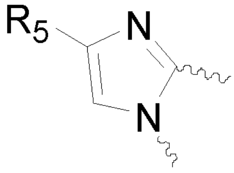, 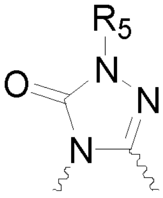, 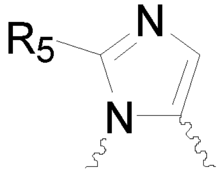 or 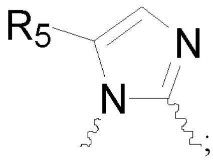;

when $R_1$ has the structure of formula (3), (4), (5) or (6), ring A has the following structure:

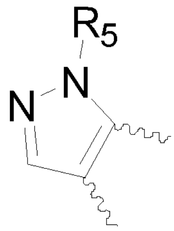, 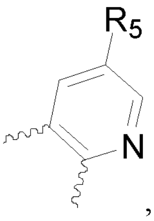, 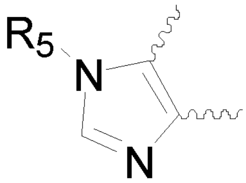 or 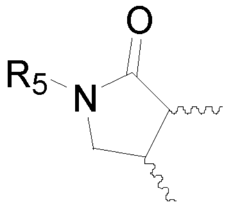;

wherein 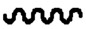 represents a linking site.

**8.** The compound according to any one of claims 3 to 7, wherein $R_1$ is a substituted or unsubstituted pyridine fused heterocyclic ring.

**9.** The compound according to claim 8, wherein $R_1$ is substituted or unsubstituted pyridotriazole or substituted or unsubstituted pyridoimidazole.

**10.** The compound according to any one of claims 3 to 7, wherein $R_1$ is selected from any one of the following compounds:

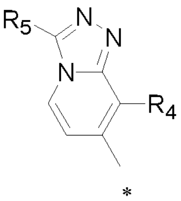, 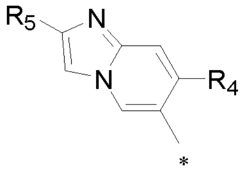, 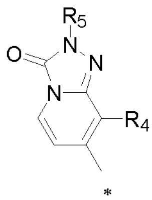, 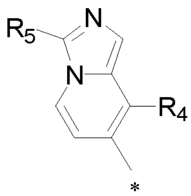, 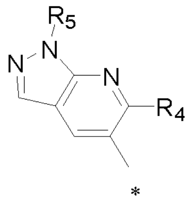,

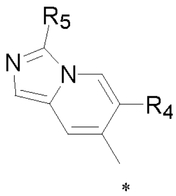, 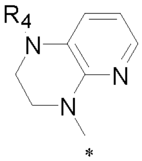, 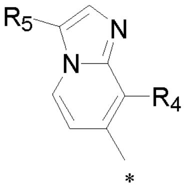, 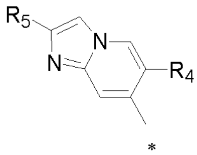, 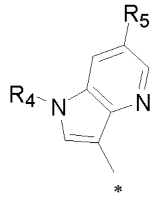, 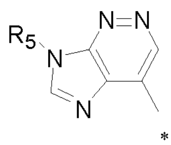,

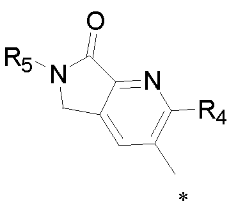, 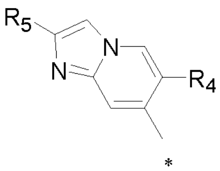, 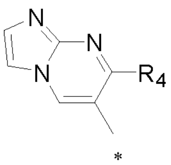, 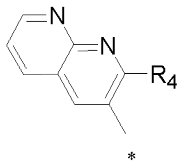, 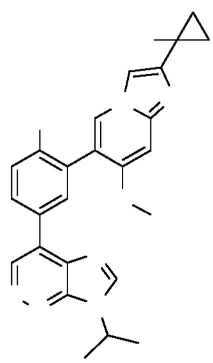

or

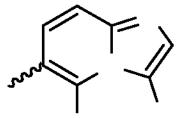

**11.** The compound according to any one of claims 3 to 7, wherein $R_1$ is selected from any one of the following compounds:

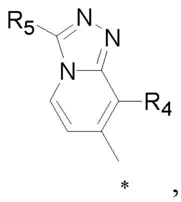 , 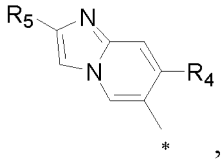 , 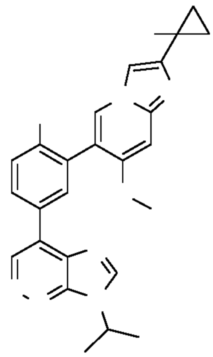 or 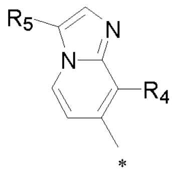 ;

and

$R_4$ is selected from methoxy-C1-C3 alkyl or C1-C3 alkoxy, and is preferably methoxymethyl or methoxy;
$R_5$ is selected from H, methyl, ethyl, isopropyl,

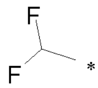,

cyclopropyl, fluorocyclopropyl,

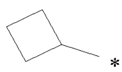, 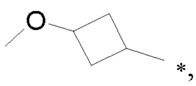, 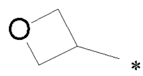, 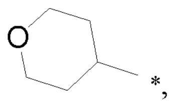, 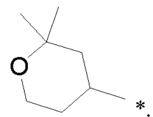.

**12.** The compound according to any one of claims 1 to 11, wherein

$R_2$ is selected from H or F;
$R_3$ is selected from H or linear or branched C1-C6 alkyl.

**13.** The compound according to any one of claims 1 to 12, wherein the compound is selected from any one of the following compounds:

| | | | |
|---|---|---|---|
| 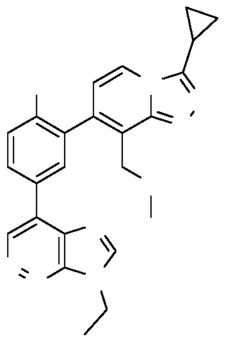 Compound 1 | 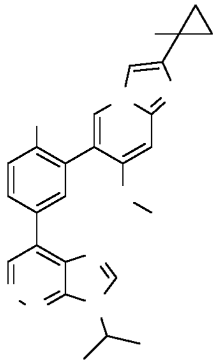 Compound 2 | 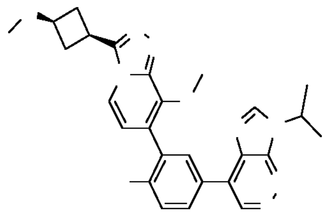 Compound 3 | 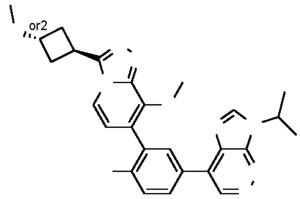 Compound 4 |
| 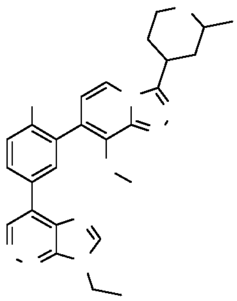 Compound 5 | 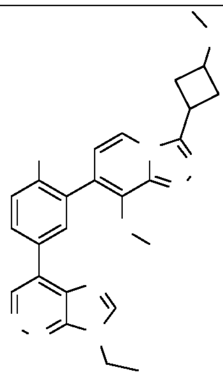 Compound 6 | 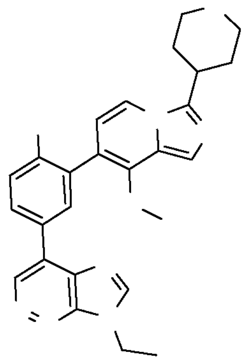 Compound 7 | 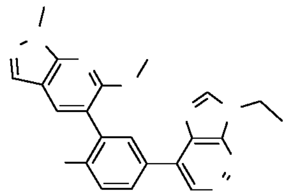 Compound 8 |
| 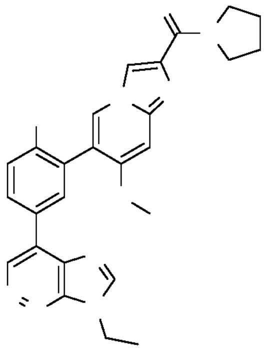 Compound 9 | 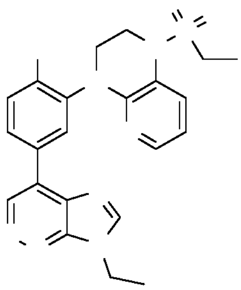 Compound 10 | 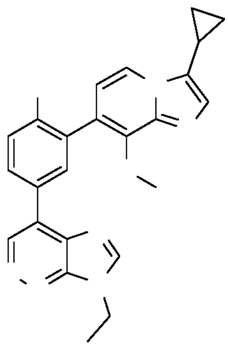 Compound 11 | 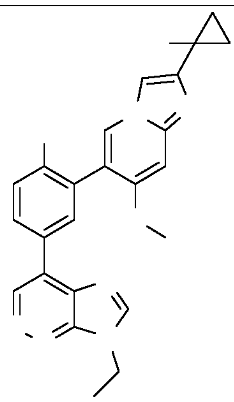 Compound 12 |

| | | | |
|---|---|---|---|
| 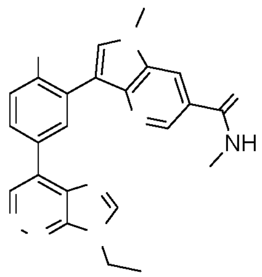<br>Compound 13 | 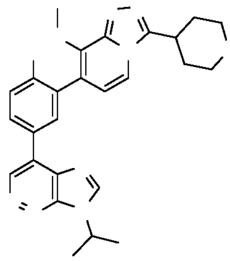<br>Compound 14 | 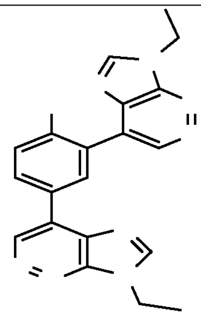<br>Compound 15 | 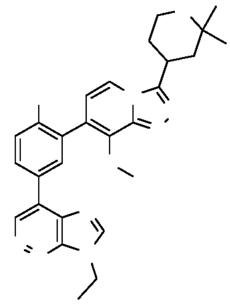<br>Compound 16 |
| 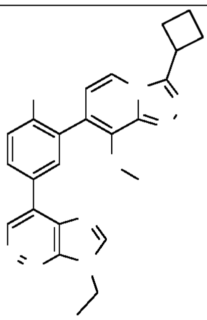<br>Compound 17 | 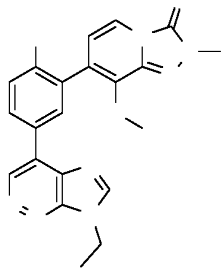<br>Compound 18 | 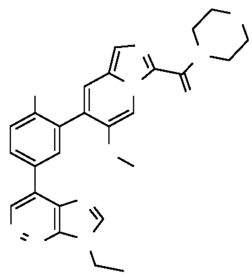<br>Compound 19 | 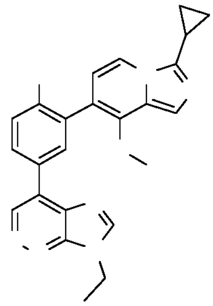<br>Compound 20 |
| 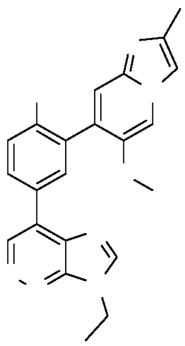<br>Compound 21 | 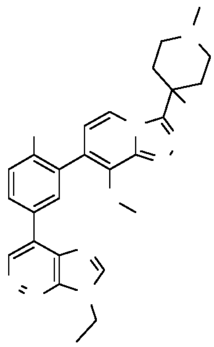<br>Compound 22 | 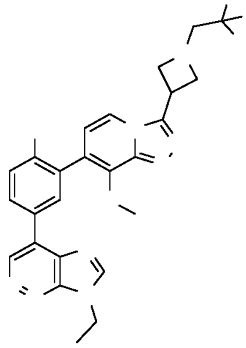<br>Compound 23 | 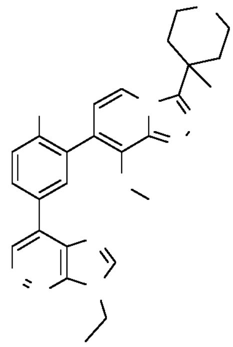<br>Compound 24 |
| 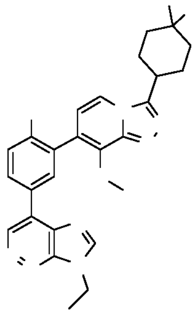<br>Compound 25 | 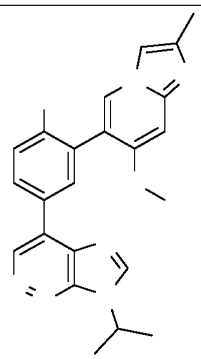<br>Compound 26 | 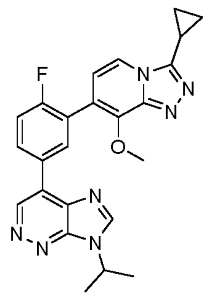<br>Compound 27 | 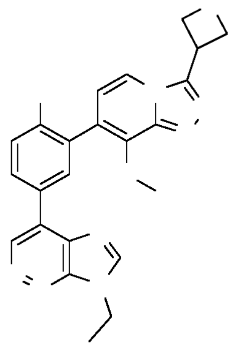<br>Compound 28 |

| | | | |
|---|---|---|---|
| 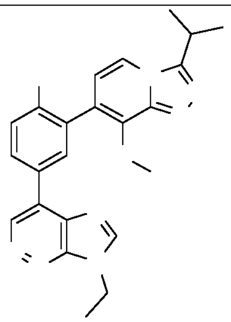<br>Compound 29 | 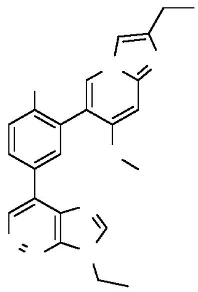<br>Compound 30 | 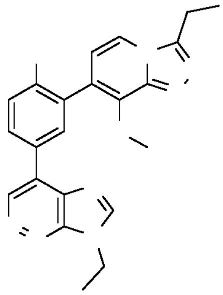<br>Compound 31 | 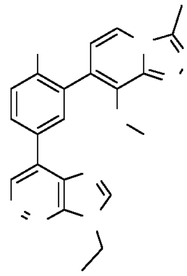<br>Compound 32 |
| 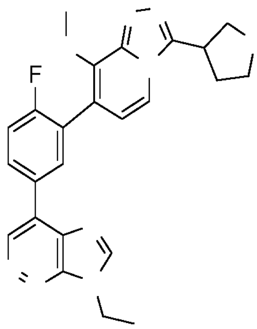<br>Compound 33 | 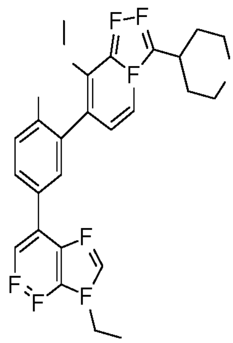<br>Compound 34 | 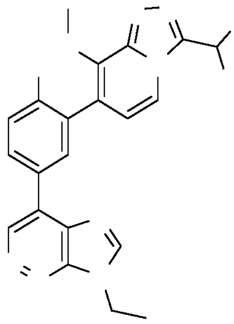<br>Compound 35 | 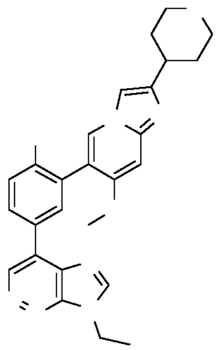<br>Compound 36 |
| 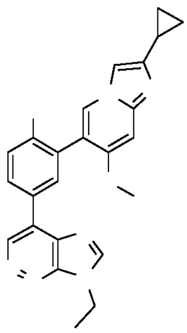<br>Compound 37 | 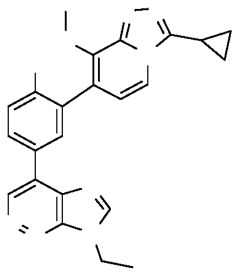<br>Compound 38 | 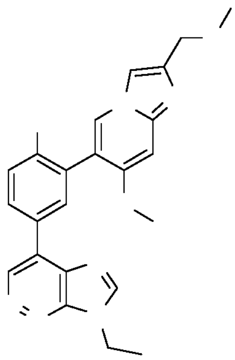<br>Compound 39 | 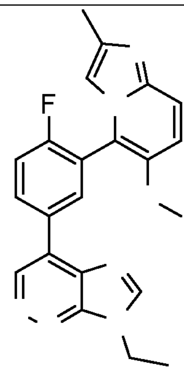<br>Compound 40 |
| 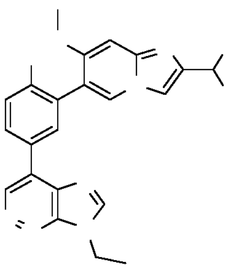<br>Compound 41 | 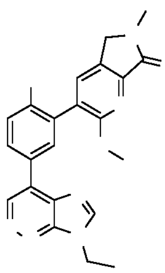<br>Compound 42 | 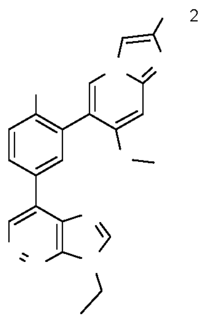<br>Compound 43 | 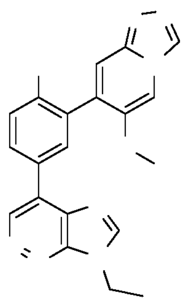<br>Compound 44 |

| | | | |
|---|---|---|---|
| 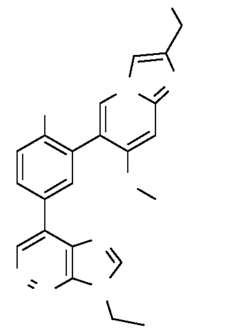 Compound 45 | 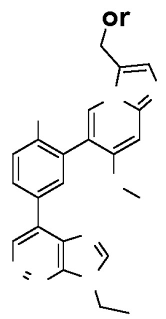 Compound 46 | 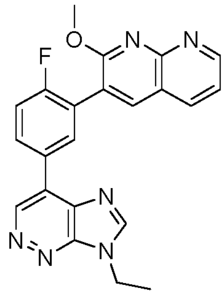 Compound 47 | 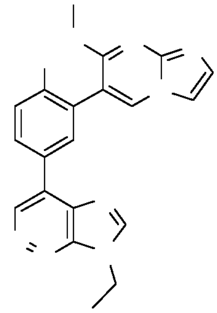 Compound 48 |
| 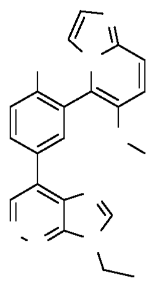 Compound 49 | 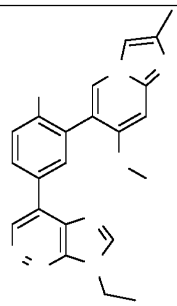 Compound 50 | 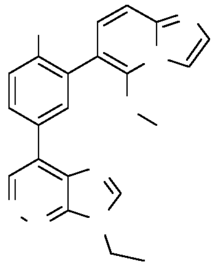 Compound 51 | 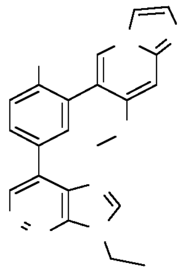 Compound 52 |
| 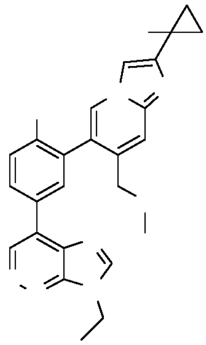 Compound 53 | 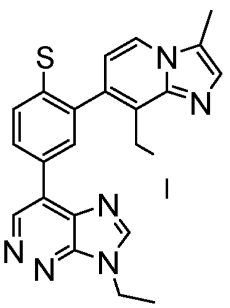 Compound 54 | 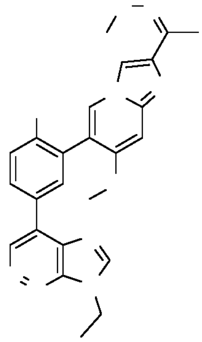 Compound 55 | 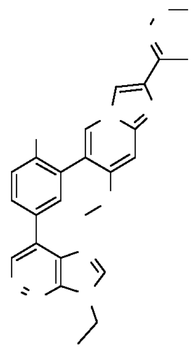 Compound 56 |
| 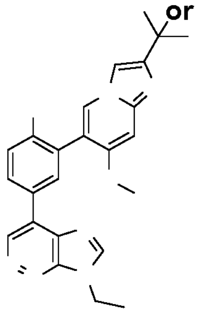 Compound 57 | 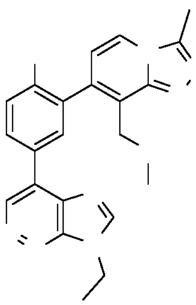 Compound 58 | | |

.

**14.** A pharmaceutical composition, wherein the pharmaceutical composition comprises at least one of the compounds or the stereoisomers thereof, the tautomers thereof, the prodrugs thereof, the pharmaceutically acceptable salts thereof, the amorphous materials thereof, the isotopic variants thereof, the polymorphs thereof or the solvates thereof according to any one of claims 1 to 13, and optionally supplemented with pharmaceutically acceptable carriers and/or adjuvants.

**15.** Use of the compound or the stereoisomer thereof, the tautomer thereof, the prodrug thereof, the pharmaceutically acceptable salt thereof, the amorphous material thereof, the isotopic variant thereof, the polymorph thereof or the solvate thereof according to any one of claims 1 to 13, or the pharmaceutical composition according to claim 10 in the manufacture of a medicament for treating or preventing a $GABA_A$ receptor-associated disease.

**16.** The use according to claim 15, wherein the $GABA_A$ receptor-associated disease is selected from at least one of the following: pain, Alzheimer's disease, multi-infarct dementia, epilepsy, pruritus or stroke.

**17.** The use according to claim 16, wherein the pain comprises neuropathic pain, inflammatory pain and cancerous pain.

**18.** The use according to claim 16 or 17, wherein the pain is selected from: headache, facial pain, neck pain, shoulder pain, back pain, chest pain, abdominal pain, dorsalgia, low back pain, lower limb pain, musculoskeletal pain, vascular pain, gout, arthritis pain, visceral pain, pain due to infectious diseases, bony pain, pain associated with sickle cell anemia, autoimmune diseases, multiple sclerosis or inflammation, chronic pain caused by injury or surgery, nociceptive pain, painful diabetes, trigeminal neuralgia, pain of lumbar or cervical radiculopathy, glossopharyngeal neuralgia, autonomic neuroreflex pain, and pain associated with reflex sympathetic dystrophy, nerve root avulsion, cancer, chemical injury, toxin, nutritional deficiencies, viral or bacterial infection or degenerative osteoarthropathy.

**INTERNATIONAL SEARCH REPORT**

International application No.
**PCT/CN2023/076805**

**A. CLASSIFICATION OF SUBJECT MATTER**

C07D 519/00(2006.01)i; A61K31/5025(2006.01)i; A61P25/00(2006.01)i

According to International Patent Classification (IPC) or to both national classification and IPC

**B. FIELDS SEARCHED**

Minimum documentation searched (classification system followed by classification symbols)

IPC: C07D, A61K, A61P

Documentation searched other than minimum documentation to the extent that such documents are included in the fields searched

Electronic data base consulted during the international search (name of data base and, where practicable, search terms used)

CNTXT, CNKI, ENTXTC, VCN, STN: 上海赛默罗生物科技有限公司, 上海赛默罗德生物科技有限公司, 咪唑并哒嗪, 氨基丁酸, GABAA受体, imidazopyridazine, GABAA receptor?, 结构检索, structure search

**C. DOCUMENTS CONSIDERED TO BE RELEVANT**

| Category* | Citation of document, with indication, where appropriate, of the relevant passages | Relevant to claim No. |
|---|---|---|
| A | CN 104837843 A (PFIZER INC.) 12 August 2015 (2015-08-12)<br>see abstract, claim 1, and embodiments 1-50 | 1-18 |
| A | WO 2015189744 A1 (PFIZER LIMITED) 17 December 2015 (2015-12-17)<br>see abstract, claim 1, and embodiments 1-23 | 1-18 |
| A | CN 101384559 A (NEUROSEARCH A/S) 11 March 2009 (2009-03-11)<br>see abstract, and claim 1 | 1-18 |
| A | WO 2017098367 A1 (PFIZER LIMITED) 15 June 2017 (2017-06-15)<br>see abstract, and claim 1 | 1-18 |

☐ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **01 June 2023** | **07 June 2023** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**
**Information on patent family members**

International application No.
**PCT/CN2023/076805**

| Patent document cited in search report | Publication date (day/month/year) | Patent family member(s) | Publication date (day/month/year) |
|---|---|---|---|
| CN 104837843 A | 12 August 2015 | PH 12015501293 B1 | 24 August 2015 |
| | | AP 201508526 A0 | 30 June 2015 |
| | | EA 201590761 A1 | 30 December 2015 |
| | | EA 025635 B1 | 30 January 2017 |
| | | UY 35185 A | 31 July 2014 |
| | | ECSP 15029822 A | 31 August 2017 |
| | | EP 2938615 A1 | 04 November 2015 |
| | | EP 2938615 B1 | 26 October 2016 |
| | | ES 2608640 T3 | 12 April 2017 |
| | | US 2015259352 A1 | 17 September 2015 |
| | | AP 201508526 D0 | 30 June 2015 |
| | | CU 20150058 A7 | 27 November 2015 |
| | | CU 24338 B1 | 03 April 2018 |
| | | MX 2015007598 A | 22 October 2015 |
| | | MX 366023 B | 24 June 2019 |
| | | TN 2015000261 A1 | 03 October 2016 |
| | | HUE 032400 T2 | 28 September 2017 |
| | | CY 1118568 T1 | 12 July 2017 |
| | | SG 11201503735 YA | 30 July 2015 |
| | | WO 2014091368 A1 | 19 June 2014 |
| | | GT 201500159 A | 23 September 2015 |
| | | GEP 201706710 B | 25 July 2017 |
| | | NI 201500081 A | 16 September 2015 |
| | | US 2015105396 A1 | 16 April 2015 |
| | | CR 20150254 A | 09 July 2015 |
| | | AR 093937 A1 | 01 July 2015 |
| | | CL 2015001652 A1 | 14 August 2015 |
| | | DOP 2015000147 A | 15 September 2015 |
| | | LT 2938615 T | 27 December 2016 |
| | | AU 2013356894 A1 | 28 May 2015 |
| | | AU 2013356894 B2 | 12 November 2015 |
| | | BR 112015014097 A2 | 17 December 2019 |
| | | BR 112015014097 B1 | 29 November 2022 |
| | | TW 201434840 A | 16 September 2014 |
| | | TWI 478926 B | 01 April 2015 |
| | | US 2014171435 A1 | 19 June 2014 |
| | | US 8952008 B2 | 10 February 2015 |
| | | MA 38112 A1 | 30 March 2018 |
| | | MA 38112 B1 | 31 October 2018 |
| | | PE 20151163 A1 | 14 August 2015 |
| | | IL 239243 A0 | 30 July 2015 |
| | | JP 2016503001 A | 01 February 2016 |
| | | JP 5869740 B2 | 24 February 2016 |
| | | HRP 20161559 T1 | 30 December 2016 |
| | | UA 112028 C2 | 11 July 2016 |
| | | PT 2938615 T | 26 December 2016 |
| | | MD 20150051 A2 | 30 September 2015 |
| | | MD 4651 B1 | 30 September 2019 |
| | | KR 20150088877 A | 03 August 2015 |
| | | KR 101746314 B1 | 12 June 2017 |

Form PCT/ISA/210 (patent family annex) (July 2022)

**INTERNATIONAL SEARCH REPORT**
**Information on patent family members**

International application No.
**PCT/CN2023/076805**

| Patent document cited in search report | Publication date (day/month/year) | Patent family member(s) | Publication date (day/month/year) |
|---|---|---|---|
| | | ZA 201504226 B | 29 June 2016 |
| | | ME 02566 B | 20 February 2017 |
| | | PL 2938615 T3 | 28 April 2017 |
| | | SI 2938615 T1 | 28 February 2017 |
| | | DK 2938615 T3 | 02 January 2017 |
| | | RS 55307 B1 | 31 March 2017 |
| | | HK 1209743 A1 | 08 April 2016 |
| | | CA 2892174 A1 | 19 June 2014 |
| | | CA 2892174 C | 06 February 2018 |
| WO 2015189744 A1 | 17 December 2015 | JP 2017517538 A | 29 June 2017 |
| | | JP 6491679 B2 | 27 March 2019 |
| | | CA 2951497 A1 | 17 December 2015 |
| | | CA 2951497 C | 09 April 2019 |
| | | ES 2664810 T3 | 23 April 2018 |
| | | EP 3154979 A1 | 19 April 2017 |
| | | EP 3154979 B1 | 07 March 2018 |
| | | US 2017197965 A1 | 13 July 2017 |
| | | US 9802945 B2 | 31 October 2017 |
| CN 101384559 A | 11 March 2009 | TW 200811113 A | 01 March 2008 |
| | | TWI 391381 B | 01 April 2013 |
| | | PT 2001855 E | 17 January 2011 |
| | | NO 20084481 L | 18 December 2008 |
| | | DE 602007010261 D1 | 16 December 2010 |
| | | KR 20080111097 A | 22 December 2008 |
| | | HRP 20100616 T1 | 28 February 2011 |
| | | JP 2009530353 A | 27 August 2009 |
| | | JP 5260489 B2 | 14 August 2013 |
| | | IL 192297 A0 | 22 September 2009 |
| | | IL 192297 A | 28 November 2013 |
| | | MY 149855 A | 31 October 2013 |
| | | AT 486856 T | 15 November 2010 |
| | | MX 2008010885 A | 08 September 2008 |
| | | AU 2007229502 A1 | 04 October 2007 |
| | | AU 2007229502 B2 | 20 September 2012 |
| | | AR 060084 A1 | 21 May 2008 |
| | | BRPI 0710203 A2 | 24 May 2011 |
| | | DK 2001855 T3 | 07 February 2011 |
| | | RU 2008129868 A | 27 April 2010 |
| | | RU 2435759 C2 | 10 December 2011 |
| | | PL 2001855 T3 | 29 April 2011 |
| | | EP 2319837 A1 | 11 May 2011 |
| | | EP 2319837 B1 | 31 October 2012 |
| | | US 2011118259 A1 | 19 May 2011 |
| | | US 8288418 B2 | 16 October 2012 |
| | | NZ 569438 A | 27 May 2011 |
| | | HK 1132257 A1 | 19 February 2010 |
| | | SI 2001855 T1 | 31 March 2011 |
| | | CA 2647252 A1 | 04 October 2007 |
| | | EP 2001855 A1 | 17 December 2008 |
| | | EP 2001855 B1 | 03 November 2010 |

Form PCT/ISA/210 (patent family annex) (July 2022)

**INTERNATIONAL SEARCH REPORT**
**Information on patent family members**

International application No.
**PCT/CN2023/076805**

| Patent document cited in search report | Publication date (day/month/year) | Patent family member(s) | Publication date (day/month/year) |
|---|---|---|---|
| | | ES 2355163 T3 | 23 March 2011 |
| | | CY 1111603 T1 | 07 October 2015 |
| | | US 2009036476 A1 | 05 February 2009 |
| | | US 7902230 B2 | 08 March 2011 |
| | | WO 2007110374 A1 | 04 October 2007 |
| WO 2017098367 A1 | 15 June 2017 | JP 2018536677 A | 13 December 2018 |
| | | SG 11201803489 SA | 28 June 2018 |
| | | EP 3386983 A1 | 17 October 2018 |
| | | US 2018346470 A1 | 06 December 2018 |
| | | US 10538523 B2 | 21 January 2020 |
| | | CA 3007595 A1 | 15 June 2017 |
| | | CA 3007595 C | 25 August 2020 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202210178772
- GB 0104948 W
- WO 2002038568 A
- GB 0203114 W
- WO 2003008418 A
- US 9914935 W
- WO 2000001697 A
- WO 2013060631 A
- WO 2014091368 A
- WO 2015054200 A
- WO 2015189744 A
- US 2019033598 W
- WO 201926820 A1
- CN 107344936 A
- WO 2014091368 A1
- WO 2015189744 A1


### Non-patent literature cited in the description


- **MIRZA, N. R.** ; **MUNRO, G.** *Drug News and Perspectives*, 2010, vol. 23 (6), 351-360
- **ROBERT M. OWEN**. *J. Med. Chem.*, 2019, vol. 62, 5773-5796
- **DUVEAU, V**. *CNS Neurosci. Ther.*, 2019, vol. 25 (2), 255-260
- **UWE RUDOLPH** ; **FREDERIC KNOFLACH**. *Nature Reviews: Drug Discovery*, 2011, vol. 10 (9), 685-697
- Design of Prodrugs. Elsevier, 1985
- **BONICA et al.** The Management of Pain. Lea&Feboger, 1990, vol. I
- Remington's Pharmaceutical Sciences. Maack Publishing Co.
- **FELGNER, P.L. et al.** *Proceedings of the National Academy of Sciences*, 1987, vol. 84, 7413-7417
- **NICKOLLS, S.A. et al.** *British Journal of Pharmacology*, 2018, vol. 175, 708-725
- OECD Guidelines for the Testing of Chemicals 487. *Vitro Mammalian Cell Micronucleus Test*, 2016
- *ICH S2(R1): Guideline on Genotoxicity Testing and Data Interpretation for Pharmaceuticals Intended for Human Use*, November 2011